# EUROPEAN PATENT APPLICATION

(11) **EP 2 103 602 A1**
(43) Date of publication of application: **23.09.2009**
(21) Application number: 08102647.8
(22) Date of filing: 17.03.2008
(51) Int. Cl.: C07D 249/08, A61K 31/4196, A61P 5/06

(54) **Novel 1,2,4-triazole derivatives and process of manufacturing thereof**

(71) Applicant: Æterna Zentaris GmbH, 60314 Frankfurt am Main (DE); Centre National de la Recherche Scientifique, 75794 Paris Cedex 16 (FR); University of Montpellier I, 34967 Montpellier Cedex 2 (FR); University of Montpellier II, 34095 Montpellier Cedex 5 (FR)
(72) Inventor: Fehrentz, Jean-Alain, 3440, St. Nazaire de Pezan (FR); Bibian, Mathieu, 12100 Creissels (FR); Moulin, Aline, 26800 Portes les Valence (FR); Martinez, Jean, 34720 Caux (FR)

(57) **Abstract**

The invention discloses a process of manufacturing 1,2,4-triazole derivatives comprising steps (a) to (e). The invention further discloses novel 1,2,4-triazoie derivatives and their use as medicaments.

## Description

### Technical field

The invention relates to a process of manufacturing 1,2,4-triazole derivatives comprising steps (a) to (e). The invention further relates to novel 1,2,4-triazole derivatives and their use as medicaments.

### Prior art

In 1997, Avalos and co-workers studied the thiophile character of metals (Avalos M et al., Tetrahedon 1997, 53 (42): 14463-14480). The selective preparation of nitriles, imides or amides is described and bis(thioacetanilide)mercury(II) as key reaction intermediate is discussed.

Hitotsuyanagi and co-workers describe a cis amide bond surrogate incorporating 1,2,4-triazole that was synthesized by the reaction of thionotripeptide, formic hydrazide and mercury(II)acetate (Hitotsuyanagi Y et al., J. Org. Chem. 2002, 67: 3266-3271).

Boeglin and co-workers describe solution and solid-supported synthesis of 1,2,4-triazole based peptidomimetics using mercury(II)acetate (Boeglin D et al., Org. Lett. 2003, 5(23): 4465-4468).

WO 00/54729 discloses heterocyclic aromatic compounds as GH secretagogues which are said to stimulate endogenous production and/or release of GH and can also contain triazole moieties. In addition, a method for increasing levels of endogenous GH or increasing the endogenous production or release of GH administering such GHS is described. Furthermore, a method is provided for preventing or treating osteoporosis (improving bone density and/or strength), or treating obesity, or increasing muscle mass and/or muscle strength and function in elderly humans, or reversal or prevention of frailty in elderly humans administering such GHS.

WO 01/36395 discloses triazole compounds for the treatment of tumor cell proliferation.

WO 2007/020013 relates to triazole derivatives as ghrelin analogue ligands of growth hormone secretagogue receptors that are useful in the treatment or prophylaxis of physiological and/or pathophysiological conditions in mammals that are mediated by GHS receptors. The application further provides GHS receptor antagonists and agonists that can be used for modulation of these receptors and are useful for treating above conditions, in particular growth retardation, cachexia, short-, medium- and/or long term regulation of energy balance; short-, medium- and/or long term regulation (stimulation and/or inhibition) of food intake; adipogenesis, adiposity and/or obesity; body weight gain and/or reduction; diabetes, diabetes type I, diabetes type II, tumor cell proliferation; inflammation, inflammatory effects, gastric postoperative ileus, postoperative ileus and/or gastrectomy (ghrelin replacement therapy).

### Description of the invention

The present invention has the object to provide a novel process of manufacturing 1,2,4-triazole derivatives that overcomes the disadvantages of the prior art, in particular the use of toxic materials and slow reaction times over several days. It is another object of the present invention to provide novel 1,2,4-triazole derivatives.

The object of the invention has been surprisingly solved in one aspect by providing a process of manufacturing 1,2,4-triazole derivatives comprising the following steps:
(a) reacting a compound of formula (I) with a compound of formula (II) in a solvent in the presence of a coupling reagent and a base at a temperature Tₐ to yield a compound of formula (III) ("peptide coupling or acylation") 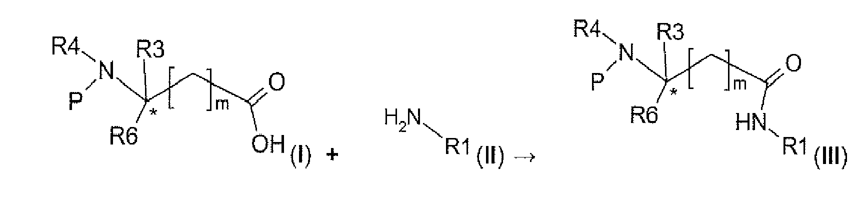
(b) reacting a compound of formula (III) in a solvent with a thionating reagent at a temperature T_{b} to yield a compound of formula (IV) ("thionation") 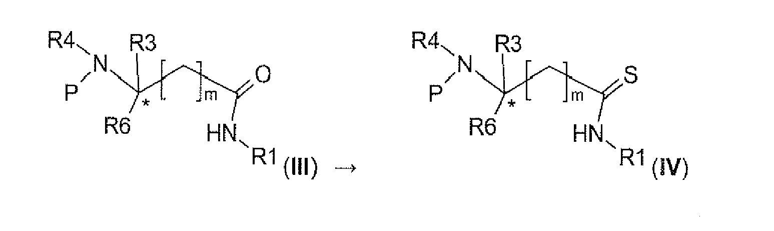
(c) optionally, reacting a compound of formula (V) with hydrazine in a solvent at a temperature T_{c} to yield a compound of formula (VI) ("hydrazinolysis") 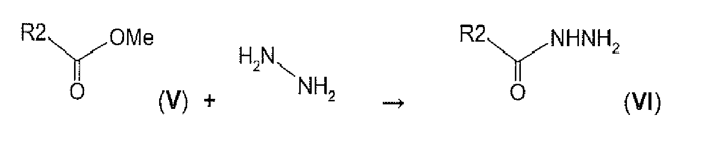
(d) reacting a compound of formula (IV) with a compound of formula (VI) in a solvent in the presence of a silver-compound and an acid at a temperature T_{d} to yield a compound of formula (VII) ("cyclization") 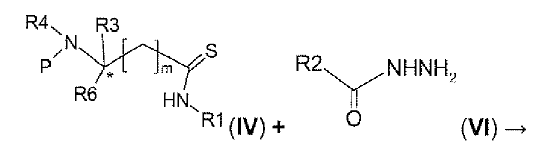 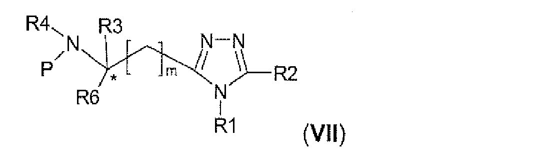
(e) reacting a compound of formula (VII) in a solvent in the presence of an acid at a temperature Tₑ to yield a compound of formula (VIII) ("deprotection") 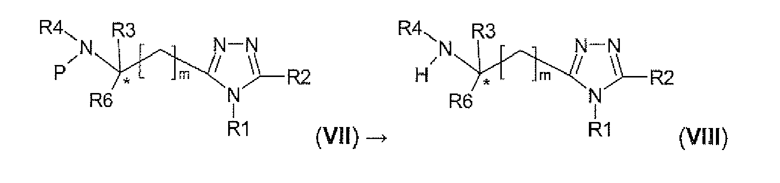 wherein:
   R1, R2 are independently of one another selected from the group consisting of "hydrogen atom, alkyl, alkenyl, alkynyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl" which are optionally substituted in the alkyl, cycloalkyl, cycloalkylalkyl, aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, - O-alkyl, -O-aryl, -O-arylalkyl", and preferably are selected from the group consisting of "alkyl, aryl, heteroaryl, arylalkyl, heteroarylalkyl" optionally being substituted by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
   one of radicals R3, R4 is a hydrogen atom, whereas the other radical is selected from the group consisting of "hydrogen atom, alkyl, aryl, heteroaryl, arylalkyl, heteroarylalkyl, heterocyclyl, heterocyclylalkyl, -alkyl-O-aryl, -alkyl-O-arylalkyl, - alkyl-O-heteroaryl, -alkyl-O-heteroarylalkyl, -alkyl-O-heterocyclyl, alkyl-O-heterocyclylalkyl, -alkyl-CO-aryl, -alkyl-CO-arylalkyl, -alkyl-CO-heteroaryl, - alkyl-CO-heteroarylalkyl, -alkyl-CO-heterocyclyl, -alkyl-CO-heterocyclylalkyl, - alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, -alkyl-NH₂, -alkyl-NH-C(NH)-NH₂, alkylsulfonyl, arylsulfonyl, arylalkylsulfonyl, alkyl-S-alkyl, alkyl-S-H" which are optionally substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, - NO₂, alkyl, aryl, arylalkyl, -O-alkyl, -O-aryl, -O-arylalkyl"; and preferably are selected from the group consisting of "arylalkyl, heteroarylalkyl, heterocyclylalkyl, - alkyl-O-aryl, -alkyl-O-arylalkyl, -alkyl-O-heteroaryl, -alkyl-O-heteroarylalkyl, - alkyl-O-heterocyclyl, alkyl-O-heterocyclylalkyl, -alkyl-CO-aryl, -alkyl-CO-arylalkyl, -alkyl-CO-heteroaryl, -alkyl-CO-heteroarylalkyl, -alkyl-CO-heterocyclyl, alkyl-CO-heterocyclylalkyl, -alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, - alkyl-NH₂, -alkyl-NH-C(NH)-NH₂," optionally being substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
   R6 is selected from the group consisting of "hydrogen atom, alkyl, cycloalkyl, cycloalkylalkyl" and preferably is a hydrogen atom;
   R7, R8 are independently of one another selected from the group consisting of "hydrogen atom, alkyl, cycloalkyl, cycloalkylalkyl" and preferably are a hydrogen atom;
   P is a protection group and preferably is selected from the group consisting of:

"Boc, Fmoc, Z, CBZ, Aloc, trityl, acetyl, benzyl"
m is 0, 1 or 2 and preferably is 0; and
* means a carbon atom of R or S configuration when chiral;
   The object of the invention has been surprisingly solved in another aspect by providing a process of manufacturing 1,2,4-triazole derivatives comprising the following step:
   (d) reacting a compound of formula (IV) with a compound of formula (VI) in a solvent in the presence of a silver-compound and an acid at a temperature T_{d} to yield a compound of formula (VII) ("cyclization") 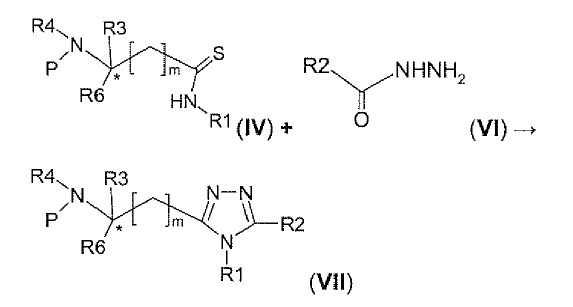 wherein:
   R1, R2, R3, R4, R6, R7, R8, P, m have the meanings as illustrated above.
   The object of the invention has been surprisingly solved in another aspect by providing a process as illustrated above, wherein following step (e)
(f) a compound of formula (VIII) is reacted in a solvent in the presence of a coupling reagent and a base or a reducing reagent or no reagent with a compound of formula (IX) at a temperature T_{f} to yield a compound of formula (X) ("R5 introduction") 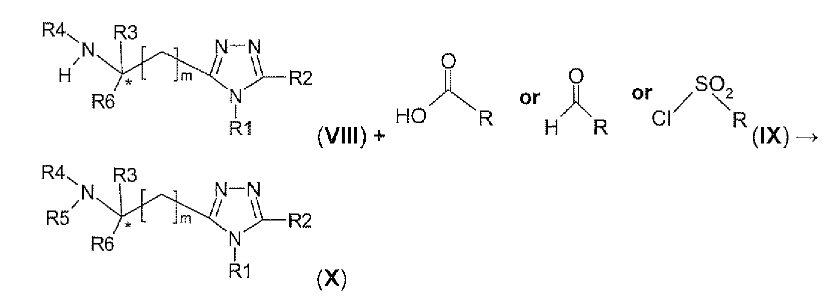
(g) optionally, reacting a compound of formula (X) in a solvent in the presence of an acid at a temperature Tg to yield a deprotected compound of formula (X) ("deprotection of R5")
   wherein:
   R5 is selected from the group consisting of "hydrogen atom, alkyl, cycloalkyl, cycloalkylalkyl, aryl, heteroaryl, arylalkyl, heteroarylalkyl, heterocyclyl, heterocyclyllalkyl, -CO-alkyl, -CO-cycloalkyl, -CO-cycloalkylalkyl, -CO-aryl, -CO-arylalkyl, - CO-heteroaryl, -CO-heteroarylalkyl, -CO-heterocyclyl, -CO-heterocyclylalkyl, - CO-C*(R9R10)-NH₂, -CO-CH₂-C*(R9R10)-NH₂, -CO-C*(R9R10)-CH₂-NH₂, alkylsulfonyl, arylsulfonyl, arylalkylsulfonyl" which are optionally substituted by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, - O-arylalkyl"; and preferably is selected from the group consisting of "hydrogen atom, -CO-alkyl, -CO-cycloalkyl, -CO-aryl, -CO-heteroaryl, -CO-arylalkyl, - CO-heteroarylalkyl, -CO-heterocyclyl, -CO-C*(R9R10)-NH₂, -CO-CH₂-C*(R9R10)-NH₂, -CO-C*(R9R10)-CH₂-NH₂, optionally being substituted by up to 3 substituents independently selected from the group consisting of "halogen, -F, - Cl, -Br, -I, -N₃, -CN, -NR7R8, -OH, -NO₂, alkyl, aryl, arylalkyl, -O-alkyl, -O-aryl, -O-arylalkyl";
   R9, R10 are independently of one another selected from the group consisting of "hydrogen atom, alkyl, natural alpha-amino acid side chain, unnatural alpha-amino acid side chain" and preferably are selected from the group consisting of "hydrogen atom, alkyl";
   m is 0, 1 or 2 and preferably is 0; and
   * means a carbon atom of R or S configuration when chiral.

The terms indicated for explanation of the above compounds of above formulae always, unless indicated otherwise in the description or in the claims, have the following meanings:
The term "substituted" means that the corresponding radical or group has one or more substituents. Where a radical has a plurality of substituents, and a selection of various substituents is specified, the substituents are selected independently of one another and need not be identical. The term "unsubstituted" means that the corresponding group has no substituent. The term "optionally substituted" means that the corresponding group is either unsubstituted or substituted by one or more substituents. The term "substituted by up to 3 substituents" means that the corresponding radical or group is substituted either by one or by two or three substituents.
The term "alkyl" includes for the purposes of this invention acyclic saturated hydrocarbons having C1-C12 carbon atoms, which may be straight-chain or branched. The term "alkyl" preferably stands for alkyl chains of 1 to 8, particularly preferably 1 to 6, carbon atoms. Examples of suitable alkyl radicals are methyl, ethyl, n-propyl, isopropyl, n-butyl, isobutyl. sec-butyl, tert-butyl, n-pentyl, tert-pentyl, 2- or 3-methyl-pentyl, n-hexyl, isohexyl, n-heptyl, n-octyl, n-nonyl, n-decyl, n-undecyl, n-dodecyl.
The term "cycloalkyl" stands for a saturated or partially unsaturated non-aromatic cyclic hydrocarbon group/radical, containing 1 to 3 rings, including monocyclic alkyl, bicyclic alkyl and tricyclic alkyl, and containing a total of 3 to 20 carbon atoms forming the rings, preferably 3 to 10, most preferably (C3-C8)-cycloalkyl. Examples of suitable cycloalkyl radicals are cyclopropyl, cyclobutyl, cyclopentyl, cyclohexyl, cycloheptyl, cyclooctyl, cyclodecyl, cyclohexenyl, cyclopentenyl, cyclooctadienyl.
The term "cycloalkylalkyl" refers to a radical in which the cycloalkyl group is linked via an alkyl group, where the alkyl and cycloalkyl groups have the meanings defined herein, preferably a (C3-C8)-cycloalkyl-(C1-C4)-alkyl radical. Examples thereof are cyclopropylmethyl, cyclohexylmethyl, cyclopentylethyl, cyclohexenylethyl.
The term "alkenyl" includes for the purposes of this invention acyclic unsaturated or partially unsaturated hydrocarbons having C2-C12 carbon atoms, which may be straight-chain or branched and contain one or more double bonds. The term "alkenyl" preferably stands for alkenyl chains of 2 to 8, particularly preferably 2 to 6, carbon atoms. Examples are vinyl, propenyl, butenyl, pentenyl, hexenyl, and octadienyl and the like.
The term "alkynyl" refers to acyclic unsaturated or partially unsaturated hydrocarbons having C2-C12 carbon atoms, which may be straight-chain or branched and contain one or more triple bonds. The term "alkynyl" preferably stands for alkynyl chains of 2 to 8, particularly preferably 2 to 6, carbon atoms. Examples are propynyl, butynyl, pentynyl, hexynyl.
The term "aryl" refers to aromatic hydrocarbon systems having 3 to 14, preferably 5 to 14, carbon atoms, which may also be fused to further saturated, (partially) unsaturated or aromatic cyclic systems. Examples of "aryl" are inter alia phenyl, biphenyl, naphthyl and anthracenyl, but also indanyl, indenyl, or 1,2,3,4-tetrahydronaphthyl.
The term "heteroaryl" refers to a 5-, 6- or 7-membered cyclic aromatic radical which comprises at least 1, where appropriate also 2, 3, 4 or 5 heteroatoms, preferably nitrogen, oxygen and/or sulfur, where the heteroatoms are identical or different. The number of nitrogen atoms is preferably between 0 and 3, and that of the oxygen and sulfur atoms is between 0 and 1. The term "heteroaryl" also includes systems in which the aromatic cycle is part of a bi- or polycyclic system, such as were the aromatic cycle is fused to an aryl, cycloalkyl, heteroaryl or heterocyclyl group as defined herein via any desired and possible ring member of the heteroaryl radical. Examples of "heteroaryl" include pyrrolyl, thienyl, furyl, imidazolyl, thiazolyl, isothiazolyl, oxazolyl, isoxazolyl, pyrazolyl, pyridinyl, pyrimidinyl, pyrazinyl, indolyl, quinolinyl, and isoquinolinyl.
The terms "arylalkyl" and "heteroarylalkyl" refer to radicals in which the aryl or heteroaryl radical is linked via an alkyl group, where the alkyl, aryl and heteroaryl groups have the meanings defined herein. Preferred "arylalkyl" groups are phenyl-(C₁-C₄)-alkyl radicals, preferably benzyl or phenylethyl radicals. Preferred "heteroarylalkyl" groups are indolyl-(C₁-C₄)-alkyl radicals, preferably 1*H*-indole-3-yl-methyl or 2(1*H*-indole-3-yl)-ethyl.
The term "heterocyclyl" refers to a mono- or polycyclic system of 3 to 14, preferably 5 or 6 to 14 ring atoms which may be exclusively carbon atoms. However, the cyclic system may also comprise 1, 2, 3, 4, or 5 heteroatoms, in particular nitrogen, oxygen and/or sulfur. The cyclic system may be saturated, mono- or polyunsaturated but may not be aromatic. In the case of a cyclic system consisting of at least two rings the rings may be fused or spiro- or otherwise connected. The "heterocyclyl" radical may be attached at any carbon or heteroatom which results in the creation of a stable structure. Examples include pyrrolidinyl, thiapyrrolidinyl, piperidinyl, piperazinyl, oxapiperazinyl, oxapiperidinyl and oxadiazolyl.
The term "heterocyclylalkyl" refers to radicals in which the heterocyclyl group is linked via an alkyl group, where the alkyl and hetercyclyl groups have the meanings defined herein.
The terms "alkylsulfonyl", "arylsulfonyl" and "arylalkylsulfonyl" refer to radicals in which the alkyl, aryl or arylalkyl group is linked via a -SO₂- group, where the alkyl, aryl and arylalkyl groups have the meanings defined herein. Examples are methylsulfonyl and phenylsulfonyl.
The term "halogen", "halogen atom" or "halogen substituent" (Hal-) refers to one, where appropriate, a plurality of fluorine (F, fluoro), bromine (Br, bromo), chlorine (Cl, chloro), or iodine (I, iodo) atoms. The designations "dihalogen", "trihalogen" and "perha-logen" refer respectively to two, three and four substituents, where each substituent can be selected independently from the group consisting of fluorine, chlorine, bromine and iodine. "Halogen" preferably means a fluorine, chlorine or bromine atom.
The term "natural alpha-amino acid side chain" for the purpose of the present invention refers to all side chains of the known 20 proteinogenic alpha-amino acids as well as to side chains of naturally occurring (i.e. in any biological systems) alpha-amino acids, such as for instance selenocystein, pyrrolysine, citrulline, ornithine, homocysteine, N-methylariginine, N-acetyllysine, gamma-carboxyglutamate, 5-hydroxylysine, 3-methylhistidine and/or *N,N,N*,-trimethyllysine. In this connection "side chain" refers to the residue that is attached to the alpha-carbon atom, e.g. methyl in case of an Ala side chain or benzyl in case of a Phe side chain.
The term "unnatural alpha amino acid side chain" for the purpose of the present invention refers to all side chains of known alpha-amino acids that are not proteinogenic nor are known to occur naturally (i.e. in any biological systems). Examples are norleucine, cyclohexylglycine, 2-naphthylalanine, substituted alpha-amino acids (e.g. halogen substituted Tyr or Phe) as well as protected alpha-amino acid side chains, where a protection group such as Fmoc, Boc, Z, CBZ, Aloc, trityl, acetyl and/or benzyl is directly attached/reacted to a functionalization (e.g. amino, hydroxy and/or carboxy residue). In this connection "side chain" is referred to as for "natural alpha amino acid side chains".

Above embodiments of radicals R1 to R10 that possess functionalization (e.g. amino, hydroxy and/or carboxy residues), such as alkyl-CO-NH₂, -alkyl-CO-OH, - alkyl-NH₂, -alkyl-NH-C(NH)-NH₂,-CO-C*(R9R10)-NH₂, -CO-CH₂-C*(R9R10)-NH₂, -CO-C*(R9R10)-CH₂-NH₂ and/or 2-amino-2-carbonyl-propane (2-amino-isobutyric acid/Aib residue), may be protected with protection groups as mentioned above. Such protection group carrying embodiments are regarded as belonging to/ within the scope and spirit of the invention.

In a preferred embodiment, the process according to above aspects and embodiments is provided, wherein the silver-compound in step (d) is selected from the group consisting of: "silver salts, silver acetate, silver benzoate, silver oxide" and preferably is silver benzoate.

In a further preferred embodiment, the process according to above aspects and embodiments is provided, wherein
the coupling reagent in step (a) and step (f) is independently from each other selected from the group consisting of: "benzotriazol-1-yl-oxy-tris-(dimethylamino)-phosphonium hexafluorphosphate (BOP), N-ethyl-N'-(3-dimethylaminopropyl) carbodiimide hydrochloride (EDC), 2-(1H-benzotriazol-1-yl)-1,1,3,3,-tetramethyluronoium hexafluorphosphate (HBTU)";
the base in step (a) and step (f) is an organic base and is independently from each other selected from the group consisting of: "N-methyl-morpholine, diisopropylethylamine";
the thionating reagent in step (b) is selected from the group consisting of: "Lawesson's reagent";
the acid in step (d), step (e) and step (g) is independently from each other an organic acid and is preferably selected from the group consisting of: "carboxylic acid, trifluoroacetic acid (TFA), TFA in the presence of anisole and thioanisole, hydrochloric acid, acetic acid";
the reducing reagent in step (f) is selected from the group consisting of: "NaBH₃CN, NaBH₄";
the solvent in steps (a) to (g) is an organic solvent and is preferably and independently from each other selected from the group consisting of: "dichloromethane (DCM), acetonitrile (ACN), ethanol, tetrahydrofuran (THF), dimethylether (DME), dimethylformamide (DMF)".

In a further preferred embodiment, the process according to above aspects and embodiments is provided, wherein
temperature Tₐ independently is room temperature (22° ± 4° C),
temperature T_{b} independently is between 75° C to 90° C, preferably 80° C or 85° C,
temperature T_{c} independently is between 75° C to 90° C, preferably 80° C or 85° C,
temperature T_{d} independently is room temperature (22° ± 4° C),
temperature Tₑ independently is room temperature (22° ± 4° C),
temperature T_{f} independently is room temperature (22° ± 4° C),
temperature Tg independently is room temperature (22° ± 4° C).

The process illustrated supra in above aspects and embodiments is advantageously **characterized in that** no toxic materials, such as toxic metals, in particular no Hg, are used. The process is further advantageously **characterized in that** through the use of silver compounds, in particular silver benzoate, the reaction time is significantly reduced, in particular of step (d) ("cyclization") from 3 days according to manufacturing procedures known from the prior art to 6 hours.

The object of the invention has been surprisingly solved in another aspect by providing a triazole compound selected from the group consisting of:

| **Compound No.** | **Structure** |
|---|---|
| **1** | 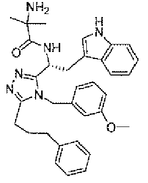 |
| **2** | 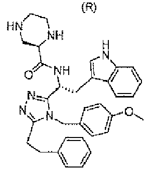 |
| **3** | 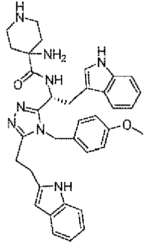 |
| **4** | 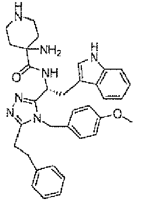 |
| **5** | 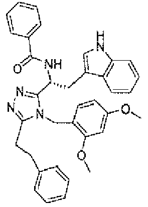 |
| **6** | 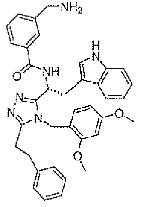 |
| **7** | 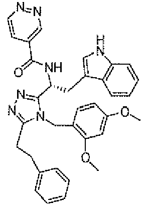 |
| **8** | 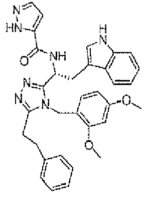 |
| **10** | 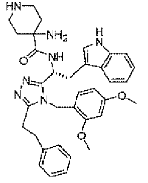 |
| **11** | 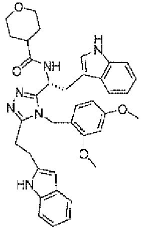 |
| **12** | 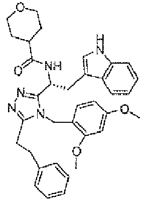 |
| **13** | 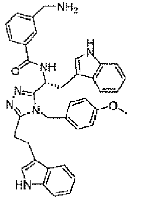 |
| **14** | 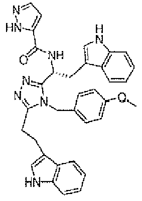 |
| **15** | 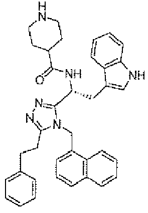 |
| **16** | 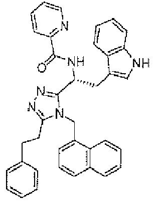 |
| **17** | 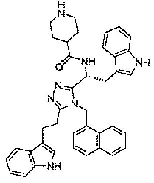 |
| **18** | 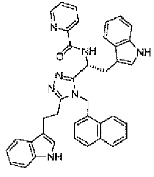 |
| **19** | 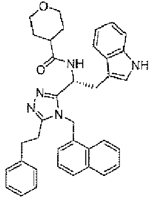 |
| **20** | 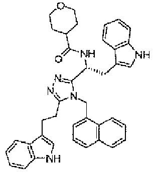 |
| **21** | 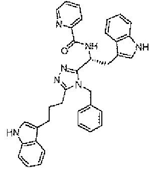 |
| **22** | 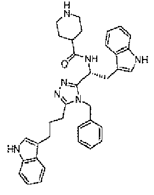 |
| **23** | 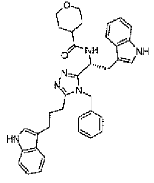 |
| **29** | 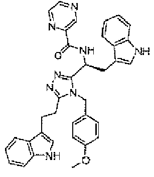 |
| **30** | 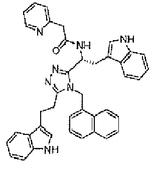 |
| **31** | 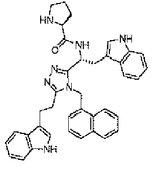 |
| **32** | 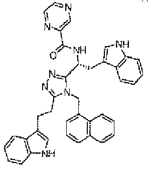 |
| **33** | 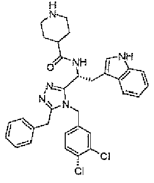 |
| **34** | 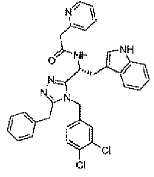 |
| **35** | 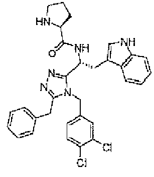 |
| **36** | 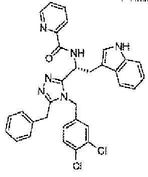 |
| **37** | 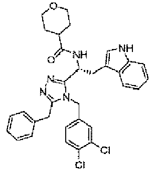 |
| **38** | 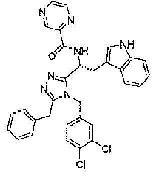 |
| **39** | 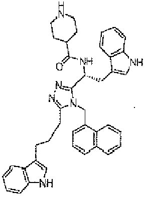 |
| **40** | 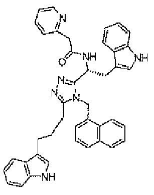 |
| **41** | 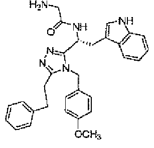 |
| **42** | 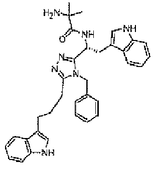 |
| **43** | 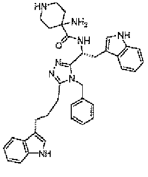 |
| **44** | 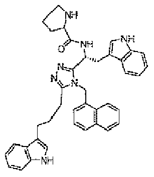 |
| **45** | 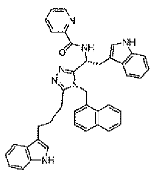 |
| **46** | 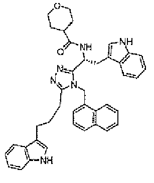 |
| **47** | 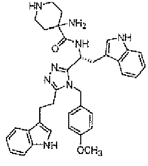 |
| **48** | 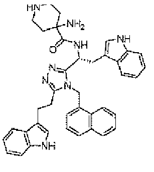 |
| **49** | 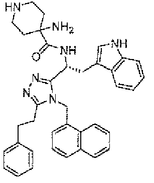 |
| **50** | 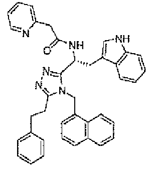 |
| **51** | 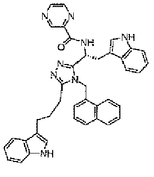 |
| **52** | 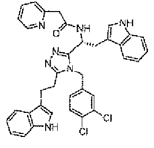 |
| **53** | 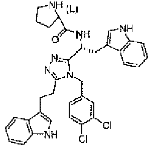 |
| **54** | 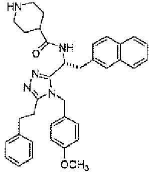 |
| **55** | 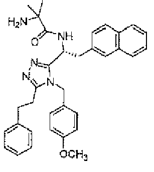 |
| **56** | 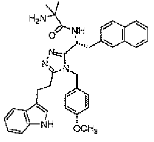 |
| **57** | 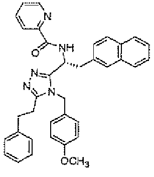 |
| **58** | 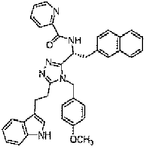 |
| **59** | 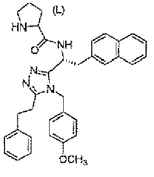 |
| **60** | 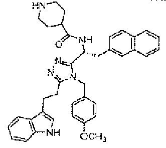 |
| **61** | 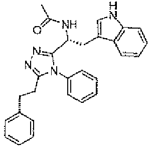 |
| **62** | 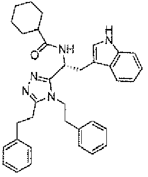 |
| **63** | 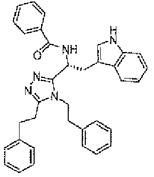 |
| **64** | 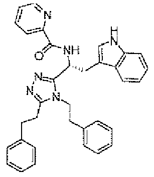 |
| **65** | 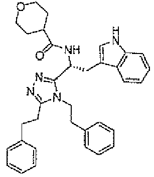 |
| **66** | 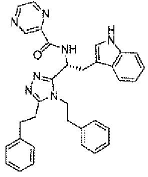 |
| **67** | 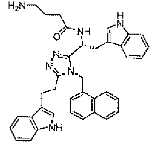 |
| **68** | 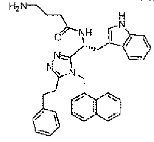 |
| **69** | 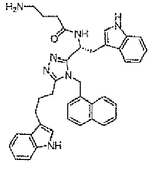 |
| **70** | 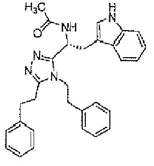 |
| **71** | 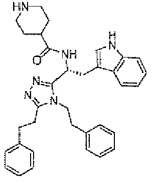 |
| **72** | 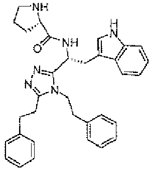 |
| **73** | 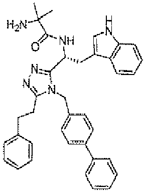 |
| **74** | 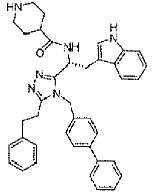 |
| **75** | 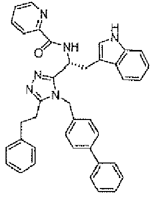 |
| **76** | 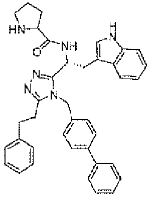 |
| **77** | 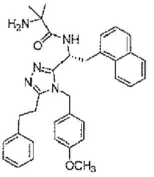 |
| **78** | 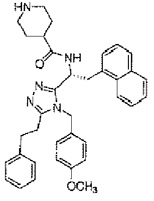 |
| **79** | 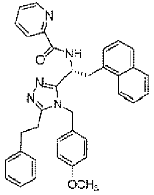 |
| **80** | 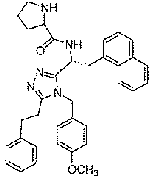 |
| **81** | 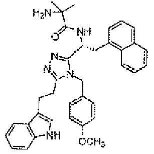 |
| **82** | 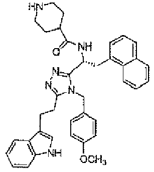 |
| **83** | 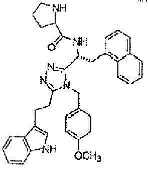 |
| **84** | 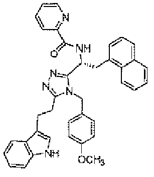 |
| **85** | 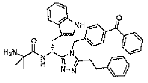 |
| **86** | 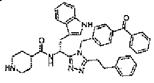 |
| **87** | 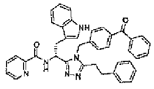 |
| **88** | 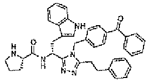 |

For the avoidance of doubt, if chemical name and chemical structure of the above illustrated compounds do not correspond by mistake, the chemical structure is regarded to unambigously define the compound.

In a preferred embodiment these compounds can be used for the manufacture of a medicament for the treatment or prophylaxis of physiological and/or pathophysiological conditions in mammals that are mediated by GHS receptors.

In a further preferred embodiment all triazole compounds as illustrated herein, in the following referred to as the compounds of the (present) invention, can be used for the manufacture of a medicament for the treatment or prophylaxis of physiological and/or pathophysiological conditions in mammals that are mediated by GHS receptors and where the treatment is achieved by modulation of GHS receptors.

In yet another preferred embodiment all compounds of the invention are antagonists of GHS receptors.
In yet a further preferred embodiment all compounds of the invention are agonists of GHS receptors.

All stereoisomers of the compounds of the invention are contemplated, either in a mixture or in pure or substantially pure form. The compounds of the present invention can have asymmetric centers at any of the carbon atoms including any one of the R radicals. Consequently, compounds of the invention can exist in the form of their racemates, in the form of the pure enantiomers and/or diastereomers or in the form of mixtures of these enantiomers and/or diastereomers. The mixtures may have any desired mixing ratio of the stereoisomers. All these different stereochemical forms and mixtures are within the scope of the present invention.

Thus, for example, the compounds of the invention which have one or more centers of chirality and which occur as racemates or as diastereomer mixtures can be fractionated by methods known per se into their optical pure isomers, i.e. enantiomers or diastereomers. The separation of the compounds of the invention can take place by column separation on chiral or nonchiral phases or by recrystallization from an optionally optically active solvent or with use of an optically active acid or base or by derivatization with an optically active reagent such as, for example, an optically active alcohol, and subsequent elimination of the radical.

Where possible, the compounds of the invention may be in the form of the tautomers.

It is likewise possible for the compounds of the invention to be in the form of any desired prodrugs such as, for example, esters, carbonates or phosphates, in which cases the actually biologically active form is released only through metabolism. Any compound that can be converted in vivo to provide the bioactive agent (i.e. a compound of the invention) is a prodrug within the scope and spirit of the invention.

Various forms of prodrugs are well known in the art and are described for instance in:
(i) The Practice of Medicinal Chemistry (Wermuth CG et al., Chapter 31, Academic Press 1996);
(ii) Design of Prodrugs (editor: Bundgaard H, Elsevier 1985); and
(iii) A Textbook of Drug Design and Development (Krogsgaard-Larson P and Bundgaard H, eds., Chapter 5: 113 - 191, Harwood Academic Publishers 1991).
   Said references are incorporated herein by reference.
   It is further known that chemical substances are converted in the body into metabolites which may where appropriate likewise elicit the desired biological effect - in some circumstances even in more pronounced form.
   Any biologically active compound that was converted in vivo by metabolism from any compound of the invention is a metabolite within the scope and spirit of the invention.

The compounds of the invention can, if they have a sufficiently basic group such as, for example, a primary, secondary or tertiary amine, be converted with inorganic and organic acids into salts. The pharmaceutically acceptable salts of the compounds of the invention are preferably formed with hydrochloric acid, hydrobromic acid, iodic acid, sulfuric acid, phosphoric acid, methanesulfonic acid, p-toluenesulfonic acid, carbonic acid, formic acid, acetic acid, sulfoacetic acid, trifluoroacetic acid, oxalic acid, malonic acid, maleic acid, succinic acid, tartaric acid, racemic acid, malic acid, embonic acid, mandelic acid, fumaric acid, lactic acid, citric acid, taurocholic acid, glutaric acid, stearic acid, glutamic acid or aspartic acid. The salts which are formed are, inter alia, hydrochlorides, chlorided, hydrobromides, bromides, iodides, sulfates, phosphates, methanesulfonates, tosylates, carbonates, bicarbonates, formates, acetates, sulfoacetates, triflates, oxalates, malonates, maleates, succinates, tartrates, malates, embonates, mandelates, fumarates, lactates, citrates, glutarate, stearate, aspartates and glutamates. The stoichiometry of the salts formed from the compounds of the invention may moreover be an integral or non-integral multiple of one.

The compounds of the invention can, if they contain a sufficiently acidic group such as, for example, the carboxy, sulfonic acid, phosphoric acid or a phenolic group, be converted with inorganic and organic bases into their physiologically tolerated salts. Examples of suitable inorganic bases are ammonium, sodium hydroxide, potassium hydroxide, calcium hydroxide, and of organic bases are ethanolamine, diethanolamine, triethanolamine, ethylenediamine, t-butylamine, t-octylamine, dehydroabietylamine, cyclohexylamine, dibenzylethylene-diamine and lysine. The stoichiometry of the salts formed from the compounds of the invention can moreover be an integral or non-integral multiple of one.

It is likewise possible for the compounds of the invention to be in the form of their solvates and, in particular, hydrates which can be obtained for example by crystallization from a solvent or from aqueous solution. It is moreover possible for one, two, three or any number of solvate or water molecules to combine with the compounds of the invention to give solvates and hydrates.

It is known that chemical substances form solids which exist in different order states which are referred to as polymorphic forms or modifications. The various modifications of a polymorphic substance may differ greatly in their physical properties. The compounds of the invention can exist in various polymorphic forms, and certain modifications may moreover be metastable. All these polymorphic forms of the compounds of the invention are to be regarded as belonging to the invention.

The triazole derivatives (compounds of the invention) as illustrated herein are ghrelin analogue ligands of GHS receptors. Thus, the aforementioned compounds of the invention are suitable for the treatment or prophylaxis of physiological and/or pathophysiological conditions mediated by GHS receptors and/or physiological and/or pathophysiological conditions which can be influenced by modulation of these receptors, and thus prevented, treated and/or alleviated.

For the purpose of the present invention, the term "treatment" is also intended to include prophylactic treatment or alleviation.

The term "ghrelin analogue ligand" or "ligand" is intended to refer for the purposes of the present invention to every compound which binds in any way to a receptor (the receptors in the present invention being GHS receptors) and induces either activation, inhibition and/or another conceivable effect at this receptor. The term "ghrelin analogue ligand" or "ligand" thus includes agonists, antagonists, partial agonists/antagonists, inverse agonists and other ligands which cause an effect at the receptor which is similar to the effect of agonists, antagonists, partial agonists/antagonists or inverse agonist.

For the purpose of the present invention, the term "GHS receptor antagonist" or "antagonist of GHS receptors" refers to compounds of the invention that bind to GHS receptors but do not elicit a proper activation of the receptors as assessed by recording an increase of intracellular calcium which is characteristic for activation of G-protein coupled receptors (GPCRs).

The ability to properly activate the receptors is assessed for any compound of the invention by comparing the degree of activation (increase of intracellular calcium) of GHS-R 1 a by the compound to be tested (at 10⁻⁶ M concentration) to the degree of activation (increase of intracellular calcium) of GHS-R 1a by 10⁻⁶ M ghrelin (100%) and to the basal level (0%). Such assessment can be readily performed by the skilled artisan due to his expert knowledge. The output is a percentage value for each compound to be tested.

Any compound of the invention that does not show a degree of activation (increase of intracellular calcium) of GHS-R 1a of at least 20 % as assessed in accordance with above specification is regarded as not eliciting a proper activation and therefore as GHS receptor antagonist. Preferably such compounds do show an antagonizing effect (counteraction/decrease) on ghrelin and/or other GHS stimulated intracellular calcium increase, prevent such stimulation or even act as inverse agonists (an inverse agonists is an ligand which binds to the same receptor binding-site as an agonist or antagonist but causes an inhibition of the basal/constitutive activity of the receptor, in principle an agonists with a negative intrinsic activity). Such compounds may furthermore exhibit an inhibitory activity on GH secretion and/or on other physiological or pathophysiological conditions or effects, such as food intake or lipogenesis. Their effects may be dissociated. Thus, they may have no impact at all on GH secretion while inhibiting other physiological effects. They may even stimulate other physiological effects.

For the purpose of the present invention, the term "GHS receptor agonist" or "agonist of GHS receptors" refers to compounds of the invention that bind to GHS receptors and elicit a proper activation of the receptor as assessed by recording an increase of intracellular calcium which is characteristic for activation of G-protein coupled receptors.

Any compound of the invention that shows a degree of activation (increase of intracellular calcium) of GHS-R 1a of at least 20 % as assessed in accordance with above specification is regarded as eliciting a proper activation and therefore as GHS receptor agonist. Such compounds may mimic the effects of ghrelin and/or GHS on GH secretion and for instance food intake or lipogenesis. Like for antagonists, the effects of agonist compounds may be dissociated from the GH secretory effect. Such compounds may even antagonize (counteract/decrease) ghrelin and/or other GHS stimulated intracellular calcium increase.

The term "GHS receptor" or "GHS-R" is intended to comprise for the purposes of the present invention receptors that bind at least one known peptidyl and/or non-peptidyl GHS and/or ghrelin. The term "GHS receptor" or "GHS-R" is also intended to comprise different GHS binding sites in the various tissues and/or organs as illustrated herein, that bind at least one known peptidyl and/or non-peptidyl GHS and/or ghrelin and which are probably not yet characterized GHS-R subtypes.

Binding of a given known peptidyl and/or non-peptidyl GHS and/or ghrelin can be easily verified by the skilled artisan on the basis of his expert knowledge, e.g. by appropriate binding assays which represent only routine experimentation.

Such GHS receptors may be stimulated/activated by ghrelin (ghrelin responsive) or may not be stimulated/activated by ghrelin (ghrelin non-responsive) - with regard to both acylated and non-acylated ghrelin, respectively. Stimulation/activation of such receptors may cause but does not compulsorily have to elicit GH production and/or GH secretion and/or increase GH plasma levels.

Preferably such GHS receptors are selected from the group consisting of "GHS type 1 receptor, GHS-R 1a, GHS-R 1b, motilin receptor, motilin receptor 1a, neurotensin receptor, TRH receptor, GPR38 (FM1), GPR39 (FM2), FM3, GHS binding site, GHS-R subtype, cardiac GHS-R, mammary GHS-R".

More preferably, such GHS receptors are selected from the group consisting of "GHS type 1 receptor, GHS-R 1a, G HS-R 1b" and most preferably are GHS-R 1a.

As discussed herein, GHS receptors (including GHS binding sites and GHS-R subtypes) are known to be concentrated in the hypothalamus-pituitary area but also appear to be distributed in other central and peripheral tissues. Furthermore, they are also expressed in various tumoral tissues, even in tumoral tissues from organs that do not express these receptors under physiological conditions.

For the purposes of the present invention, all these GHS receptor (including GHS binding sites and GHS-R subtypes) expressing organs and/or tissues are intended to be comprised by the scope of the present invention. Expression of GHS receptors (including GHS binding sites and GHS-R subtypes) in a given organ and/or tissue can be easily verified by the skilled artisan on the basis of his expert knowledge, e.g. by appropriate molecular biologic assays, such as immunofluorescence or immunoprecipitation assays, which represent only routine experimentation.

Preferably, such GHS receptors are located in tissues and/or organs selected from the group consisting of "endocrine tissue, exocrine tissue, peripheral tissue, adipose/fat tissue, brain, hypothalamus, thalamus, hippocampus, striatum, cortex, pituitary, central nervous system, spinal cord, gland, adrenal gland, thyroid gland, salivary gland, mammary gland, neuron, bowel, intestine, stomach, heart, liver, pancreas, kidney, bile, gall, bladder, prostate, spleen, muscle, skeletal muscle, aorta, artery, vein, immune cell, leukocyte, lymphocyte, T cell, B cell, granulocyte, monocyte, macrophage, dendritic cell, mast cell, NK cell, neutrophil, eosinophil, basophil, lymph node, bone, bone marrow, tonsil, thymus, placenta, testes, ovary, uterus, lung, adipocyte, tumor/cancer cell, carcinoma cell, prostate cancer cell, thyroid cancer cell, lung cancer cell, breast cancer cell".

As illustrated supra, the compounds of the invention are ghrelin analogue ligands of GHS receptors. They can be administered to various mammalian species, including human, for the treatment or prophylaxis of physiological and/or pathophysiological condition in such mammals.
For the purpose of the present invention, all mammalian species are regarded as being comprised. Preferably, such mammals are selected from the group consisting of "human, domestic animals, cattle, livestock, pets, cow, sheep, pig, goat, horse, pony, donkey, hinny, mule, hare, rabbit, cat, dog, guinea pig, hamster, rat, mouse". More preferably, such mammals are human.

The compounds of the invention being non-peptidic ghrelin analogue ligands of GHS receptors are surprisingly characterized by a strong binding affinity to such receptors. Such compounds for instance may preferably exhibit an IC₅₀ value of less than 1000 nM for binding to GHS-R 1 a. More preferably, such compounds may exhibit an IC₅₀ value of less than 500 nM, even more preferably of less than 300 nM and most preferably of less than 100 nM for binding to GHS-R 1 a.

Due to their surprisingly strong receptor binding, the compounds of the invention can be advantageously administered at lower doses compared to other less potent binders while still achieving equivalent or even superior desired biological effects. In addition, such a dose reduction may advantageously lead to less or even no medicinal adverse effects. Further, the high binding specificity of the compounds of the invention may translate into a decrease of undesired side effects on its own regardless of the dose applied.

Furthermore, the compounds of the invention, being of non-peptidic nature, are resistant to degradation by enzymes of the gastro-intestinal tract. Hence, they offer the advantage to be given by oral route. They surprisingly display an improved metabolic stability and/or an improved bioavailability. Hence, again an advantageous dose reduction may be achievable which may cause less or even no side effects.

The compounds of the invention can either be antagonists or agonists of GHS receptors as illustrated and defined herein.

GHS receptor antagonists of the present invention can for instance be employed for the inhibition of GHS receptors stimulated by ghrelin and/or other GHS thus decreasing and/or blocking GH production and/or secretion and/or GH plasma levels. In addition, such GHS receptor antagonists may also be employed for the inhibition or prevention of physiological or pathophysiological effects of ghrelin which are not related to GH production and/or GH secretion.

Therefore, GHS receptor antagonists of the present invention are suitable for the treatment and/or prophylaxis of various physiological and pathophysiological conditions as disclosed herein, in particular for the short-, medium- and/or long term regulation of energy balance, the short-, medium- and/or long term regulation (stimulation and/or inhibition) of food intake, the treatment of adipogenesis, adiposity and/or obesity, body weight gain and/or reduction and the treatment of tumor cell proliferation.

In contrast, GHS receptor agonists of the present invention can for instance be employed for the activation of GHS receptors and stimulation/increase of GH production and/or GH secretion and would thus have similar effects or uses as growth hormone itself, ghrelin and/or known GHS.

Thus, GHS receptor agonists of the present invention are suitable for the treatment and/or prophylaxis of various physiological and pathophysiological conditions as disclosed herein, in particular for growth retardation, cachexia, inflammation, inflammatory effects, gastric postoperative ileus, postoperative ileus and/or gastrectomy (ghrelin replacement therapy).

For the purpose of the present invention, all physiological and/or pathophysiological conditions are intended to be comprised that are known to be mediated by GHS receptors.

Preferably, these physiological and/or pathophysiological conditions are selected from the group consisting of "acute fatigue syndrome and muscle loss following election surgery, adipogenesis, adiposity, age-related decline of thymic function, age-related functional decline ("ARFD") in the elderly, aging disorder in companion animals, Alzheimer's disease, anorexia (e.g.associated with cachexia or aging); anxiety, blood pressure (lowering), body weight gain/reduction, bone fracture repair (acceleration), bone remodeling stimulation, cachexia and protein loss reduction due to chronic illness such as cancer or AIDS, cardiac dysfunctions (e.g. associated with valvular disease, myocarial infarction, cardiac hypertrophy or congestive heart failure), cardiomyopathy, cartilage growth stimulation, catabolic disorders in connection with pulmonary dysfunction and ventilator dependency, catabolic side effects of glucocorticoids, catabolic state of aging, central nervous system disorders (in combination with antidepressants), chronic dialysis, chronic fatigue syndrome (CFS), cognitive function improvement (e.g. in dementia, Alzheimer's disease), complicated fractures (e.g. disctraction osteogenesis), complications associated with transplantation, congestive heart failure (alone/in combination with corticotropin releasing factor antagonists), Crohn's disease and ulcerative colits, Cushing's syndrome, dementia, depressions, short-, medium- and/or long-term regulation of energy balance, short-, medium- and/or long-term regulation of food intake (stimulation and/or inhibition), fraility (e.g. in elderly humans), gastrectomy (ghrelin replacement therapy), gastric postoperative ileus, glycemic control improvement, growth hormone release stimulation in the elderly, growth hormone replacement in stressed patients, growth promotion in livestock, growth retardation associated with the Prader-Willi syndrome and Turner's syndrome, growth retardation in connection with Crohn's disease, growth retardation, hair/nail growth maintenance, hip fractures, hunger, hypercortisolism, hyperinsulinemia including nesidioblastosis, hypothermia, immune deficiency in individuals with a depressed T4/T8 cell ratio, immune response improvement to vaccination, immune system stimulation in companion animals, immune system stimulation, immunosuppression in immunosuppressed patients, inflammation or inflammatory effects, inflammatory bowel disease, insulin resistance in the heart, insulin resistance in type 2 diabetic patients, insulin resistance including NIDDM, diabetes, diabetes type I, diabetes type II, intrauterine growth retardation, irritable bowel syndrome, lipodystrophy (e.g. HIV-induced), metabolic homeostasis maintenance, milk production increase in livestock, muscle mass/strength increase, muscle mobility improvement, muscle strength improvement, muscle strength/function maintenance in elderly humans, muscular atrophy, musculoskeletal impairment (e.g. in elderly), Noonan's syndrome, obesity and growth retardation associated with obesity, osteoblast stimulation, osteochondrodysplasias, osteoporosis, ovulation induction (adjuvant treatment), physiological short stature including growth hormone deficient children, postoperative ileus, protein catabolic response attenuation after major surgery/trauma, protein kinase B activity enhancement, psychosocial deprivation, pulmonary dysfunction and ventilator dependency, pulmonary function improvement, pulsatile growth hormone release induction, recovery of burn patients and reducing hospitalization of burn patients (acceleration), renal failure or insufficiency resulting from growth retardation, renal homeostasis maintenance in the frail elderly, sarcopenia, schizophrenia, sensory function maintenance (e.g. hearing, sight, olefaction and taste), short bowel syndrome, short stature associated with chronic illness, skeletal dysplasia, skin thickness maintenance, sleep disorders, sleep quality improvement, thrombocytopenia, thymic development stimulation, tooth repair or growth, tumor cell proliferation, ventricular dysfunction or reperfusion events, wasting in connection with AIDS, wasting in connection with chronic liver disease, wasting in connection with chronic obstructive pulmonary disease (COPD), wasting in connection with multiple sclerosis or other neurodegenerative disorders, wasting secondary to fractures, wool growth stimulation in sheep, wound healing (acceleration), wound healing delay". More preferably these physiological and/or pathophysiological conditions are selected from the group consisting of "growth retardation, cachexia, short-, medium- and/or long term regulation of energy balance; short-, medium- and/or long term regulation (stimulation and/or inhibition) of food intake; adipogenesis, adiposity and/or obesity; body weight gain and/or reduction; diabetes, diabetes type I, diabetes type II, tumor cell proliferation; inflammation, inflammatory effects, gastric postoperative ileus, postoperative ileus and/or gastrectomy (ghrelin replacement therapy)".

In a further aspect of the present invention, the compounds of the invention may be used in combination with at least one additional pharmacologically active substance.

Such additional pharmacologically active substance may be other compounds of the present invention and/or other "suitable therapeutic agents" useful in the treatment and/or prophylaxis of the aforementioned physiological and/or pathophysiological conditions. The additional pharmacologically active substance may be an antagonist of GHS receptors and/or an agonist of GHS receptors depending on the purpose of the combined use. Selection and combination of the additional pharmacologically active substance(s) can be easily performed by the skilled artisan on the basis of his expert knowledge and depending on the purpose of the combined use and physiological and/or pathophysiological conditions targeted.

In a preferred embodiment, the compounds of the invention are used for the treatment and/or prophylaxis of the aforementioned physiological and/or pathophysiological conditions in the form of a medicament, where such medicament comprises at least one additional pharmacologically active substance.

In another preferred embodiment, the compounds of the invention are used for the treatment and/or prophylaxis of the aforementioned physiological and/or pathophysiological conditions in the form of a medicament, where the medicament is applied before and/or during and/or after treatment with at least one additional pharmacologically active substance.

The above mentioned "suitable therapeutic agents" include: "GHS, anti-diabetic agents; anti-osteoporosous agents; anti-obesity agents; anti-inflammatory agents; anti-anxiety agents; anti-depressants; anti-hypertensive agents; anti-platelet agents; antithrombotic and thrombolytic agents; cardiac glycosides; cholesterol/lipid lowering agents; mineralocorticoid receptor antagonists; phospodiesterase inhibitors; protein tyrosine kinase inhibitors; thyroid mimetics (including thyroid receptor antagonists); anabolic agents; HIV or AIDS therapies; therapies useful in the treatment of Alzheimer's disease and other cognitive disorders; therapies useful in the treatment of sleeping disorders; anti-proliferative agents; anti-tumor agents; anti-ulcer and gastroesopheageal reflux disease agents; progestin receptor agonists ("PRA");estrogen; testosterone; a selective estrogen receptor modulator; a selective androgen receptor modulator; parathyroid hormone; and/or bisphosphonate", and preferably, a "suitable therapeutic agents" is selected of the group consisting of this agents.

Examples of suitable GHS for use in combination with the compounds of the present invention include GHRP-6, GHRP-1 as described in U.S. Patent No. 4,411,890; and publications WO 89/07110 and WO 89/07111 and B-HT920 or growth hormone releasing factor and its analogs or growth hormone and its analogs or somatomedins including IGF-1 and IGF-2 as well as GHS described in WO 01/96300.

Examples of suitable anti-diabetic agents for use in combination with the compounds of the present invention include biguanides (e.g. metformin), glucosidase inhibitors (e.g. acarbose), insulins (including insulin secretagogues or insulin sensitizers), meglitinides (e.g. repaglinide), sulfonylureas (e.g., glimepiride, glyburide and glipizide), biguanide/glyburide combinations (e.g., glucovance), thiozolidinediones (e.g. troglitazone, rosiglitazone and pioglitazone), PPAR-alpha agonists, PPAR-gamma agonists, PPAR alpha/gamma dual agonists, SGLT2 inhibitors, inhibitors of fatty acid binding protein (aP2) such as those disclosed in US patent 6,548,529, glucagon-like peptide-1 (GLP-1), and dipeptidyl peptidase IV (DP4) inhibitors.

Examples of suitable anti-osteoporosous agents for use in combination with the compounds of the present invention include alendronate, risedronate, raloxifene, calcitonin, non-steroidal progestin receptor agonists, RANK ligand agonists, calcium sensing receptor antagonists, TRAP inhibitors, selective estrogen receptor modulators (SERM), estrogen and AP-1 inhibitors.

Examples of suitable anti-obesity agents for use in combination with the compounds of the present invention include endocannabinoid receptor antagonists, e.g. CB1 receptor antagonists such as rimonabant (1H-Pyrazole-3-carboxamide, 5-(4-chlorophenyl)-1-(2,4-dichlorophenyl)-4-methyl-N-1-piperidinyl-, monohydrochloride; CAS Registry Number: 158681-13-1; SR-141716A; US patent 5,624,941), aP2 inhibitors such as those disclosed in US patent 6,548,529, PPAR gamma antagonists, PPAR delta agonists, and orlistat.

Examples of suitable antinflammatory agents for use in combination with the compounds of the present invention include prednisone, dexamethasone, Enbrel, cyclooxygenase inhibitors (i.e., COX-1 and/or COX-2 inhibitors such as NSAIDs, aspirin, indomethacin, ibuprofen, piroxicam, Naproxen, Celebrex, Vioxx), CTLA4-Ig agonists/antagonists, CD40 ligand antagonists, integrin antagonists, alpha4 beta7 integrin antagonists, cell adhesion inhibitors, interferon gamma antagonists, ICAM-1, tumor necrosis factor (TNF) antagonists (e.g., infliximab, OR1384), prostaglandin synthesis inhibitors, budesonide, clofazimine, CNI-1493, CD4 antagonists (e.g., priliximab), p38 mitogen-activated protein kinase inhibitors, protein tyrosine kinase (PTK) inhibitors, IKK inhibitors, and therapies for the treatment of irritable bowel syndrome (e.g., zelmac and Maxi-K openers such as those disclosed in US Patent No. 6,184,231).

Examples of suitable anti-anxiety agents for use in combination with the compounds of the present invention include diazepam, lorazepam, buspirone, oxazepam, and hydroxyzine pamoate.

Examples of suitable anti-depressants for use in combination with the compounds of the present invention include citalopram, fluoxetine, nefazodone, sertraline, and paroxetine.

Examples of suitable anti-hypertensive agents for use in combination with the compounds of the present invention include beta adrenergic blockers, calcium channel blockers (L-type and T-type; e.g. diltiazem, verapamil, nifedipine, amlodipine and mybefradii), diruetics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamtrenene, amiloride, spironolactone), renin inhibitors, ACE inhibitors (e.g., captopril, zofenopril, fosinopril, enalapril, ceranopril, cilazopril, delapril, pentopril, quinapril, ramipril, lisinopril), AT-1 receptor antagonists (e.g., losartan, irbesartan, valsartan), ET receptor antagonists (e.g., sitaxsentan, atrsentan and compounds disclosed in U.S. Patent Nos. 5,612,359 and 6,043,265, Dual ET/All antagonist (e.g., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Examples of suitable anti-platelet agents for use in combination with the compounds of the present invention include GPIIb/IIIa blockers (e.g., abciximab, eptifibatide, tirofiban), P2Y12 antagonists (e.g., clopidogrel, ticlopidine, CS-747), thromboxane receptor antagonists (e.g., ifetroban), aspirin, and PDE-III inhibitors (e.g., dipyridamole) with or without aspirin.

Examples of suitable cardiac glycosides for use in combination with the compounds of the present invention include digitalis and ouabain.

Examples of suitable cholesterol/lipid lowering agents for use in combination with the compounds of the present invention include HMG-CoA reductase inhibitors [e.g., pravastatin lovastatin, atorvastatin, simvastatin, NK-104 (a.k.a. itavastatin, or nisvastatin or nisbastatin] and ZD-4522 (a.k.a. rosuvastatin, or atavastatin or visastatin)), squalene synthetase inhibitors, fibrates, bile acid sequestrants, ACAT inhibitors, MTP inhibitors, lipooxygenase inhibitors, choesterol absorption inhibitors, and cholesterol ester transfer protein inhibitors (e.g., CP-529414).

Examples of suitable mineralocorticoid receptor antagonists for use in combination with the compounds of the present invention include spironolactone and eplerinone.

Examples of suitable phospodiesterase inhibitiors for use in combination with the compounds of the present invention include PDE III inhibitors such as cilostazol, and PDE V inhibitors such as sildenafil.

Examples of suitable thyroid mimetics for use in combination with the compounds of the present invention include thyrotropin, polythyroid, KB-130015, and dronedarone.

Examples of suitable anabolic agents for use in combination with the compounds of the present invention include testosterone and SARMs.

Examples of suitable HIV or AIDS therapies for use in combination with the compounds of the present invention include indinavir sulfate, saquinavir, saquinavir mesylate, amprenavir, ritonavir, lopinavir, ritonavir/lopinavir combinations, lamivudine, zidovudine, lamivudine/zidovudine combinations, zalcitabine, didanosine, stavudine, and megestrol acetate.

Examples of suitable therapies for treatment of Alzheimer's disease and cognitive disorders for use in combination with the compounds of the present invention include donepezil, tacrine, revastigmine, 5HT6, gamma secretase inhibitors, beta secretase inhibitors, SK channel blockers, Maxi-K blockers, and KCNQs blockers.

Examples of suitable therapies for treatment of sleeping disorders for use in combination with the compounds of the present invention include melatonin analogs, melatonin receptor antagonists, ML1B agonists, and GABA/NMDA receptor antagonists.

Examples of suitable anti-proliferative agents for use in combination with the compounds of the present invention include cyclosporin A, taxol, FK 506, and adriamycin.

Examples of suitable anti-tumor agents for use in combination with the compounds of the present invention include taxol, adriamycin, epothilones, cisplatin and carboplatin.

Examples of suitable a selective estrogen receptor modulator for use in combination with the compounds of the present invention include tamoxifen and raloxifene.

Examples of suitable a selective androgen receptor modulator for use in combination with the compounds of the present invention include such disclosed in Edwards, J. P. et al., Bio. Med. Chem. Let., 9, 1003-1008 (1999) and Hamann, L. G. et al., J. Med. Chem., 12, 210-212 (1999).

Examples of suitable a bisphosphonate for use in combination with the compounds of the present invention include MK-217 (alendronate).

The above other therapeutic agents, when employed in combination with the compounds of the present invention, may be used, for example, in those amounts indicated in the Physicians' Desk Reference (PDR) or as otherwise determined by one of ordinary skill in the art.

In a preferred embodiment, the compounds of the invention are used for the treatment and/or prophylaxis of the aforementioned physiological and/or pathophysiological conditions in the form of a medicament, where such medicament comprises as additional pharmacologically active substance an endocannabinoid receptor antagonist, preferably a CB1 receptor antagonist, most preferably rimonabant (1H-Pyrazole-3-carboxamide, 5-(4-chlorophenyl)-1-(2,4-dichlorophenyl)-4-methyl-N-1-piperidinyl-, monohydrochloride; CAS Registry Number: 158681-13-1; SR-141716A; US patent 5,624,941) and as compound of the invention a GHS-R antagonist.

In another preferred embodiment, the compounds of the invention are used for the treatment and/or prophylaxis of the aforementioned physiological and/or pathophysiological conditions in the form of a medicament, where the medicament is applied before and/or during and/or after treatment with at least one additional pharmacologically active substance, where such additional pharmacologically active substance is an endocannabinoid receptor antagonist, preferably a CB1 receptor antagonist, most preferably rimonabant (1H-Pyrazole-3-carboxamide, 5-(4-chlorophenyl)-1-(2,4-dichlorophenyl)-4-methyl-N-1-piperidinyl-, monohydrochloride; CAS Registry Number: 158681-13-1; SR-141716A; US patent 5,624,941) and the compound of the invention is a GHS-R antagonist.

The compounds of the present invention can be administered in a known manner. The route of administration may thereby be any route which effectively transports the active compound to the appropriate or desired site of action, for example orally or non-orally, in particular topically, transdermally, pulmonary, rectally, intravaginally, nasally or parenteral or by implantation. Oral administration is preferred.

The compounds of the invention are converted into a form which can be administered and are mixed where appropriate with pharmaceutically acceptable carriers or diluents. Suitable excipients and carriers are described for example in Ullman's Encyclopedia of Technical Chemistry, Vol. 4, (1953), 1-39; Journal of Pharmaceutical Sciences, Vol. 52 (1963), 918 et seq.; H. v. Czetsch-Lindenwald, "Hilfsstoffe für Pharmazie and angrenzende Gebiete"; Pharm. Ind. 2, 1961, 72 et seq.; Dr. H.P. Fiedler, "Lexikon der Hilfsstoffe für Pharmazie, Kosmetik and angrenzende Gebiete", Cantor KG, Aulendorf in Württemberg, 1971.

Oral administration can take place for example in solid form as tablet, capsule, gel capsule, coated tablet, granulation or powder, but also in the form of a drinkable solution. The compounds of the invention can for oral administration be combined with known and ordinarily used, physiologically tolerated excipients and carriers such as, for example, gum arabic, talc, starch, sugars such as, for example, mannitol, methylcellulose, lactose, gelatin, surface-active agents, magnesium stearate, cyclodextrins, aqueous or nonaqueous carriers, diluents, dispersants, emulsifiers, lubricants, preservatives and flavorings (e.g. essential oils). The compounds of the invention can also be dispersed in a microparticulate, e.g. nanoparticulate, composition.

Non-oral administration can take place for example by intravenous, subcutaneous, intramuscular injection of sterile aqueous or oily solutions, suspensions or emulsions, by means of implants or by ointments, creams or suppositories. Administration as sustained release form is also possible where appropriate. Implants may comprise inert materials, e.g. biodegradable polymers or synthetic silicones such as, for example, silicone rubber. Intravaginal administration is possible for example by means of vaginal rings. Intrauterine administration is possible for example by means of diaphragms or other suitable intrauterine devices. Transdermal administration is additionally provided, in particular by means of a formulation suitable for this purpose and/or suitable means such as, for example, patches.

The dosage may vary within a wide range depending on type and/or severity of the physiological and/or pathophysiological condition, the mode of administration, the age, gender, bodyweight and sensitivity of the subject to be treated. It is within the ability of a skilled worker to determine a "pharmacologically effective amount" of a compound of the invention and/or additional pharmacologically active substance. Administration can take place in a single dose or a plurality of separate dosages.

A suitable unit dose is, for example, from 0.001 mg to 100 mg of the active ingredient, i.e. at least one compound of the invention and, where appropriate, at least one additional pharmacologically active substance, per kg of a patient's bodyweight.

In another aspect, the present invention relates to a pharmaceutical composition comprising a pharmacologically active amount of at least one triazole compound selected from the group consisting of: **compound 1, 2, 3, 4, 5, 6, 7, 8, 10, 11, 12, 13, 14, 15, 16, 17, 18, 19, 20, 21, 22, 23, 29, 30, 31, 32, 33, 34, 35, 36, 37, 38, 39, 40, 41, 42, 43, 44, 45, 46, 47, 48, 49, 50, 51, 52, 53, 54, 55, 56, 51, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87 and/or compound 88.**

In a further aspect, such a pharmaceutical composition may additionally comprise at least one pharmaceutically acceptable carrier and/or excipient and/or may comprise at least one further pharmacologically active substance.

In a preferred embodiment, such further pharmacologically active substance is an endocannabinoid receptor antagonist, preferably a CB1 receptor antagonist, most preferably rimonabant [1H-Pyrazole-3-carboxamide, 5-(4-chlorophenyl)-1-(2,4-dichlorophenyl)-4-methyl-N-1-piperidinyl-, monohydrochloride].

Concerning the pharmaceutical compositions of the invention, at least one of the triazole compounds as listed above is present in a pharmacologically effective amount, preferably in a unit dose, e.g. the aforementioned unit dose, specifically and preferably in an administration form which makes oral administration possible. Furthermore, reference may be made to that already said in connection with the possible uses and administrations of the compounds of the invention.

In another aspect, the present invention relates to a medicament comprising at least one compound of the invention as illustrated above or at least one pharmaceutical composition of the invention as illustrated supra.

In another aspect, the present invention relates to a medicament comprising at least one compound of the invention as illustrated above or at least one pharmaceutical composition of the invention as illustrated supra for use in the prophylaxis or treatment of physiological and/or pathophysiological conditions selected from the group consisting of "acute fatigue syndrome and muscle loss following election surgery, adipogenesis, adiposity, age-related decline of thymic function, age-related functional decline ("ARFD") in the elderly, aging disorder in companion animals, Alzheimer's disease, anorexia (e.g.associated with cachexia or aging); anxiety, blood pressure (lowering), body weight gain/reduction, bone fracture repair (acceleration), bone remodeling stimulation, cachexia and protein loss reduction due to chronic illness such as cancer or AIDS, cardiac dysfunctions (e.g. associated with valvular disease, myocarial infarction, cardiac hypertrophy or congestive heart failure), cardiomyopathy, cartilage growth stimulation, catabolic disorders in connection with pulmonary dysfunction and ventilator dependency, catabolic side effects of glucocorticoids, catabolic state of aging, central nervous system disorders (in combination with antidepressants), chronic dialysis, chronic fatigue syndrome (CFS), cognitive function improvement (e.g. in dementia, Alzheimer's disease), complicated fractures (e.g. disctraction osteogenesis), complications associated with transplantation, congestive heart failure (alone/in combination with corticotropin releasing factor antagonists), Crohn's disease and ulcerative colits, Cushing's syndrome, dementia, depressions, short-, medium- and/or long-term regulation of energy balance, short-, medium- and/or long-term regulation of food intake (stimulation and/or inhibition), fraility (e.g. in elderly humans), gastrectomy (ghrelin replacement therapy), gastric postoperative ileus, glycemic control improvement, growth hormone release stimulation in the elderly, growth hormone replacement in stressed patients, growth promotion in livestock, growth retardation associated with the Prader-Willi syndrome and Turner's syndrome, growth retardation in connection with Crohn's disease, growth retardation, hair/nail growth maintenance, hip fractures, hunger, hypercortisolism, hyperinsulinemia including nesidioblastosis, hypothermia, immune deficiency in individuals with a depressed T4/T8 cell ratio, immune response improvement to vaccination, immune system stimulation in companion animals, immune system stimulation, immunosuppression in immunosuppressed patients, inflammation or inflammatory effects, inflammatory bowel disease, insulin resistance in the heart, insulin resistance in type 2 diabetic patients, insulin resistance including NIDDM, diabetes, diabetes type I, diabetes type II, intrauterine growth retardation, irritable bowel syndrome, lipodystrophy (e.g. HIV-induced), metabolic homeostasis maintenance, milk production increase in livestock, muscle mass/strength increase, muscle mobility improvement, muscle strength improvement, muscle strength/function maintenance in elderly humans, muscular atrophy, musculoskeletal impairment (e.g. in elderly), Noonan's syndrome, obesity and growth retardation associated with obesity, osteoblast stimulation, osteochondrodysplasias, osteoporosis, ovulation induction (adjuvant treatment), physiological short stature including growth hormone deficient children, postoperative ileus, protein catabolic response attenuation after major surgery/trauma, protein kinase B activity enhancement, psychosocial deprivation, pulmonary dysfunction and ventilator dependency, pulmonary function improvement, pulsatile growth hormone release induction, recovery of burn patients and reducing hospitalization of burn patients (acceleration), renal failure or insufficiency resulting from growth retardation, renal homeostasis maintenance in the frail elderly, sarcopenia, schizophrenia, sensory function maintenance (e.g. hearing, sight, olefaction and taste), short bowel syndrome, short stature associated with chronic illness, skeletal dysplasia, skin thickness maintenance, sleep disorders, sleep quality improvement, thrombocytopenia, thymic development stimulation, tooth repair or growth, tumor cell proliferation, ventricular dysfunction or reperfusion events, wasting in connection with AIDS, wasting in connection with chronic liver disease, wasting in connection with chronic obstructive pulmonary disease (COPD), wasting in connection with multiple sclerosis or other neurodegenerative disorders, wasting secondary to fractures, wool growth stimulation in sheep, wound healing (acceleration) and/or wound healing delay".

In a preferred embodiment, the physiological and/or pathophysiological conditions are selected from the group consisting of "growth retardation, cachexia, short-, medium- and/or long term regulation of energy balance; short-, medium- and/or long term regulation (stimulation and/or inhibition) of food intake; adipogenesis, adiposity and/or obesity; body weight gain and/or reduction; diabetes, diabetes type I, diabetes type II, tumor cell proliferation; inflammation, inflammatory effects, gastric postoperative ileus, postoperative ileus and/or gastrectomy (ghrelin replacement therapy)".

The compounds of the invention were named from the drawn formula using the Chem Draw Ultra 8 software (CambridgeSoft Corporation, Cambridge, USA).

The contents of all cited references are hereby incorporated by reference in their entirety. The invention is explained in more detail by means of the following examples without, however, being restricted thereto.

### Examples

### I) Synthesis and physicochemical characterization of selected compounds of the invention

The compounds of the invention were synthesized according to the following syntheses schemes:

### Synthesis of 1,2,4-triazoles substituted with monomethoxybenzyl in position 4.

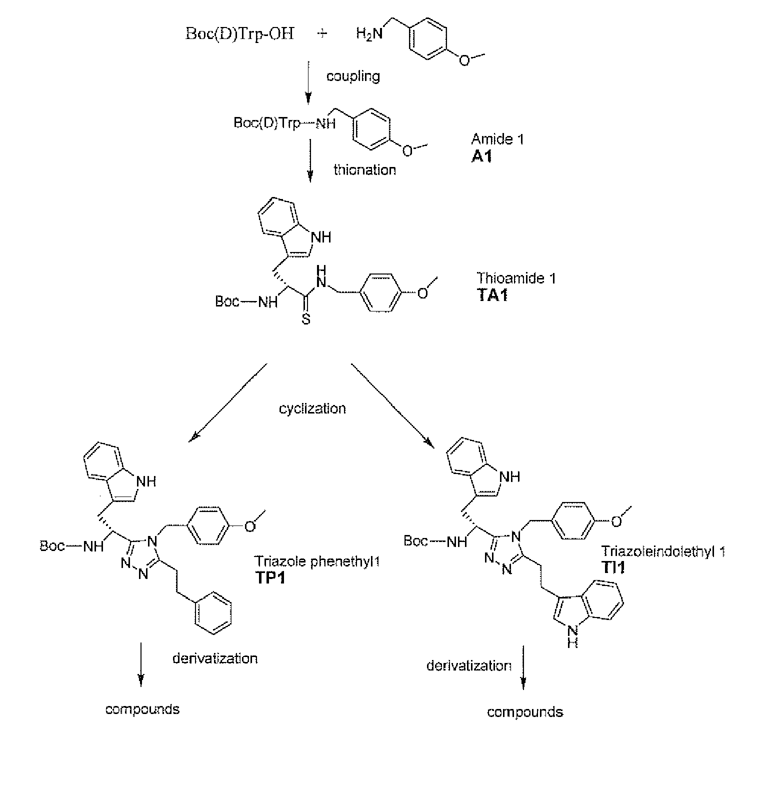

### Synthesis of 1,2,4-triazoles substituted with 2,4-dimethoxybenzyl in position 4.

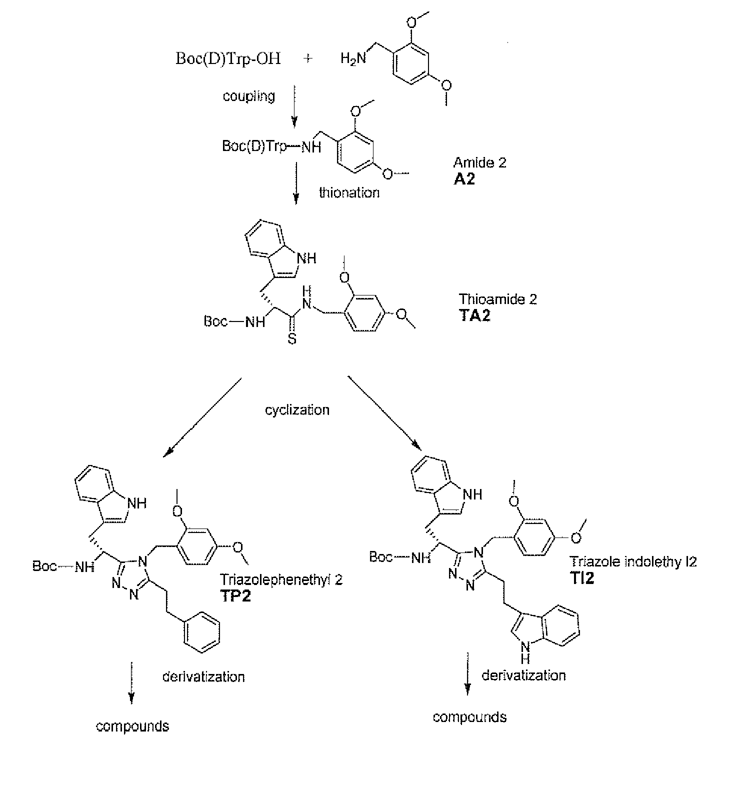

### Synthesis of 1,2,4-triazoles substituted with 1-naphtylmethyl in position 4.

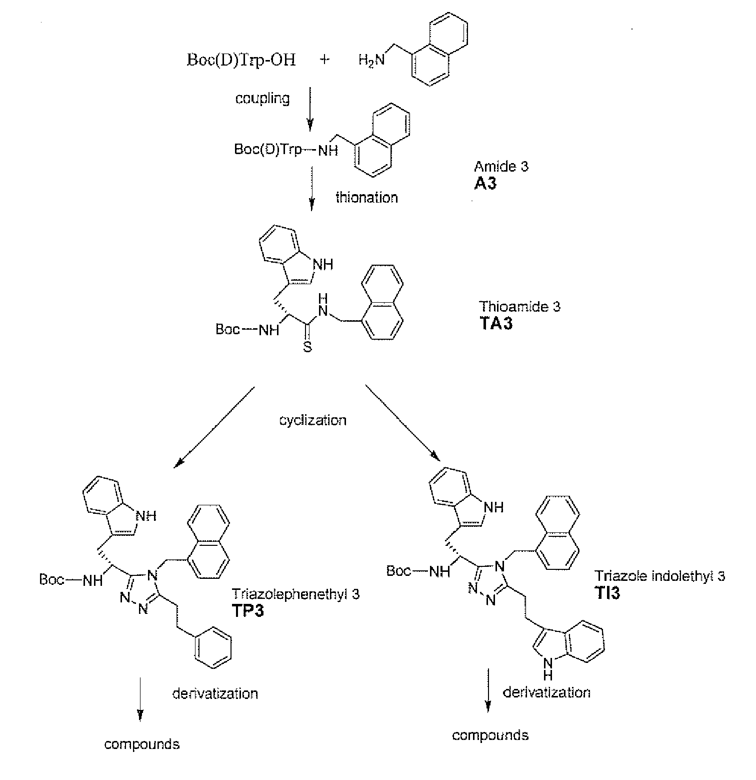

**General procedure for hydrazide preparation**. When hydrazides were not commercially available, they were synthesized in two steps via the corresponding esters as described below.

R-COOH → R-COOCH₃ → R-CO-NH-NH₂

**Preparation of the ester.** 1.0 equivalent carboxylic acid was dissolved in acetonitrile (0.5 mol/L). Then 1.2 equivalent DBU and 5.0 equivalent methyl iodide were added dropwise consecutively under stirring. After 8h under reflux, the solvent was removed in vacuo. The residue was diluted in dichloromethane, washed with aqueous potassium hydrogen sulfate (1M), saturated aqueous sodium hydrogen carbonate, and saturated aqueous sodium chloride. The organic layer was dried over sodium sulfate, filtered, and the solvent was removed *in vacuo* to afford the corresponding ester, as a colorless oil.

### Methyl 3-phenylpropanoate. 3.7 g (84%).

¹H NMR (300 MHz, DMSO-d₆): δ 2.58 (t, 2H, J = 7 Hz, CH₂-CH₂-phenyl), 2.84 (t, 2H, J = 7 Hz, CH₂-CH₂-phenyl), 3.55 (s, 3H, OCH₃), 7.21 (m, 5H, CH phenyl).

¹³C NMR (75 MHz, DMSO-d₆): δ 30.7 (CH₂-CH₂-phenyl), 35.3 (CH₂-CH₂-phenyl), 51.5 (OCH₃), 126.4 (C₄ phenyl), 128.5 (C₂ and C₆ phenyl), 128.7 (C₃ and C₅ phenyl), 140.9 (C₁ phenyl), 173.0 (CO ester).
MS (ES), *m*/*z*: 165.0 [M + H]⁺.

### Methyl 3-(1H-indol-3-yl)propanoate. 4.0 g (94%).

¹H NMR (300 MHz, DMSO-d₆): δ 2.64 (t, 2H, J = 8 Hz, CH₂-CH₂-indole), 2.94 (t, 2H, J = 8 Hz, CH₂-CH₂-indole), 3.55 (s, 3H, OCH₃), 6.95 (t, 1H, J = 7 Hz, H₅ indole), 7.04 (t, 1H, J = 7 Hz, H₆ indole), 7.08 (s, 1H, H₂ indole), 7.32 (d, 1H, J = 8 Hz, H₄ indole), 7.48 (d, 1H, J = 8 Hz, H₇ indole), 10.78 (brs, 1H, NH indole).
¹³C NMR (75 MHz, DMSO-d₆): δ 20.7 (CH₂-CH₂-indole), 34.7 (CH₂-CH₂-indole), 51.2 (OCH₃), 111.8 (C₇ indole), 113.5 (C₃ indole), 118.5 (C₄ and C₅ indole), 121.3 (C₆ indole), 122.7 (C₂ indole), 127.3 (C₉ indole), 136.6 (C₈ indole), 173.5 (CO ester).
MS (ES), *m*/*z*: 204.1 [M + H]⁺.

### Methyl 4-(1H-indol-3-yl)butanoate. 0.69 g (52%).

¹H NMR (300 MHz, DMSO-d₆): δ 1.87 (m, 2H, CH₂-CH₂-CH₂-indole), 2.32 (t, 2H, J = 7 Hz, CH₂-CH₂-CH₂-indole), 2.67 (t, 2H, J = 7 Hz, CH₂-CH₂-CH₂-indole), 3.55 (s, 3H, OCH₃), 6.94 (t, 1H, J = 7 Hz, H₅ indole), 7.03 (t, 1H, J = 7 Hz, H₆ indole), 7.07 (d, 1H, J = 2 Hz, H₂ indole), 7.31 (d, 1H, J = 8 Hz, H₄ indole), 7.47 (d, 1H, J = 8 Hz, H₇ indole), 10.74 (s, 1H, NH indole).
¹³C NMR (75 MHz, DMSO-d₆): δ 24.4 (CH₂-CH₂-CH₂-indole), 25.7 (CH₂-CH₂-CH₂-indole), 33.4 (CH₂-CH₂-CH₂-indole), 51,5 (OCH₃), 111.7 (C₇ indole), 114.1 (C₃ indole), 118.5 (C₄ indole), 118.6 (C₅ indole), 121.2 (C₆ indole), 122.7 (C₂ indole), 127.5 (C₉ indole), 136.5 (C₈ indole), 173.8 (CO ester).
MS (ES), *m*/*z*: 218.1 [M + H]⁺.

**Hydrazide preparation.** 1.0 equivalent of the corresponding ester and 10.0 equivalents hydrazine monohydrate were dissolved in ethanol (0.3 mol/L). The mixture was stirred overnight at reflux. The solvent was then removed in vacuo, the residue washed with cold diethylether and dried in vacuo to afford the corresponding hydrazide as a white powder.

### 3-phenylpropanehydrazide. 3.3 g (91%).

¹H NMR (300 MHz, DMSO-d₆): δ 2.28 (t, 2H, J = 7 Hz, CH₂-CH₂-phenyl), 2.77 (t, 2H, J = 7 Hz, CH₂-CH₂-phenyl), 3.69 (brs, 2H, NH₂), 7.11-7.26 (m, 5H, CH aromatic), 8.94 (brs, 1H, NH).
¹³C NMR (75 MHz, DMSO-d₆): δ 31.4 (CH₂-CH₂-phenyl), 35.5 (CH₂-CH₂-phenyl), 126.3 (C₄ phenyl), 128.6 (C₂ and C₆ phenyl), 128.7 (C₃ and C₅ phenyl), 140.1 (C₁ phenyl), 173.1 (CO).
MS (ES), *m*/*z*: 165.1 [M + H]⁺.

### 3-(1H-indol-3-yl)propanehydrazide. 3.1 g (76%).

¹H NMR (300 MHz, DMSO-d₆): δ 2.41 (m, 2H, CH₂-CH₂-indole), 2.90 (m, 2H, CH₂-CH₂-indole), 5.72 (brs, 2H, NH₂), 6.93 (t, 1H, J = 7 Hz, H₅ indole), 7.02 (t, 1H, J = 8 Hz, H₆ indole), 7.07 (s, 1H, H₂ indole), 7.31 (d, 1H, J = 8 Hz, H₄ indole), 7.48 (d, 1H, J = 8 Hz, H₇ indole), 9.03 (brs, 1H, NH hydrazide), 10.79 (brs, 1H, NH indole).
¹³C NMR (75 MHz, DMSO-d₆): δ 21.4 (CH₂-CH₂-indole), 34.7 (CH₂-CH₂-indole), 111.7 (C₇ indole), 114.1 (C₃ indole), 118.5 (C₄ indole), 118.7 (C₅ indole), 121.3 (C₆ indole), 122.5 (C₂ indole), 127.4 (C₉ indole), 136.6 (C₈ indole), 171.9 (CO).
MS (ES), *m*/*z*: 204.1 [M + H]⁺.

### 4-(1H-indol-3-yl)butanehydrazide. 1.0 g (90%).

¹H NMR (300 MHz, DMSO-d₆): δ 1.83 (m, 2H, CH₂-CH₂-CH₂-indole), 2.06 (t, 2H, J = 7 Hz, CH₂-CH₂-CH₂-indole), 2.62 (t, 2H, J = 8 Hz, CH₂-CH₂-CH₂-indole), 4.35 (brs, 2H, NH₂), 6.92 (t, 1H, J = 7 Hz, H₅ indole), 7.02 (t, 1H, J = 7 Hz, H₆ indole), 7.06 (s, 1H, H₂ indole), 7.29 (d, 1H, J = 8 Hz, H₄ indole), 7.46 (d, 1H, J = 8 Hz, H₇ indole), 8.93 (brs, 1H, NH hydrazine), 10.72 (s, 1H, NH indole).
¹³C RMN (75 MHz, DMSO-d₆): δ 24.8 (CH₂-CH₂-CH₂-indole), 26.5 (CH₂-CH₂-CH₂-indole), 33.7 (CH₂-CH₂-CH₂-indole), 111.7 (C₇ indole), 114.5 (C₃ indole), 118.5 (C₄ indole), 118.7 (C₅ indole), 121.2 (C₆ indole), 122.6 (C₂ indole), 127.6 (C₉ indole), 136.7 (C₈ indole), 172.0(CO).
MS (ES), *m*/*z*: 218.1 [M + H]⁺.

**General procedure for thioamide TA preparation.** In a solution of DCM, amine (1.0 eq), Boc-D-Trp (1.0 eq), NMM (2.2 eq) and BOP (1.0 eq) were successively added. After one hour stirring at room temperature, the mixture was concentrated in vacuo and dissolved in dichloromethane. The organic layer was successively washed with aqueous solutions of 1 M KHSO₄, saturated NaHCO₃ and brine. The organic layer was then dried over Na₂SO₄, filtered and concentrated in vacuo to yield amide A that was used without purification. To 1.0 eq. of amide A in DME or THF (10 mmol/mL) was added Lawesson's reagent (0.5 eq). The reaction was heated to 85 °C for 2 hours and then concentrated in vacuo. The residue was purified by chromatography on silica gel with a mixture of AcOEt/hexane 3/7 as eluent. The thioamide TA was obtained as a white powder (yields between 35 and 70% for the two steps).

### Tert-butyl (R)-1-(4-methoxybenzylthiocarbamoyl)-2-(1H-indol-3-yl)ethylcarbamate (TA1). Obtained from Boc-D-Trp and 4-methoxybenzylamine. 4.4 g (83%).

¹H NMR (300 MHz, DMSO d₆, 300 °K): δ 1.27 (s, 9H, CH₃ Boc), 2.94 (dd, 1H, J = 9 Hz and 14 Hz, CH₂ βTrp), 3.13 (dd, 1H, J = 5 Hz and 14 Hz, CH₂ βTrp), 3.69 (s, 3H, OCH₃), 4.62 (m, 3H, CH₂ *p*-methoxybenzyl and CH αTrp), 6.76 (s, 4H, CHar *p-*methoxybenzyl), 6.94 (m, 2H, H₄ and H₅ Trp), 7.03 (t, 1H, H₆ Trp), 7.16 (s, 1H, H₂ Trp), 7.30 (d, 1H, Jₒ= 8 Hz, H₇ Trp), 7.59 (d, 1H, J= 8 Hz, NH Boc), 10.21 (t, 1H, J= 5 Hz, NH thioamide), 10.78 (s, 1H, NH indole Trp).
¹³C NMR (75 MHz, DMSO d₆, 300 °K): δ 28.5 (CH₃ Boc), 31.3 (C βTrp), 48.2 (CH₂ *p*-methoxybenzyl), 55.3 (OCH₃), 62.0 (C αTrp), 78.6 (Cq Boc), 110.4 (C₃ Trp), 111.7 (C₇ Trp), 114.0 (C₃ and C₅ *p*-methoxybenzyl), 118.6 (C₄ Trp), 118.9 (C₅ Trp), 121.2 (C₆ Trp), 124.3 (C₂ Trp), 127.7 (C₉ Trp), 129.0 (C₁ *p*-methoxybenzyl), 129.2 (C₂ and C₆ *p-*methoxybenzyl), 136.5 (C₈ Trp), 155.2 (CO Boc), 158.8 (C₄ *p*-methoxybenzyl), 204.8 (CS thioamide).
MS (ES), *m*/*z*: 439.9 [M+H]⁺, 383.9 [M+H-*t*Bu]⁺, 339.9 [M+H-Boc]⁺.

### Tert-butyl (R)-1-(2,4-dimethoxybenzylthiocarbamoyl)-2-(1H-indol-3-yl)ethylcarbamate (TA2). Obtained from Boc-D-Trp and 2,4-dimethoxybenzylamine.7.42g, 96%.

¹H NMR (300 MHz, DMSO-d₆, 300 °K): δ(ppm) 1.27 (s, 9H, CH₃ Boc), 2.97 (dd, 1H, ³J = 8 Hz and 14 Hz, CH₂ βTrp), 3.30 (dd, 1H, ³J = 4 Hz and 14 Hz, CH₂ βTrp), 3.71 (s, 3H, CH₃O), 3.75 (s, 3H, CH₃O), 4.58 (m, 3H, CH₂-*o*,*p*-dimethoxybenzyl and CH αTrp), 6.37 (dd, 1H, Jₒ= 8 Hz and Jₘ= 2 Hz, H₅ *o*,*p*-dimethoxybenzyl), 6.53 (d, 1H, Jₘ= 2 Hz, H₃ *o*,*p*-dimethoxybenzyl), 6.86 (d, 1H, Jₒ= 8 Hz, H₄ Trp), 6,87 (d, 1H, Jₒ= 8 Hz, H₆ *o*,*p*-dimethoxybenzyl), 6.95 (t, 1H, Jₒ= 7 Hz, H₅ Trp), 7.03 (t, 1H, Jₒ= 7 Hz, H₆ Trp), 7.11 (s, 1H, H₂ Trp), 7.30 (d, 1H, Jₒ= 8 Hz, H₇ Trp), 7.60 (m, 1H, NH Trp), 9.97 (t, 1H, J = 6 Hz, NH-thioamide), 10.78 (s, 1H, NH indole Trp).
¹³C NMR (75 MHz, DMSO-d₆, 300 °K): δ(ppm) 28.5 (CH₃ Boc), 31.3 (CH₂ βTrp), 44.2 (CH₂-*o*,*p*-dimethoxybenzyl), 55.6 (OCH₃), 55.9 (OCH₃), 61.9 (CH αTrp), 78.6 (Cq Boc), 98.7 (C₃ *o*,*p*-dimethoxybenzyl), 104.8 (C₅ *o*,*p*-dimethoxybenzyl), 110.4 (C₃ Trp), 111.7 (C₇ Trp), 116.7 (C₁ *o*,*p*-dimethoxybenzyl), 118.6 (C₄ Trp), 118.9 (C₅ Trp), 121.2 (C₆ Trp), 124.4 (C₂ Trp), 127.8 (C₉ Trp), 130.1 (C₆ *o*,*p*-dimethoxybenzyl), 136.5 (C₈ Trp), 155.5 (CO Boc), 158.6 (C₂ *o*,*p*-dimethoxybenzyl), 161.1 (C₄ *o*,*p-*dimethoxybenzyl), 210.4 (CS thioamide).
MS (ES): m/z 470.0 [M+H]⁺.

### Tert-butyl (R)-1-((naphthalen-1-yl)methylthiocarbamoyl)-2-(1H-indol-3-yl)ethylcarbamate (TA3). Obtained from Boc-D-Trp and (naphthalen-1-yl)methanamine. 10.1 g, 73%.

¹H NMR (300 MHz, DMSO d⁶, 300 K): δ(ppm) 1.29 (9H, s, CH₃ Boc) ; 3.06 (1H, dd, ³J= 14 Hz and 9 Hz, CH₂ β Trp) ; 3.22 (1H, dd, ³J= 14 Hz and 5 Hz, CH₂ β Trp); 4.74 (1H, CH α Trp); 5.20 (2H, m, CH₂ naphtyle); 6.85 (1H, d, Jₒ= 7 Hz, H₄ Trp); 6.97 (1H, t, Jₒ= 7 Hz, H₅ Trp); 7.06 (2H, m, H₄ and H₆ Trp); 7.20 (1H, d, Jₘ= 2 Hz, H₂ Trp) ; 7.33 (1H, d, Jₒ= 6 Hz, H₇ Trp); 7.37 (2H, m, H₂ and H₃ naphtyle) ; 7.50-7.52 (2H, m, H₆ and H₇ naphtyle); 7.64 (1H, d, J= 7 Hz, NH amide) ; 7.84 (1H, d, Jₒ= 8 Hz, H₄ naphtyle); 7.91 (1H, d, Jₒ= 6 Hz, H₈ naphtyle); 7.93 (1H, d, Jₒ= 6 Hz, H₅ naphtyle); 10.40 (1H, s broad, NH-CH₂-naphtyle), 10.83 (1H, s, NH indole Trp).
¹³C NMR (75 MHz, DMSO d⁶, 300 K): δ(ppm) 28,5 (CH₃ Boc); 31.4 (CH₂ β Trp); 47.3 (CH₂-naphtyle); 62.0 (CH α Trp) ; 78.6 (Cquaternary Boc); 110.4 (C₃ Trp); 111.7 (C₇ Trp); 118.6 (C₄ Trp); 119.0 (C₅ Trp); 121.3 (C₆ Trp); 123.8 (C₂ naphtyle) ; 124.4 (C₂ Trp); 125.7 (C₈ naphtyle); 126.2 (C₃ naphtyle); 126.3 (C₆ naphtyle); 126.8 (C₇ naphtyle) ; 131.4 (C₉ Trp); 132.5 (C₄ naphtyle); 133.7 (C₅ naphtyle); 128.9 (C₉ naphtyle); 132.5 (C₁ naphtyle) ; 134.7 (C₁₀ naphtyle) ; 136.5 (C₈ Trp) ; 155.3 (CO Boc); 205.3 (CS thioamide).

**General procedure for preparation of triazole.** To a solution of 1.0 eq. of thioamide **TA** in 5 mL of dichloromethane (10 mmol/mL) were added 2.0 eq. of hydrazide and then 2 eq. of silver benzoate and 3 eq. of acetic acid at room temperature. After stirring overnight, the mixture was concentrated in vacuo to give a black oil which was diluted in a minimum of dichloromethane. The residue was purified by chromatography on silica gel with a mixture of AcOEt/MeOH (100/0 to 90/10) as eluent. The desired compounds were obtained as a white powder (yield ranging between 40-80%).

**(*R*)-*tert*-butyl 1-(4-(4-methoxybenzyl)-5-phenethyl-4*H*-1,2,4-triazol-3-yl)-2-(1*H-*indol-3-yl)ethylcarbamate (TP1).** 3,06 g of **TA1** (6,95 mmol) and 2,29 g of phenylacetic hydrazide (13,9 mmol) were diluted in the minimum of dichloromethane. 3,18 g of silver benzoate (13,9 mmol) were then added immediately followed by 1,19 ml of acetic acid (20,55 mmol). The mixture was stirred overnight at room temperature. Distillation of the solvent under reduced pressure gave a black oil, which was diluted in the minimum of dichloromethane. The residue was purified by flash chromatography on silica gel, eluting with Ethyl Acetate/MeOH (100/0 to 90/10) to afford 2,8 g of triazole as a white solid.
LC/MS = 552.1; Yield : 73%.

¹H NMR (300 MHz, DMSO d₆, 300 K): δ 1.21 (s, 9H, CH₃ Boc), 2.79 (m, 2H, CH₂-CH₂-phenyl), 2.88 (m, 2H, CH₂-CH₂-phenyl), 3.33 (m, 2H, CH₂ βTrp), 3.67 (s, 3H, OCH₃), 5.03 (m, 1H, CH αTrp), 5.17 (s, 2H, CH₂-*p*-methoxyphenyl), 6.72 (d, 2H, Jₒ= 8 Hz, H₃ and H₅ *p*-methoxyphenyl), 6.81 (d, 2H, Jₒ= 8 Hz, H₂ and H₆ *p*-methoxyphenyl), 6.88 (t, 1H, Jₒ= 8 Hz, H₅ Trp), 7.02 (t, 1H, Jₒ= 8 Hz, H₆ Trp), 7.04 (m, 2H, H₂ and H₆ phenyl), 7.06 (s, 1H, H₂ Trp), 7.16 (d, 1H, Jₒ= 7 Hz, H₄ Trp), 7.19-7.32 (m, 4H, H₇ Trp, H₃, H₄ and H₅ phenyl), 7.73 (d, 1H, J= 8 Hz, NH Boc), 10.84 (s, 1H, NH indole Trp).

¹³C NMR (75 MHz, DMSO d₆, 300 °K): δ 26.2 (CH₂-CH₂-phenyl), 28.4 (CH₃ Boc), 28.7 (C βTrp), 32.0 (CH₂-CH₂-phenyl), 46.3 (CH₂-*p*-methoxyphenyl), 46.8 (C αTrp), 55.5 (OCH₃), 79.0 (Cq Boc), 109.7 (C₃ Trp), 111.8 (C₇ Trp), 114.5 (C₃ and C₅ *p-*methoxyphenyl), 118.4 (C₄ Trp), 118.8 (C₅ Trp), 121.3 (C₆ Trp), 124.5 (C₂ Trp), 126.7 (C₄ phenyl, C₉ Trp), 127.4 (C₁ *p*-methoxyphenyl), 128.2 (C₂ and C₆ *p*-methaxyphenyl), 128.8 (C₂, C₃, C₅ and C₆ phenyl), 136.4 (C₈ Trp), 140.1 (C₁ phenyl), 154.9 (CO Boc), 155.6 (Cq triazole), 156.0 (Cq triazole), 159.3 (C₄ *p*-methoxyphenyl).
MS (ES), *m*/*z*: 552.1 [M+H]⁺.

### (R)-tert-butyl 1-(5-(2-(1H-indol-3-yl)ethyl)-4-(4-methoxybenzyl)-4H-1,2,4-triazol-3-yl)-2-(1H-indol-3-yl)ethylcarbamate (TI1). Obtained from TA1 and 3-(1H-indol-3-yl)propanehydrazide. 1.4 g (62%).

¹H NMR (300 MHz, DMSO d₆, 300 °K): δ 1.20 (s, 9H, CH₃ Boc), 2.93 (m, 4H, CH₂-CH₂-indole), 3.32 (m, 2H, CH₂ βTrp), 3.66 (s, 3H, OCH₃), 5.02 (m, 1H, CH αTrp), 5.18 (m, 2H, CH₂-*p*-methoxyphenyl), 6.71 (d, 2H, Jₒ= 8 Hz, H₃ and H₅ *p*-methoxyphenyl), 6.81 (d, 2H, Jₒ= 8 Hz, H₂ and H₆ *p*-methoxyphenyl), 6.90 (t, 3H, Jₒ= 7 Hz, H₅ and H₆ Trp, H₅ indole), 7.00-7.07 (m, 4H, H₂ and H₆ indole, H₂ and H₄ Trp), 7.26-7.32 (m, 3H, H₄ and H₇ indole, H₇ Trp), 7.72 (d, 1H, J= 8 Hz, NH Boc), 10.80 (s, 1H, NH indole), 10.83 (s, 1H, NH indole Trp).
¹³C NMR (75 MHz, DMSO d₆, 300 °K): δ 22.1 (CH₂-CH₂-indole), 25.7 (CH₂-CH₂-indole), 27.5 (CH₃ Boc), 27.7 (C βTrp), 46.5 (CH₂-*p*-methoxyphenyl), 46.9 (C αTrp), 55.5 (OCH₃), 79.0 (Cq Boc), 109.7 (C₃ Trp), 111.8 (C₇ Trp and C₇ indole), 112.6 (C₃ indole), 114.3 (C₃ and C₅ *p*-methoxyphenyl), 118.1 (C₄ Trp and C₄ indole), 119.1 (C₅ Trp and C₅ indole), 121.5 (C₆ Trp and C₆ indole), 123.1 (C₂ indole), 124.5 (C₂ Trp), 126.6 (C₉ indole), 127.0 (C₉ Trp), 127.4 (C₁ *p*-methoxyphenyl), 128.1 (C₂ and C₆ *p-*methoxyphenyl), 136.4 (C₈ Trp), 136.6 (C₈ indole), 155.4 (CO Boc), 155.5 (Cq triazole), 156.0 (Cq triazole), 162.2 (C₄ *p*-methoxyphenyl).
MS (ES), m/z: 561.1 [M+H]⁺.

### (R)-tert-butyl 1-(4-(2,4-dimethoxybenzyl)-5-phenethyl-4H-1,2,4-triazol-3-yl)-2-(1H-indol-3-yl)ethylcarbamate (TP2). Obtained from TA2 and phenylacetic hydrazide. 2.2 g (65%).

¹H NMR (300 MHz, DMSO d₆, 300 °K): δ 1.23 (s, 9H, CH₃ Boc), 2.81 (m, 2H, CH₂-CH₂-phenyl), 2.94 (m, 2H, CH₂-CH₂-phenyl), 3.28 (m, 2H, CH₂ βTrp), 3.58 (s, 3H, OCH₃), 3.68 (s, 3H, OCH₃), 5.05 (m, 3H, CH₂-*o*,*p*-dimethoxyphenyl and CH αTrp), 6.30 (d, 1H, Jₒ= 8 Hz, H₅ *o*,*p*-dimethoxyphenyl), 6.51 (s, 1H, H₃ *o*,*p*-dimethoxyphenyl), 6.62 (d, 1H, Jₒ= 8 Hz, H₆ *o*,*p*-dimethoxyphenyl), 6.89 (t, 1H, Jₒ= 7 Hz, H₅ Trp), 7.02 (t, 1H, Jₒ= 8 Hz, H₆ Trp), 7.06 (d, 2H, Jₒ= 8 Hz, H₂ and H₆ phenyl), 7.08 (s, 1H, H₂ Trp), 7.17 (d, 1H, Jₒ= 7 Hz, H₄ Trp), 7.21-7.31 (m, 4H, H₇ Trp, H₃, H₄ and H₅ phenyl), 7.65 (d, 1H, J= 8 Hz, NH Boc), 10.85 (s, 1H, NH indole Trp).
¹³C NMR (75 MHz, DMSO d₆, 300 °K): δ 26.1 (CH₂-CH₂-phenyl), 28.4 (CH₃ Boc), 28.7 (C βTrp), 32.0 (CH₂-CH₂-phenyl), 43.1 (CH₂-*o*,*p*-dimethoxyphenyl), 46.8 (C αTrp), 55.7 (OCH₃), 55.8 (OCH₃), 79.0 (Cq Boc), 99.0 (C₃ *o*,*p*-dimethoxyphenyl), 105.2 (C₅ *o*,*p-*dimethoxyphenyl), 109.7 (C₃ Trp), 111.8 (C₇ Trp), 114.2 (C₁ *o*,*p*-dimethoxyphenyl), 118.4 (C₄ Trp), 118.8 (C₅ Trp), 121.3 (C₆ Trp), 124.4 (C₂ Trp), 126.8 (C₆ *o*,*p-*dimethoxyphenyl), 127.4 (Cg Trp), 128.6-129.1 (C₂, C₃, C₄, C₅ and C₆ phenyl), 136.4 (C₈ Trp), 140.0 (C₁ phenyl), 155.1 (Cq triazole), 155.5 (Cq triazole), 156.2 (CO Boc), 158,0 (C₂ *o*,*p*-dimethoxyphenyl), 161.2 (C₄ *o*,*p*-dimethoxyphenyl).
MS (ES), *m*/*z*: 583.0 [M+H]⁺.

### (R)-tert-butyl 1-(5-(2-(1H-indol-3-yl)ethyl)-4-(2,4-dimethoxybenzyl)-4H-1,2,4-triazol-3-yl)-2-(1H-indol-3-yl)ethylcarbamate (TI2). Obtained from TA2 and 3-(1H-indol-3-yl)propanehydrazide. 1.2 g (60%).

¹H NMR (300 MHz, DMSO d₆, 300 °K): δ 1.21 (s, 9H, CH₃ Boc), 2.90 (m, 4H, CH₂-CH₂-indole), 3.28 (m, 2H, CH₂ βTrp), 3.59 (s, 3H, OCH₃), 3.66 (s, 3H, OCH₃), 5.01 (m, 3H, CH₂-*o*,*p*-dimethoxyphenyl and CH αTrp), 6.26 (d, 1H, Jₒ= 8 Hz, H₅ *o*,*p-*dimethoxyphenyl), 6.49 (d, 1H, Jₒ= 8 Hz, H₆ *o*,*p*-dimethoxyphenyl), 6.51 (s, 1H, H₃ *o*,*p-*dimethoxyphenyl), 6.89 (m, 2H, H₅ Trp and H₅ indole), 7.02 (m, 2H, H₆ indole and H₆ Trp), 7.03 (s, 1H, H₂ indole), 7.05 (s, 1H, H₂ Trp), 7.26 (d, 1H, Jₒ= 8 Hz, H₄ Trp), 7.29 (m, 3H, H₄ and H₇ indole, H₇ Trp), 7.57 (d, 1H, J= 9 Hz, NH Boc), 10.79 (s, 1H, NH indole), 10.81 (1H, s, NH indole Trp).
¹³C NMR (75 MHz, DMSO d₆, 300K): δ 22.4 (CH₂-CH₂-indole), 25.7 (CH₂-CH₂-indole), 28.4 (CH₃ Boc), 28,9 (C βTrp), 42.6 (CH₂-*o*,*p*-dimethoxyphenyl), 46.8 (C αTrp), 55.6 (OCH₃), 55.8 (OCH₃), 78.8 (Cq Boc), 98.9 (C₃ *o*,*p*-dimethoxyphenyl), 105.1 (C₅ *o*,*p*-dimethoxyphenyl), 110.0 (C₃ Trp), 111.7 (C₇ Trp), 111.8 (C₇ indole), 112.9 (C₃ indole), 114.8 (C₁ *o*,*p*-dimethoxyphenyl), 118.4 (C₄ indole and C₄ Trp), 118.7 (C₅ indole and C₅ Trp), 121.3 (C₆ Trp), 121.4 (C₆ indole), 123.0 (C₂ indole), 125.1 (C₂ Trp), 127.1 (C₉ indole), 127.5 (C₉ Trp), 128.5 (C₆ *o,p*-dimethoxyphenyl), 136.4 (C₈ Trp), 136.6 (C₈ indole), 155.5 (Cq triazole and CO Boc), 156.1 (Cq triazole), 157.8 (C₂ *o,p*-dimethoxyphenyl), 161.0 (C₄ *o,p*-dimethoxyphenyl).
MS (ES), *m*/*z*: 621.0 [M+H]⁺.

**tert-butyl (R)-2-(1H-indol-3-yl)-1-(4-((naphthalen-1-yl)methyl)-5-phenethyl-4H-1,2,4-triazol-3-yl)ethylcarbamate (TP3).** 2,5 g of **TA3** (5,45 mmol) and 1,79 g of phenylacetic hydrazide (10,9 mmol) were diluted in the minimum of dichloromethane. 2,49 g of silver benzoate (10,9 mmol) were then added immediately followed by 0,93 ml of acetic acid (16,35 mmol). The mixture was stirred overnight at room temperature. Distillation of the solvent under reduced pressure gave a black oil, which was diluted in the minimum of dichloromethane. The residue was purified by flash chromatography on silica gel, eluting with Hexane/Ethyl Acetate/MeOH (8/2/0 to 0/95/5) to afford 2,70 g of triazole as a white solid.
MS (ES), *m*/*z*: 572.2 [M+H]⁺

### tert-butyl (R)-1-(5-(2-(1H-indol-3-yl)ethyl)-4-((naphthalen-1-yl)methyl)-4H-1,2,4-triazol-3-yl)-2-(1H-indol-3-yl)ethylcarbamate (TI3). Obtained from TA3 and 3-(1 H-indol-3-yl)propanehydrazide. Yield 40%.

¹H NMR (300 MHz, DMSO d⁶, 300 K): δ(ppm) 0.98 (9H, s, CH₃ Boc) ; 2.77 (2H, m, CH₂-CH₂-indole); 2.94 (2H, m, CH₂-CH₂-indole) ; 3.29 (2H, m, CH₂ β Trp); 4.79 (1H, m, CH α Trp); 5.55 (2H, s, CH₂-naphtyle) ; 6.24 (1H, d, Jₒ= 7 Hz, H₂ naphtyle) ; 6.69 (2H, m, H₅ indole and H₅ Trp); 6.92-7.17 (6H, m, H₂, H₄ and H₆ Trp, H₂, H₄ and H₆ indole) ; 7.24 (3H, m, H₇ Trp, H₇ indole, H₃ naphtyle); 7.39 (1H, d, J= 8 Hz, NH Boc); 7.53 (2H, m, H₆ and H₇ naphtyle) ; 7.77 (1H, d, Jₒ= 8 Hz, H₄ naphtyle); 7.93 (2H, m, H₅ and H₈ naphtyle); 10.67 (1H, s, NH indole); 10.71 (1H, s, NH indole Trp).
¹³C NMR (75 MHz, DMSO d⁶, 300 K): δ(ppm) 23.2 (CH₂-CH₂-indole) ; 25.9 (CH₂-CH₂-indole); 28.2 (CH₃ Boc) ; 29.2 (CH₂ β Trp) ; 43.7 (CH₂-naphtyle) ; 46.8 (CH α Trp) ; 78.2 (Cquaternary Boc) ; 110.7 (C₃ Trp) ; 111.6 (C₇ indole) ; 111.7 (C₇ Trp) ; 113.5 (C₃ indole); 118.3 (C₄ indole, C₄ Trp) ; 118.5 (C₅ indole, C₅ Trp) ; 121.1 (C₆ Trp); 121.2 (C₆ indole, C₂ naphtyle); 123.0 (C₂ indole, C₈ naphtyle); 124.4 (C₂ Trp) ; 125.7 (C₃ naphtyle) ; 126.5 (C₆ naphtyle); 126.8 (C₇ naphtyle); 127.0 (C₉ Trp) ; 127.6 (C₉ indole) ; 127.9 (C₄ naphtyle); 128.9 (C₅ naphtyle) ; 130.0 (C₉ naphtyle) ; 132.1 (C₁ naphtyle) ; 133.5 (C₁₀ naphtyle) ; 136.4 (Cg Trp); 136.5 (C₈ indole) ; 155.2 (CO Boc); 155.4 (Cquaternary triazole) ; 155.9 (Cquaternary triazole).
(M+H)⁺: 611.2

**General procedure for preparation of final compound.** The Boc protecting group of previous compound was removed at room temperature for one hour with a solution of AcOEt/HCl 4M. The mixture was then concentrated in vacuo, diluted with MeOH and concentrated several times in vacuo. The residue was then coupled with the corresponding acid (1.1 eq.), in the presence of BOP (1.1 eq.) and NMM (2.2 eq.) for two hours, in DCM. The mixture was then concentrated in vacuo and the residue dissolved in AcOEt. The organic layer was successively washed with aqueous solutions of 1 M KHSO₄, saturated NaHCO₃ and brine. The organic layer was then dried over Na₂SO₄, filtered and concentrated in vacuo to yield the desired compound which was then treated with 4M AcOEt/HCl as already described if necessary. The final compound purified by preparative HPLC on a C18 column using a water/acetonitrile/TFA 0.1 % gradient (yield around 50% for the three steps).

The following compounds have been synthesized according to above syntheses schemes and have been physico-chemically characterized:

### Compound 2:

¹H NMR (300 MHz, DMSO d^{6.} 300°K): δ(ppm) 2.79 (4H, m, CH₂-CH₂-phenyle); 3.02 (1H, m, H₃ *o*-piperazinyle); 3.15 and 3.23 (2H, m, H₅ *o*-piperazinyle); 3.32 (1H, m, CH₂ β Trp); 3.44 (1H, m, CH₂ β Trp); 3.46 (2H, m, H₆ *o*-piperazinyle); 3.68 (OCH₃); 4.22 (H₂ *o*-piperazinyle); 4.87 (2H, s, CH₂-*p*-methoxyphenyle); 5.23 (1H, m, CH α Trp); 6.68 (4H, s, CHaromatics *p-*methoxyphenyle); 6.91 (1H, t, Jₒ= 7 Hz, H₅ Trp); 7.04 (1H, s, H₂ Trp); 7.09 (1H, t, Jₒ= 7 Hz, H₆ Trp); 7.10 (2H, m, H₂ and H₆ phenyle); 7.17-7.24 (3H, m, H₃, H₄ and H₅ phenyle); 7.32 (1H, d, Jₒ= 8 Hz, H₄ Trp); 7.36 (1H, d, Jₒ= 8 Hz, H₇ Trp); 9.64 (1H, d, J= 7 Hz, NH amide); 10.90 (1H, s, NH indole Trp)
¹³C NMR (75 MHz, DMSO d^{6.} 300°K): δ(ppm) 26.0 (CH₂-CH₂-phenyle); 29.2 (CH₂ β Trp); 32.2 (CH₂-CH₂-phenyle); 39.4 (C₅ and C₆ *o*-piperazinyle); 41.9 (C₃ *o*-piperazinyle); 44.9 (CH₂-*p*-methoxyphenyle); 45.7 (CH α Trp); 53.0 (C₂ *o*-piperazinyle); 55.0 (OCH₃); 109.0 (C₃ Trp); 111.4 (C₇ Trp); 114.0 (C₃ and C₅ *p*-methoxyphenyle); 117.8 (C₄ Trp); 118.5 (C₅ Trp); 121.0 (C₆ Trp); 124.2 (C₂ Trp); 126.1 (C₄ phenyle); 126.9 (C₉ Trp and C₁ *p*-methoxyphenyle); 127.4 (C₂ and C₆ *p*-methoxyphenyle); 128.3 (C_{2.} C_{3.} C₅ and C₆ phenyle); 136.0 (C₈ Trp); 140.3 (C₁ phenyle); 153.9 (Cquaternary triazole); 154.0 (Cquaternary triazole); 158.7 (C₄ *p-*methoxyphenyle); 164.3 (CO amide).

### Compound 3:

¹H NMR (300 MHz, DMSO d^{6.} 300°K): δ(ppm) 1.80 (2H, m, H₃ and H₅ piperidine); 2.35 (2H, m, H₃ and H₅ piperidine); 2.91 (6H, m, CH₂-CH₂-indole, H₂ and H₆ piperidine); 3.09 (2H, m, H₂ and H₆ piperidine); 3.35 (2H, m, CH₂ β Trp); 3.66 (3H, s, OCH₃); 4.92 (2H, s, CH₂-*p-*methoxyphenyle); 5.23 (1H, m, CH α Trp); 6.68 (4H, s, CHaromatics *p*-methoxyphenyle); 6.86 (2H, m, H₅ indole and H₅ Trp); 7.03 (5H, m, H₂ and H₆ indole, H_{2.} H₄ and H₆ Trp); 7.20 (1H, d, Jₒ= 8 Hz, H₄ indole); 7.30 (2H, m, H₇ indole and H₇ Trp); 8.50 (1H, m, NH piperidine TFA salt); 9.02 (3H, m, NH amide and NH piperidine TFA salt); 10.75 (1H, s, NH indole); 10.80 (1H, s, NH indole Trp)
¹³C NMR (75 MHz, DMSO d^{6.} 300°K): δ(ppm) 20.7 (C₃ and C₅ piperidine); 22.8 (CH₂-CH₂-indole); 25.9 (CH₂-CH₂-indole); 28.4 (CH₂ β Trp); 40.8 (C₂ and C₆ piperidine); 45.5 (CH₂-*p-*methoxyphenyle); 46.3 (CH α Trp); 55.5 (OCH₃); 56.0 (C₄ piperidine); 109.6 (C₃ Trp); 111.8 (C₇ indole and C₇ Trp); 113.4 (C₃ indole); 114.5 (C₃ and C₅ *p*-methoxyphenyle); 118.3 (C₄ indole); 118.5 (C₄ Trp); 118.6 (C₅ indole and C₅ Trp); 121.4 (C₆ indole and C₆ Trp); 123.1 (C₂ indole); 124.5 (C₂ Trp); 127.2 (C₉ indole); 127.3 (C₉ Trp); 127.6 (C₁ *p*-methoxyphenyle); 127.8 (C₂ and C₆ *p*-methoxyphenyle); 136.5 (C₈ Trp); 136.6 (C₈ indole); 154.5 (Cquaternary triazole); 154.9 (Cquaternary triazole); 159.1 (C₄ *p*-methoxyphenyle); 168.9 (CO amide).

### Compound 4:

¹H NMR (300 MHz, DMSO d^{6.} 300°K): δ(ppm) 1.82 (2H, m, H₃ and H₅ piperidine); 2.37 (2H, m, H₃ and H₅ piperidine); 2.78 (4H, m, CH₂-CH₂-phenyle); 2.96-3.16 (4H, m, H₂ and H₆ piperidine); 3.36 (2H, m, CH₂ β Trp); 3.66 (3H, s, OCH₃); 4.94 (2H, s, CH₂-*p*-methoxyphenyle); 5.23 (1H, m, CH α Trp); 6.69 (4H, s, CHaromatics *p*-methoxyphenyle); 6.85 (1H, t, Jₒ= 7 Hz, H₅ Trp); 7.02 (1H, t, Jₒ= 8 Hz, H₆ Trp); 7.03 (1H, s, H₂ Trp); 7.09 (2H, d, Jₒ= 8 Hz, H₂ and H₆ phenyle); 7.16 (1H, d, Jₒ= 7 Hz, H₄ Trp); 7.22 (3H, m, H_{3.} H₄ and H₅ phenyle); 7.31 (1H, d, Jₒ= 8 Hz, H₇ Trp); 8.52 (1H, m, NH piperidine TFA salt); 9.05 (2H, m, NH amide and NH piperidine TFA salt); 10.80 (1H, s, NH indole Trp)
¹³C NMR (75 MHz, DMSO d^{6.} 300°K): δ(ppm) 26.5 (CH₂-CH₂-phenyle); 28.4 (CH₂ β Trp); 29.5 (C₃ and C₅ piperidine); 32.7 (CH₂-CH₂-phenyle); 40.8 (C₂ and C₆ piperidine); 45.4 (CH₂-*p-*methoxyphenyle); 46.3 (CH α Trp); 55.5 (OCH₃); 56.0 (C₄ piperidine); 109.6 (C₃ Trp); 111.9 (C₇ Trp); 114.5 (C₃ and C₅ *p*-methoxyphenyle); 118.3 (C₄ Trp); 118.9 (C₅ Trp); 121.4 (C₆ Trp); 122.9 (C₂ Trp); 126.6 (C₄ phenyle); 127.3 (C₉ Trp); 127.6 (C₁ *p*-methoxyphenyle); 127.9 (C₂ and C₆ *p*-methoxyphenyle); 128.7 (C_{2.} C_{3.} C₅ and C₆ phenyle); 136.5 (C₈ Trp); 140.8 (C₁ phenyle); 154.4 (Cquaternary triazole); 154.5 (Cquaternary triazole); 159.1 (C₄ *p-*methoxyphenyle); 168.9 (CO amide).

### Compound 6:

1H NMR (300 MHz, DMSO d^{6.} 300°K): δ(ppm) 2.82 (4H, m, CH₂-CH₂-phenyle); 3.44 (2H, d, J= 7 Hz, CH₂ β Trp); 3.60 (3H, s, OCH₃); 3.64 (3H, s, OCH₃); 4.01 (2H, m, CH₂-NH₂); 4.91 (1H, d, J= 17 Hz, CH₂-*o,p*-dimethoxybenzylamine); 5.13 (1H, d, J= 17 Hz, CH₂-*o,p-*dimethoxybenzylamine); 5.49 (1H, m, CH α Trp); 6.19 (1H, dd, Jₒ= 8 Hz and Jₘ= 2 Hz, H₅ *o,p-*dimethoxybenzylamine); 6.41 (1H, d, Jₒ= 8 Hz, H₆ *o,p*-dimethoxybenzylamine); 6.47 (1H, d, Jₘ= 2 Hz, H₃ *o,p*-dimethoxybenzylamine); 6.87 (1H, t, Jₒ= 7 Hz, H₅ Trp); 7.00 (1H, t, Jₒ= 8 Hz, H₆ Trp); 7.07 (1H, s, H₂ Trp); 7.08 (2H, d, Jₒ= 8 Hz, H₂ and H₆ phenyle); 7.14 (1H, d, Jₒ= 7 Hz, H₄ Trp); 7.19 (1H, d, Jₒ= 7 Hz, H₇ Trp); 7.29 (3H, t, J= 8 Hz, H₃. H₄ and H₅ phenyle); 7.43 (1H, t, Jₒ= 8 Hz, H₅ *m*-aminophenyle); 7.55 (1H, d, Jₒ= 8 Hz, H₄ *m*-aminophenyle); 7.77 (1H, d, Jₒ= 8 Hz, H₆ *m*-aminophenyle); 7.85 (1H, s, H₂ *m*-aminophenyle); 8.17 (3H, s large, NH₂ TFA salt); 9.08 (1H, d, J= 8 Hz, NH amide); 10.76 (1H, s, NH indole Trp).
¹³C NMR (75 MHz, DMSO d^{6.} 300°K): δ(ppm) 26.4 (CH₂CH₂-phenyle); 29.0 (CH₂ β Trp); 32.5 (CH₂-CH₂-phenyle); 42.1 (CH₂-NH₂); 42.5 (CH₂-*o,p*-dimethoxybenzylamine); 45.6 (CH α Trp); 55.6 (OCH₃); 55.9 (OCH₃); 98.9 (C₃ *o,p*-dimethoxybenzylamine); 105.1 (C₅ *o,p-*dimethoxybenzylamine); 110.3 (C₃ Trp); 111.7 (C₇ Trp); 115.4 (C₁ *o,p*-dimethoxybenzylamine); 118.4 (C₄ Trp); 118.8 (C₅ Trp); 121.3 (C₆ Trp); 124.4 (C₂ Trp); 126.8 (C₆ *o,p-*dimethoxybenzylamine); 127.5 (C₉ Trp); 127.6 (C₄ phenyle); 128.2-128.8 (C_{2.} C₅ and C₄ *m-*aminophenyle, C_{2.} C_{3.} C₅ and C₆ phenyle); 132.2 (C₄ *m*-aminophenyle); 134.1 (C₁ *m-*aminophenyle); 136.4 (C₈ Trp); 140.7 (C₃ *m*-aminophenyle and C₁ phenyle); 154.7 (Cquaternary triazole); 155.7 (Cquaternary triazole); 157.8 (C₂ *o,p*-dimethoxybenzylamine); 160.8 (C₄ *o,p*-dimethoxybenzylamine); 166.0 (CO amide).

### Compound 7:

1H NMR (300 MHz, DMSO d^{6.} 300°K): δ(ppm) 2.89 (4H, m, CH₂-CH₂-phenyle); 3.49 (2H, m, CH₂ β Trp); 3.56 (3H, s, OCH₃); 3.60 (3H, s, OCH₃); 4.95 (1H, d, J= 17 Hz, CH₂-*o,p-*dimethoxyphenyle); 5.07 (1H, d, J= 17 Hz, CH₂-*o,p*-dimethoxyphenyle); 5.53 (1H, m, CH α Trp); 6.13 (1H, dd, Jₒ= 8 Hz and Jₘ= 2 Hz, H₅ *o,p*-dimethoxyphenyle); 5.70 (1H, d, Jₒ= 8 Hz, H₆ *o,p*-dimethoxyphenyle); 6.36 (1H, d, Jₘ= 2 Hz, H₃ *o,p*-dimethoxyphenyle); 6.88 (1H, t, Jₒ= 8 Hz, H₅ Trp); 7.00 (1H, t, Jₒ= 8 Hz, H₆ Trp); 7.08 (1H, s, H₂ Trp); 7.10 (2H, d, Jₒ= 8 Hz, H₂ and H₆ phenyle); 7.14 (1H, d, Jₒ= 8 Hz, H₄ Trp); 7.16-7.28 (3H, m, H_{3.} H₄ and H₅ phenyle); 7.41 (1H, d, Jₒ= 7 Hz, H₇ Trp); 7.71 (1H, d, J= 6 Hz, NH amide); 9.24 (1H, d, Jₘ= 2 Hz, H₂ pyridazine); 9.29 (1H, d, Jₒ= 5 Hz, H₆ pyridazine); 9.49 (1H, d, Jₒ= 8 Hz, H₅ pyridazine); 10.77 (1H, s, NH indole Trp).
¹³C NMR (75 MHz, DMSO d^{6.} 300°K): δ(ppm) 26.1 (CH₂-CH₂-phenyle); 28.3 (CH₂ β Trp); 32.2 (CH₂-CH₂-phenyle); 42.7 (CH₂-*o,p*-dimethoxyphenyle); 45.7 (CH α Trp); 55.5 (OCH₃); 55.8 (OCH₃); 98.8 (C₃ *o,p*-dimethoxyphenyle); 105.0 (C₅ *o,p*-dimethoxyphenyle); 109.9 (C₃ Trp); 111.8 (C₇ Trp); 114.7 (C₁ *o,p*-dimethoxyphenyle); 118.4 (C₄ Trp); 118.8 (C₅ Trp); 121.4 (C₆ Trp); 124.3 (C₆ pyridazine); 124.4 (C₂ Trp); 126.7 (C₆ *o,p*-dimethoxyphenyle); 127.5 (C₄ phenyle); 127.8 (C₉ Trp); 128.7 (C_{2.} C_{3.} C₅ and C₆ phenyle); 130.5 (C₁ pyridazine); 136.4 (C₈ Trp); 140.3 (C₁ phenyle); 150.2 (C₂ and C₅ puridazine); 155.1 (quaternary triazole); 155.3 (Cquaternary triazole); 157.6 (C₂ *o,p*-dimethoxyphenyle); 160.7 (C₄ *o,p*-dimethoxyphenyle); 163.1 (CO amide).

### Compound 12:

1H NMR (300 MHz, DMSO d^{6.} 300°K): δ(ppm) 1.30 (4H, m, H₃ and H₅ tetrahydropyrane); 2.15 (1H, m, H₄ tetrahydropyrane); 2.85 (4H, m, CH₂-CH₂-phenyle); 2.99 (2H, m, H₂ and H₆ tetrahydropyrane); 3.29 (2H, m, CH₂ β Trp); 3.59 (3H, s, OCH₃); 3.68 (3H, s, OCH₃); 3.73 (2H, m, H₂ and H₆ tetrahydropyrane); 4.95 (1H, d, J= 17 Hz, CH₂-*o,p*-dimethoxyphenyle); 5.02 (1H, d, J= 17 Hz, CH₂-*o,p*-dimethoxyphenyle); 5.29 (1H, m, CH α Trp); 6.27 (1H, dd, Jₒ= 8 Hz and Jₘ= 2 Hz, H₅ *o,p*-dimethoxyphenyle); 6.49 (1H, s, H₃ *o,p*-dimethoxyphenyle); 6.51 (1H, d, Jₒ= 8 Hz, H₆ *o,p*-dimethoxyphenyle); 6.88 (1H, t, Jₒ= 8 Hz, H₅ Trp); 7.01 (1H, s, H₂ Trp); 7.02 (1H, t, Jₒ= 8 Hz, H₆ Trp); 7.07 (2H, d, Jₒ= 8 Hz, H₂ and H₆ phenyle); 7.15-7.31 (5H, m, H₄ and H₇ Trp, H_{3.} H₄ and H₅ phenyle); 8.47 (1H, d, J= 8 Hz, NH amide); 10.77 (1H, s, NH indole Trp).
¹³C NMR (75 MHz, DMSO d^{6.} 300°K): δ(ppm) 26.2 (CH₂-CH₂-phenyle); 28.8 (C₃ and C₅ tetrahydropyrane); 29.1 (CH₂ β Trp); 32.2 (CH₂-CH₂-phenyle); 40.7 (C₄ tetrahydropyrane); 42.7 (CH₂-*o,p*-dimethoxyphenyle); 44.8 (CH α Trp); 55.7 (OCH₃); 55.9 (OCH₃); 66.6 and 66.7 (C₂ and C₆ tetrahydropyrane); 99.0 (C₃ *o,p*-dimethoxyphenyle); 105.2 (C₅ *o,p*-dimethoxyphenyle); 109.8 (C₃ Trp); 111.7 (C₇ Trp); 114.7 (C₁ *o,p*-dimethoxyphenyle); 118.4 (C₄ Trp); 118.7 (C₅ Trp); 121.3 (C₆ Trp); 124.4 (C₂ Trp); 126.7 (C₄ phenyle and C₆ *o,p*-dimethoxyphenyle); 127.5 (C₉ Trp); 128.7 (C_{2.} C_{3.} C₅ and C₆ phenyle); 136.4 (C₈ Trp); 140.2 (C₁ phenyle); 154.9 (Cquaternary triazole); 156.0 (Cquaternary triazole); 158.8 (C₂ *o,p*-dimethoxyphenyle); 161.1 (C₄ *o,p*-dimethoxyphenyle); 174.2 (CO amide).

### Compound 13:

1H NMR (300 MHz, DMSO d^{6.} 300°K): δ(ppm) 2.88 (4H, m, CH₂-CH₂-indole); 3.47 (2H, m, CH₂ β Trp); 3.62 (3H, s, OCH₃); 4.00 (2H, m, CH₂-NH₂); 5.05 (1H, d, J= 16 Hz, CH₂-*p-*methoxyphenyle); 5.15 (1H, d, J= 16 Hz, CH₂-*p*-methoxyphenyle); 5.48 (1H, m, CH α Trp); 6.63 (2H, d, Jₒ= 9 Hz, H₃ and H₅ *p*-methoxyphenyle); 6.72 (2H, d, Jₒ= 9 Hz, H₂ and H₆ *p-*methoxyphenyle); 6.87 (2H, t, Jₒ= 7 Hz, H₅ Trp and H₅ indole); 6.97-7.07 (4H, m, H₂ and H₆ Trp, H₂ and H₆ indole); 7.27 (3H, m, H₄ Trp, H₄ and H₇ indole); 7.31 (1H, d, Jₒ= 8 Hz, H₇ Trp); 7.43 (1H, t, Jₒ= 8 Hz, H₅ *m*-aminomethylphenyle); 7.53 (1H, m, H₄ *m*-aminomethylphenyle); 7.77 (1H, d, Jₒ= 8 Hz, H₆ *m*-aminomethylphenyle); 7.84 (1H, s, H₂ *m*-aminomethylphenyle); 8.13 (3H, s large, NH₂ TFA salt); 9.13 (1H, d, J= 8 Hz, NH amide); 10.75 (2H, s, NH indole and NH indole Trp).
¹³C NMR (75 MHz, DMSO d^{6.} 300°K): δ(ppm) 22.7 (CH₂)-CH₂-indole); 25.9 (CH₂-CH₂-indole); 29.1 (CH₂ β Trp); 42.5 (CH₂-*p*-methoxyphenyle and CH₂-NH₂); 45.7 (CH α Trp); 55.4 (OCH₃); 110.3 (C₃ Trp); 111.7 (C₇ indole and C₇ Trp); 113.3 (C₃ indole); 114.4 (C₃ and C₅ *p-*methoxyphenyle); 118.4 (C₄ indole and C₄ Trp); 118.6 (C₅ indole); 118.8 (C₅ Trp); 121.3 (C₆ indole and C₆ Trp); 122.9 (C₂ indole); 124.5 (C₂ Trp); 127.2 (C₉ indole); 127.5 (C₉ Trp); 127.6 (C₂ and C₆ m-aminomethylphenyle); 127.7 (C₂ and C₆ *p*-methoxyphenyle); 128.8 (C₅ *m-*aminomethylphenyle); 132.2 (C₄ *m*-aminomethylphenyle); 134.1 (C₁ *m*-aminomethylphenyle); 136.4 (C₈ indole); 136.6 (C₈ Trp); 155.0 (quaternary triazole); 155.4 (Cquaternary triazole); 159.0 (C₄ *p*-methoxyphenyle); 166.0 (CO amide).

### Compound 15:

1H NMR (300 MHz, DMSO d^{6.} 300°K): δ(ppm) 1.00 (2H, m, H₃ and H₅ piperidine); 1.21 (1H, m, H₅ piperidine); 1.38 (1H, m, H₃ piperidine); 1.93 (1H, m, H₄ piperidine); 2.57 (2H, m, H₂ and H₆ piperidine); 2.79 (2H, m, CH₂-CH₂-phenyle); 2.90 (4H, m, CH₂-CH₂-phenyle, H₂ and H₆ piperidine); 3.28 (1H, dd, J= 14 Hz and 7 Hz, CH₂ β Trp); 3.36 (1H, dd, J= 14 Hz and 6 Hz, CH, β Trp); 5.12 (1H, m, CH α Trp); 5.51 (1H, d, J= 18 Hz, CH₂-naphtyle); 5.64 (1H, d, J= 18 Hz, CH₂-naphtyle); 6.15 (1H, d, Jₒ= 7 Hz, H₂ naphtyle); 6.68 (1H, t, Jₒ= 8 Hz, H₅ Trp); 6.94 (1H, t, Jₒ= 8 Hz, H₆ Trp); 7.00-7.20 (8H, m, H₂ and H₄ Trp, H₃ naphtyle and CHaromatics phenyle); 7.24 (1H, d, Jₒ= 8 Hz, H₇ Trp); 7.55 (2H, m, H₆ and H₇ naphtyle); 7.78 (1H, d, Jₒ= 8 Hz, H₄ naphtyle); 8.05 and 8.35 (2H, 2 m, NH piperidine TFA salt); 7.90 (2H, m, H₅ and H₈ naphtyle); 8.54 (1H, d, J= 8 Hz, NH amide); 10.72 (1H, s, NH indole Trp).
¹³C NMR (75 MHz, DMSO d^{6.} 300°K): δ(ppm) 24.4 (C₃ piperidine); 25.3 (C₅ piperidine); 26.3 (CH₂-CH₂-phenyle); 29.1 (CH₂ β Trp); 32.6 (CH₂-CH₂-phenyle); 38.4 (C₄ piperidine); 42.4 and 42.5 (C₂ and C₆ piperidine); 43.8 (CH₂-naphtyle); 44.6 (CH α Trp); 110.3 (C₃ Trp); 111.6 (C₇ Trp); 118.4 (C₄ Trp); 118.5 (C₅ Trp); 121.2 (C₆ Trp); 121.8 (C₂ naphtyle); 123.0 (C₈ naphtyle); 124.9 (C₂ Trp); 125.7 (C₃ naphtyle); 126.5 (C₆ naphtyle and C₄ phenyle); 126.7 (C₄ naphtyle); 127.0 (C₇ naphtyle); 127.5 (C₉ Trp); 128.1 (C₅ naphtyle); 128.7 (C_{2.} C_{3.} C₅ and C₆ phenyle); 129.9 (C₉ naphtyle); 131.8 (C₁ naphtyle); 133.5 (C₁₀ naphtyle); 136.4 (C₈ Trp); 140.8 (C₁ phenyle); 154.8 (Cquaternary triazole); 155.8 (Cquaternary triazole); 172.8 (CO amide).

### Compound 16:

1H NMR (300 MHz, DMSO d^{6.} 300°K): δ(ppm) 2.91 (4H, s, CH₂-CH₂-phenyle); 3.51 (2H, m, CH₂ β Trp); 5.40 (1H, m, CH α Trp); 5.69 (1H, d, J= 17 Hz, CH₂-naphtyle); 5.77 (1H, d, J= 17 Hz, CH₂-naphtyle); 6.17 (1H, d, Jₒ= 7 Hz, H₂ naphtyle); 6.71 (1H, t, Jₒ= 7 Hz, H₅ Trp); 6.96 (1H, t, Jₒ= 7 Hz, H₆ Trp); 6.98-7.16 (8H, m, H₃ naphtyle, CHaromatics phenyle, H₂ and H₄ Trp); 7.25 (1H, d, Jₒ= 8 Hz, H₇ Trp); 7.48-7.58 (5H, m, H_{4.} H_{5.} H_{6.} H₇ and H₈ naplatyle); 7.73 (1H, t, J= 7 Hz, NH amide); 7.84 (2H, m, H₄ and H₅ *o*-pyridyle); 8.39 (1H, d, J_{αβ}= 4 Hz, H₆ *o-*pyridyle); 9.06 (1H, d, Jₒ= 8 Hz, H₃ *o*-pyridyle); 10.74 (1H, s, NH indole Trp).
¹³C NMR (75 MHz, DMSO d^{6.} 300°K): δ(ppm) 26.0 (CH₂-CH₂-phenyle); 28.7 (CH₂ β Trp); 32.2 (CH₂-CH₂-phenyle); 44.6 (CH₂-naphtyle); 45.4 (CH α Trp); 109.7 (C₃ Trp); 111.7 (C₇ Trp); 118.3 (C₄ Trp); 118.7 (C₅ Trp); 121.3 (C₆ Trp); 121.8 (C₃ *o*-pyridyle); 122.0 (C₂ naphtyle); 123.2 (C₈ naphtyle); 124.5 (C₂ Trp); 125.4 (C₃ naphtyle); 126.6 (C₆ naphtyle and C₄ phenyle); 126.8 (C₄ naphtyle); 126.9 (C₅ naphtyle); 127.4 (Cg Trp); 128.0 (C₇ naphtyle and C₅ *o*-pyridyle); 128.7 (C_{2.} C_{3.} C₅ and C₆ phenyle); 129.7 (C₉ naphtyle); 130.7 (C₁ naphtyle); 133.3 (C₁₀ naphtyle); 136.4 (C₈ Trp); 137.8 (C₄ *o*-pyridyle); 140.4 (C₁ phenyle); 148.4 (C₆ *o*-pyridyle); 149.0 (C₂ *o*-pyridyle); 155.3 (Cquaternary triazole); 155.8 (Cquaternary triazole); 163.9 (CO amide).

### Compound 17:

1H NMR (300 MHz, DMSO d^{6.} 300°K): δ(ppm) 0.99 (2H, m, H₃ and H₅ piperidine); 1.22 (1H, m, H₃ piperidine); 1.40 (1H, m, H₅ piperidine); 1.93 (1H, m, H₄ piperidine); 2.57 (2H, m, H₂ and H₆ piperidine); 2.81-3.00 (6H, m, H₂ and H₆ piperidine, CH₂-CH₂-indole); 3.34 (2H, m, CH₂ β Trp); 5.17 (1H, m, CH α Trp); 5.49 (1H, d, J= 18 Hz, CH₂-naphtyle); 5.60 (1H, d, J= 18 Hz, CH₂-naphtyle); 6.22 (1H, d, Jo= 7 Hz, H₂ naphtyle); 6.70 (2H, t, Jₒ= 8 Hz, H₅ indole and H₅ Trp); 6.95-7.03 (4H, m, H₂ and H₆ Trp, H₂ and H₆ indole); 7.12 (3H, m, H₃ naphtyle, H₄ indole and H₄ Trp); 7.25 (2H, d, Jₒ= 8 Hz, H₇ indole and H₇ Trp); 7.55 (2H, m, H₆ and H₇ naphtyle); 7.78 (2H, m, H₅ and H₈ naphtyle); 7.94 (1H, m, H₄ naphtyle); 8.05 and 8.41 (2H, 2 m, NH piperidine TFA salt); 8.55 (1H, d, J= 8 Hz, NH amide); 10.72 (1H, s, NH indole); 10.73 (1H, s, NH indole Trp).
¹³C NMR (75 MHz, DMSO d^{6.} 300°K): δ(ppm) 23.0 (CH₂-CH₂-indole); 24.3 (C₃ piperidine); 25.3 (C₅ piperidine); 25.8 (CH₂-CH₂-indole); 29.0 (CH₂ β Trp); 38.4 (C₄ piperidine); 42.4 (C₂ piperidine); 42.5 (C₆ piperidine); 44.0 (CH₂-naphtyle); 44.6 (CH α Trp); 110.3 (C₃ Trp); 111.7 (C₇ indole and C₇ Trp); 113.2 (C₃ indole); 118.3 (C₄ indole); 118.4 (C₄ Trp); 118.5 (C₅ indole and C₅ Trp); 121.2 (C₆ Trp); 121.3 (C₆ indole); 121.9 (C₂ naphtyle); 123.0 (C₂ indole and C₈ naphtyle); 124.4 (C₂ Trp); 125.7 (C₃ naphtyle); 126.7 (C₆ naphtyle); 127.0 (C₇ naphtyle); 127.5 (C₉ Trp); 128.1 (C₉ indole); 128.9 (C₄ naphtyle); 129.9 (C₅ naphtyle); 131.7 (C₉ naphtyle); 133.5 (C₁ and C₁₀ naphtyle); 136.4 (C₈ Trp); 136.5 (C₈ indole); 155.4 (Cquaternary triazole); 155.7 (Cquaternary triazole); 172.8 (CO amide).

### Compound 18:

1H NMR (300 MHz, DMSO d^{6.} 300°K): δ(ppm) 2.97 (4H, m, CH₂-CH₂-indole); 3.52 (2H, m, CH₂ β Trp); 5.40 (1H, m, CH α Trp); 5.68 (1H, d, J= 18 Hz, CH₂-naphtyle); 5.74 (1h, d, J= 18 Hz, CH₂-naphtyle); 6.22 (1H, d, Jₒ= 7 Hz, H₂ naphtyle); 6.70 (2H, m, H₅ indole and H₅ Trp); 6.97 (5H, m, H_{2.} H₄ and H₆ Trp, H₂ and H₆ indole); 7.10 (2H, m, H₃ naphtyle and H₄ indole); 7.25 (2H, d, Jₒ= 8 Hz, H₇ indole and H₇ Trp); 7.52 (5H, m, H_{4.} H_{5.} H_{6.} H₇ and H₈ naphtyle); 7.56 (1H, t, J= 8 Hz, NH amide); 7.81 (2H, m, H₄ and H₅ *o*-pyridyle); 8.39 (1H, d, J_{αβ}= 5 Hz, H₆ *o-*pyridyle); 9.08 (1H, d, Jₒ= 8 Hz, H₃ *o*-pyridyle); 10.74 (2H, s, NH indole and NH indole Trp). ¹³C NMR (75 MHz, DMSO d^{6.} 300°K): δ(ppm) 22.6 (CH₂-CH₂-indole); 25.5 (CH₂-CH₂-indole); 28.6 (CH₂ β Trp); 44.7 (CH₂-naphtyle); 45.5 (CH α Trp); 109.7 (C₃ Trp); 111.7 (C₇ indole and C₇ Trp); 112.8 (C₃ indole); 118.3 (C₄ indole and C₄ Trp); 118.6 (C₅ indole); 118.7 (C₅ Trp); 121.3 (C₆ indole and C₆ Trp); 121.8 (C₂ naphtyle); 122.0 (C₃ *o*-pyridyle); 123.1 (C₂ indole and C₈ naphtyle); 124.4 (C₂ Trp); 125.4 (C₃ naphtyle); 126.5 (C₆ naphtyle); 126.8 (C₇ naphtyle); 126.9 (C₅ *o*-pyridyle); 127.0 (C₉ Trp); 127.4 (C₉ indole); 128.0 (C₄ naphtyle); 128.8 (C₅ naphtyle); 129.7 (C₉ naphtyle); 130.7 (C₁ naphtyle); 133.3 (C₁₀ naphtyle); 136.4 (C₈ Trp); 136.5 (C₈ indole); 137.8 (C₄ *o*-pyridyle); 149.0 (C₂ and C₆ *o*-pyridyle); 155.7 (Cquatemarytriazole); 155.8 (Cquaternary triazole); 163.9 (CO amide).

### Compound 19:

1H NMR (300 MHz, DMSO d^{6.} 300°K): δ(ppm) 0.75 (1H, m, H₅ tetrahydropyrane); 0.90-1.13 (3H, m, H₃ and H₅ tetrahydropyrane); 1.81 (H₄ tetrahydropyrane); 2.84-3.02 (6H, m, CH₂-CH₂-phenyle, H₂ and H₆ tetrahydropyrane); 3.31 (1H, dd, J= 14 Hz and 9 Hz, CH₂ β Trp); 3.38 (1H, dd, J= 14 Hz and 6 Hz, CH₂ β Trp); 3.54 (2H, m, H₂ and H₆ tetrahydropyrane);5.16 (1H, m, CH α Trp); 5.63 (1H, d, J= 17 Hz, CH₂-naphtyle); 5.71 (1H, d, J= 17 Hz, CH₂-naphtyle); 6.20 (1H, d, Jₒ= 7 Hz, H₂ naphtyle); 6.70 (1H, t, Jₒ= 8 Hz, H₅ Trp); 6.94 (1H, t, Jₒ= 8 Hz, H₆ Trp); 7.01-7.09 (8H, m, H₂ and H₄ Trp, CHaromatics phenyle, H₃ naphtyle); 7.24 (1H, d, Jo= 8 Hz, H₇ Trp); 7.56 (2H, m, H₆ and H₇ naphtyle); 7.80 (1H, d, Jₒ= 8 Hz, H₄ naphtyle); 7.92 (2H, m, H₅ and H₈ naphtyle); 8.37 (1H, d, J= 8 Hz, NH amide); 10.78 (1H, d, J= 2 Hz, NH indole Trp). ¹³C NMR (75 MHz, DMSO d^{6.} 300°K): δ(ppm) 26.1 (CH₂-CH₂-phenyle); 28.2 (C₃ and C₅ tetrahydropyrane); 29.0 (CH₂ β Trp); 32.2 (CH₂-CH₂-phenyle); 40.8 (C₄ tetrahydropyrane); 44.3 (CH₂-naphtyle); 44.6 (CH α Trp); 66.4 and 66.6 (C₂ and C₆ tetrahydropyrane); 110.0 (C₃ Trp); 111.7 (C₇ Trp); 118.3 (C₄ Trp); 118.6 (C₅ Trp); 121.2 (C₆ Trp); 121.9 (C₂ naphtyle); 123.1 (C₈ naphtyle); 124.5 (C₂ Trp); 125.7 (C₃ naphtyle); 126.6 (C₄ phenyle); 126.7 (C₆ naphtyle); 127.0 (C₄ naphtyle); 127.4 (C₇ naphtyle and C₉ Trp); 128.2 (C₅ naphtyle); 128.8 (C_{2.} C_{3.} C₅ and C₆ phenyle); 129.9 (C₉ naphtyle); 131.2 (C₁ naphtyle); 133.5 (C₁₀ naphtyle); 136.4 (C₈ Trp); 140.5 (C₁ phenyle); 155.1 (Cquaternary triazole); 156.1 (Cquaternary triazole); 173.9 (CO amide).

### Compound 20:

1H NMR (300 MHz, DMSO d^{6.} 300°K): δ(ppm) 0.65 (1H, d, J= 14 Hz, H₅ tetrahydropyrane); 0.90-1.12 (3H, m, H₃ and H₅ tetrahydropyrane); 1.80 (1H, m, H₄ tetrahydropyrane); 2.90-3.04 (6H, m, CH₂-CH₂-phenyle, H₂ and H₆ tetrahydropyrane); 3.28 (1H, dd, J= 14 Hz and 9 Hz, CH₂ β Trp); 3.40 (1H, dd, J= 14 Hz and 6 Hz, CH₂ β Trp); 3.53 (2H, m, H₂ and H₆ tetrahydropyrane); 5.16 (1H, m, CH α Trp); 5.58 (1H, d, J= 18 Hz, CH₂-naphtyle); 5.69 (1H, d, J= 18 Hz, CH₂-naphtyle); 6.25 (1H, d, Jₒ= 7 Hz, H₂ naphtyle); 6.72 (2H, t, Jₒ= 7 Hz, H₅ indole and H₅ Trp); 6.93-7.18 (7H, m, H_{2.} H₄ and H₆ indole, H_{2.} H₄ and H₆ Trp, H₃ naphtyle); 7.25 (2H, d, Jₒ= 8 Hz, H₇ indole and H₇ Trp); 7.55 (2H, m, H₆ and H₇ naphtyle); 7.80 (2H, m, H₄ and H₅ naphtyle); 7.92 (1H, m, H₈ naphtyle); 8.36 (1H, d, J= 8 Hz, NH amide); 10.72 (2H, s, NH indole and NH indole Trp).
¹³C NMR (75 MHz, DMSO d^{6.} 300°K): δ(ppm) 22.7 (CH₂-CH₂-indole); 25.6 (CH₂-CH₂-indole); 28.2 (C₃ and C₅ tetrahydropyrane); 29.0 (CH₂ β Trp); 40.5 (C₄ tetrahydropyrane); 44.4 (CH₂-naphtyle); 44.7 (CH α Trp); 66.4 and 66.6 (C₂ and C₆ tetrahydropyrane); 110.0 (C₃ Trp); 111.7 (C₇ indole and C₇ Trp); 112.9 (C₃ indole); 118.3 (C₄ indole and C₄ Trp); 118.6 (C₅ indole and C₅ Trp); 121.2 (C₆ indole and C₆ Trp); 121.3 (C₂ naphtyle); 121.9 (C₈ naphtyle); 123.0 (C₂ indole); 124.4 (C₂ Trp); 125.7 (C₃ naphtyle); 126.7 (C₆ naphtyle); 127.0 (C₇ naphtyle); 127.5 (C₉ indole); 128.2 (C₉ Trp); 128.9 (C₄ naphtyle); 129.9 (C₅ naphtyle); 131.2 (C₁ and C₉ naphtyle); 133.5 (C₁₀ naphtyle); 136.4 (C₈ Trp); 136.5 (C₈ indole); 155.6 (Cquaternary triazole); 156.1 (Cquaternary triazole); 173.9 (CO amide).

### Compound 21:

1H NMR (300 MHz, DMSO d^{6.} 300°K): δ(ppm) 1.86 (2H, m, CH₂-CH₂-CH₂-indole); 2.64 (4H, m, CH₂-CH₂-CH₂-indole); 3.51 (2H, m, CH₂ β Trp); 5.22 (2H, m, CH₂-phenyle); 5.48 (1H, m, CH α Trp); 6.78 (2H, m, H₂ and H₆ phenyle); 6.84 (2H, t, Jₒ= 8 Hz, H₅ indole and H₅ Trp); 6.90 (2H, t, Jₒ= 8 Hz, H₆ indole, and H₆ Trp); 6.97-7.07 (5H, m, H₂ indole, H₂ Trp, H_{3.} H₄ and H₅ phenyle); 7.27 (3H, d, Jₒ= 8 Hz, H₄ and H₇ Trp, H₇ indole); 7.38 (1H, d, Jₒ= 8 Hz, H₄ indole); 7.53 (1H, t, Jₒ= 8 Hz, NH amide); 7.85 (2H, m, H₄ and H₅ *o*-pyridyle); 8.55 (1H, d, J_{αβ}= 5 Hz, H₆ *o*-pyridyle); 9.22 (1H, d, Jₒ= 8 Hz, H₃ *o*-pyridyle); 10.70 (1H, s, NH indole); 10.78 (1H, s, NH indole Trp).
¹³C NMR (75 MHz, DMSO d^{6.} 300°K): δ(ppm) 24.0 (CH₂-CH₂-CH₂-indole); 24.4 (CH₂-CH₂-CH₂-indole); 27.1 (CH₂-CH₂-CH₂-indole); 28.6 (CH₂ β Trp); 45.6 (CH α Trp); 46.9 (CH₂-phenyle); 109.5 (C₃ Trp); 111.7 (C₇ indole and C₇ Trp); 113.7 (C₃ indole); 118.4 (C₄ indole); 118.5 (C₄ Trp); 118.7 (C₅ indole); 118.8 (C₅ Trp); 121.3 (C₆ Trp); 121.4 (C₆ indole); 122.4 (C₂ indole and C₃ *o*-pyridyle); 122.8 (C₂ Trp); 126.4 (C₂ and C₆ phenyle); 127.2 (C₅ *o*-pyridyle); 127.3 (C₉ indole and C₉ Trp); 128.0 (C₄ phenyle); 129.0 (C₃ and C₅ phenyle); 134.7 (C₁ phenyle); 136.4 (C₈ Trp); 136.7 (C₈ indole); 138.1 (C₄ *o*-pyridyle); 149.2 (C₂ and C₆ *o-*pyridyle); 155.4 (Cquaternary triazole); 155.8 (Cquaternary triazole); 164.1 (CO amide).

### Compound 22:

1H NMR (300 MHz, DMSO d^{6.} 300°K): δ(ppm) 1.36 (3H, m, H₃ and H₅ piperidine); 1.49 (1H, m, H₅ piperidine); 1.84 (2H, m, CH₂-CH₂-CH₂-indole); 2.15 (1H, m, H₄ piperidine); 2.54 (2H, t, J= 8 Hz, CH₂-CH₂-CH₂-indole); 2.62 (2H, t, J= 8 Hz, CH₂-CH₂-CH₂-indole); 2.69 (2H, m, H₂ and H₆ piperidine); 3.07 (2H, m, H₂ and H₆ piperidine); 3.33 (2H, m, CH₂ β Trp); 5.04 (2H, s, CH₂-phenyle); 5.21 (1H, m, CH α Trp); 6.73 (2H, d, Jₒ= 8 Hz, H₂ and H₆ phenyle); 6.85 (1H, t, Jₒ= 7 Hz, H₅ Trp); 6.91 (1H, t, Jₒ= 7 Hz, H₆ Trp); 6.95 (1H, d, J= 2 Hz, H₂ Trp); 7.00 (3H, m, H_{2.} H₅ and H₆ indole); 7.16 (3H, m, H_{3.} H₄ and H₅ phenyle); 7.28 (3H, m, H₄ Trp, H₄ and H₇ indole); 7.38 (1H, d, Jₒ= 8 Hz, H₇ Trp); 8.15 and 8.46 (2H, 2 m, NH piperidine TFA salt); 8.63 (1H, d, J= 8 Hz, NH amide); 10.69 (1H, s, NH indole); 10.75 (1H, d, J= 2 Hz, NH indole Trp). ¹³C NMR (75 MHz, DMSO d^{6.} 300°K): δ(ppm) 24.2 (CH₂-CH₂-CH₂-indole); 24.5 (CH₂-CH₂-CH₂-indole); 24.8 (C₃ piperidine); 25.4 (C₅ piperidine); 27.5 (CH₂-CH₂-CH₂-indole); 29.1 (CH₂ β Trp); 38.6 (C₄ piperidine); 42.6 (C₂ and C₆ piperidine); 44.8 (CH α Trp); 45.9 (CH₂-phenyle); 110.2 (C₃ Trp); 111.7 (C₇ indole and C₇ Trp); 118.5 (C₄ indole and C₄ Trp); 118.7 (C₅ indole and C₅ Trp); 121.2 (C₆ indole and C₆ Trp); 122.7 (C₂ indole); 124.3 (C₂ Trp); 126.3 (C₂ and C₆ phenyle); 127.4 (C₉ Trp); 127.5 (C₉ indole); 127.9 (C₄ phenyle); 129.0 (C₃ and C₅ phenyle); 135.9 (C₈ indole); 136.4 (C₈ Trp); 136.7 (C₁ phenyle); 155.1 (quaternary triazole); 155.4 (Cquaternary triazole); 173.1 (CO amide).

### Compound 23:

1H NMR (300 MHz, DMSO d^{6.} 300°K): δ(ppm) 1.12 (1H, m, H₅ tetrahydropyrane); 1.24 (3H, m, H₃ and H₅ tetrahydropyrane); 1.85 (2H, m, CH₂-CH₂-CH₂-indole); 2.10 (1H, m, H₄ tetrahydropyrane); 2.60 (4H, m, CH₂-CH₂-CH₂-indole); 3.11 (2H, m, H₂ and H₆ tetrahydropyrane); 3.33 (2H, m, CH₂ β Trp); 3.66 (2H, m, H₂ and H₆ tetrahydropyrane); 5.11 (2H, s, CH₂-phenyle); 5.24 (1H, m, CH α Trp); 6.76 (2H, d, Jₒ= 8 Hz, H₂ and H₆ phenyle); 6.85 (1H, t, Jₒ= 8 Hz, H₅ Trp); 6.91 (1H, t, Jₒ= 8 Hz, H₅ indole); 6.96 (2H, m, H₆ indole and H₆ Trp); 7.02 (2H, s, H₂ Trp and H₂ indole); 7.17 (3H, m, H₄ and H₇ Trp, H₇ indole); 7.28 (3H, m, H_{3.} H₄ and H₅ phenyle); 7.38 (1H, d, Jₒ= 8 Hz, H₄ indole); 8.48 (1H, d, Jo= 8 Hz, NH amide), 10.69 (1H, s, NH indole); 10.75 (1H, s, NH indole Trp).
¹³C NMR (75 MHz, DMSO d^{6.} 300°K): δ(ppm) 24.1 (CH₂-CH₂-CH₂-indole); 24.4 (CH₂-CH₂-CH₂-indole); 27.3 (CH₂-CH₂-CH₂-indole); 28.6 (C₃ and C₅ tetrahydropyrane); 29.1 (CH₂ β Trp); 40.8 (C₄ tetrahydropyrane); 44.8 (CH α Trp); 46.3 (CH₂-phenyle); 66.6 and 66.7 (C₂ and C₆ tetrahydropyrane); 109.9 (C₃ Trp); 111.7 (C₇ indole and C₇ Trp); 113.8 (C₃ indole); 118.4 (C₄ Trp and C₅ indole); 118.5 (C₄ indole); 118.7 (C₅ Trp); 121.3 (C₆ indole and C₆ Trp); 122.3 (C₂ indole and C₂ Trp); 126.4 (C₂ and C₆ phenyle); 127.4 (C₉ Trp); 127.5 (C₉ indole); 128.0 (C₄ phenyle); 129.0 (C₃ and C₅ phenyle); 135.5 (C₁ phenyle); 136.4 (C₈ Trp); 136.7 (C₈ indole); 155.4 (Cquaternary triazole); 155.7 (Cquaternary triazole); 174.2 (CO amide).

### Compound 29:

1H NMR (300 MHz, DMSO d^{6.} 300°K): δ(ppm) 3.03 (4H, m, CH₂-CH₂-indole); 3.56 (2H, m, CH₂ β Trp); 3.59 (3H, s, OCH₃); 5.18 (1H, d, J= 17 Hz, CH₂-*p*-methoxyphenyle); 5.23 (1H, d, J= 17 Hz, CH₂-*p*-methoxyphenyle); 5.54 (1H, m, CH α Trp); 6.56 (2H, d, Jₒ= 8 Hz, H₃ and H₅ *p*-methoxyphenyle); 6.72 (2H, d, Jₒ= 8 Hz, H₂ and H₆ *p*-methoxyphenyle); 6.90 (3H, t, Jₒ= 7 Hz, H₅ and H₆ Trp, H₅ indole); 7.03 (1H, m, H₆ indole); 7.09 (1H, d, J= 2 Hz, H₂ Trp); 7.12 (1H, d, J= 2 Hz, H₂ indole); 7.32 (3H, m, H₄ and H₇ indole, H₄ Trp); 7.41 (1H, d, Jₒ= 8 Hz, H₇ Trp); 8.64 (1H, dd, J= 2 Hz and 1 Hz, H₅ *o*-pyrazine); 8.81 (1H, d, J= 2 Hz, H₆ *o*-pyrazine); 8.94 (1H, d, J= 1 Hz, H₃ *o*-pyrazine); 9.37 (1H, d, J= 8 Hz, NH amide); 10.81 (2H, s large, NH indole and NH indole Trp).
¹³C NMR (75 MHz, DMSO d^{6.} 300°K): δ(ppm) 21.8 (CH₂-CH₂-indole); 25.1 (CH₂-CH₂-indole); 27.7 (CH₂ β Trp); 45.1 (CH α Trp); 45.9 (CH₂-*p*-methoxyphenyle); 54.9 (OCH₃); 109.2 (C₃ Trp); 111.3 (C₇ indole and C₇ Trp); 112.2 (C₃ indole); 113.7 (C₃ and C₅ *p*-methoxyphenyle); 118.0 (C₄ indole); 118.1 (C₄ Trp); 118.2 (C₅ indole); 118.3 (C₅ Trp); 120.9 (C₆ indole and C₆ Trp); 122.7 (C₂ indole); 124.9 (C₂ Trp); 126.0 (C₉ indole and C₉ Trp); 126.6 (C₁ *p-*methoxyphenyle); 127.0 (C₂ and C₆ *p*-methoxyphenyle); 136.0 (C₈ Trp); 136.1 (C₈ indole); 143.8 (C_{2.} C_{3.} C₅ and C₆ *o*-pyrazine); 154.7 (Cquaternary triazole); 155.2 (Cquaternary triazole); 158.4 (CO amide); 162.7 (C₄ *p*-methoxyphenyle).

### Compound 30:

1H NMR (300 MHz, DMSO d^{6.} 300°K): δ(ppm) 2.92 (2H, t, J= 6 Hz, CH₂-CH₂-indole); 2.99 (2H, m, CH₂-CH₂-indole); 3.37 (4H, m, CH₂ β Trp and CH₂-*o*-pyridyle); 5.13 (1H, m, CH α Trp); 5.57 (1H, d, J= 18 Hz, CH₂-naphtyle); 5.69 (1H, d, J= 18 Hz, CH₂-naphtyle); 6.22 (1H, d, Jₒ= 7 Hz, H₂ naphtyle); 6.67 (1H, t, Jₒ= 8 Hz, H₅ Trp); 6.77 (1H, t, Jₒ= 7 Hz, H₅ indole); 6.97-7.07 (6H, m, H₂, H₄ and H₆ Trp, H_{2.} H₄ and H₆ indole); 7.10 (1H, d, Jₒ= 8 Hz, H₇ Trp); 7.16 (1H, t, Jₒ= 8 Hz, H₃ naphtyle); 7.29 (2H, m, H₇ indole and H₃ *o*-pyridyle); 7.52 (1H, t, Jₒ= 8 Hz, H₅ *o-*pyridyle); 7.57 (2H, m, H₆ and H₇ naphtyle); 7.83 (3H, m, H₄ and H₈ naphtyle, H₄ *o*-pyridyle); 7.97 (1H, d, Jₒ= 7 Hz, H₅ naphtyle); 8.54 (1H, d, J_{αβ}= 5 Hz, H₆ *o*-pyridyle); 9.07 (1H, d, J= 8 Hz, NH amide); 10.75 (1H, s, NH indole); 10.79 (1H, s, NH indole Trp).
¹³C NMR (75 MHz, DMSO d^{6.} 300°K): δ(ppm) 22.9 (CH₂-CH₂-indole); 25.7 (CH₂-CH₂-indole); 29.3 (CH₂ β Trp); 42.0 (CH₂-*o*-pyridyle); 44.0 (CH₂-naphtyle); 45.2 (CH α Trp); 109.9 (C₃ Trp); 111.7 (C₇ indole and C₇ Trp); 118.3 (C₄ indole and C₄ Trp); 118.6 (C₅ indole and C₅ Trp); 121.2 (C₆ Trp); 121.3 (C₆ indole); 121.8 (C₂ naphtyle); 123.0 (C₂ indole and C₈ naphtyle); 123.6 (C₂ Trp); 125.7 (C₃ naphtyle, C₃ and C₅ *o*-pyridyle); 126.7 (C₆ naphtyle); 127.0 (C₇ naphtyle); 127.1 (C₉ indole); 127.3 (C₉ Trp); 128.2 (C₄ naphtyle); 129.0 (C₅ naphtyle); 129.8 (C₉ naphtyle); 131.4 (C₁ naphtyle); 133.4 (C₁₀ naphtyle); 136.4 (C₈ Trp); 136.5 (C₈ indole); 141.4 (C₄ and C₆ *o*-pyridyle); 153.4 (C₂ *o*-pyridyle); 155.4 (Cquaternary triazole); 155.7 (Cquaternary triazole); 167.9 (CO amide).

### Compound 31:

1H NMR (300 MHz, DMSO d^{6.} 300°K): δ(ppm) 1.39 (1H, m, H₃ proline); 1.53 (1H, m, H₄ proline); 1.73 (1H, m, H₄ proline); 2.11 (1H, m, H₃ proline); 2.91-3.06 (6H, m, CH₂-CH₂-indole and H₅ proline); 3.33 (2H, m, CH₂ β Trp); 4.00 (1H, m, CH α proline); 5.01 (1H, m, CH α Trp); 5.62 (1H, d, J= 18 Hz, CH₂-naphtyle); 5.73 (1H, d, J= 18 Hz, CH₂-naphtyle); 6.27 (1H, d, Jₒ= 7 Hz, H₂ naphtyle); 6.56 (1H, t, Jₒ= 7 Hz, H₅ indole); 6.78 (1H, t, Jₒ= 8 Hz, H₅ Trp); 6.79 (1H, d, Jₒ= 8 Hz, H₄ Trp); 6.93 (1H, t, Jₒ= 8 Hz, H₆ Trp); 7.00 (2H, m, H₆ indole and H₂ Trp); 7.05 (1H, s, H₂ indole); 7.14 (1H, d, Jₒ= 8 Hz, H₄ indole); 7.21-7.31 (3H, m, H₃ naphtyle, H₇ indole and H₇ Trp); 7.61 (2H, m, H₆ and H₇ naphtyle); 7.87 (1H, m, H₄ and H₅ naphtyle); 8.01 (1H, d, Jₒ= 8 Hz, H₈ naphtyle); 8.35 (1H, m, NH proline TFA salt); 9.11 (1H, m, NH proline TFA salt); 9.32 (1H, d, J= 8 Hz, NH amide); 10.75 (1H, s, NH indole); 10.80 (1H, d, J= 2 Hz, NH indole Trp). ¹³C NMM (75 MHz, DMSO d^{6.} 300°K): δ(ppm) 22.7 (C₄ proline); 23.0 (CH₂-CH₂-indole); 25.3 (CH₂-CH₂-indole); 29.3 (C₃ proline and CH₂ β Trp); 43.3 (CH₂-naphtyle); 44.8 (CH α Trp); 45.5 (C₅ proline); 58.8 (CH α proline); 109.2 (C₃ Trp); 111.3 (C₇ indole and C₇ Trp); 112.9 (C₃ indole); 117.6 (C₄ indole); 117.8 (C₅ indole); 118.1 (C₄ and C₅ Trp); 120.8 (C₆ indole and C₆ Trp); 121.4 (C₈ naphtyle); 122.4 (C₂ indole); 124.2 (C₂ Trp); 125.4 (C₃ naphtyle); 126.3 (C₆ naphtyle); 126.7 (C₇ naphtyle); 127.9 (C₉ indole and C₉ Trp); 128.6 (C₄ naphtyle); 129.4 (C₅ naphtyle); 131.4 (C₉ naphtyle); 133.1 (C₁ naphtyle); 135.9 (C₁₀ naphtyle); 136.1 (C₈ indole and C₈ Trp); 154.7 (Cquaternary triazole); 154.8 (Cquaternary triazole); 167.7 (CO amide).

### Compound 32:

¹H NMR (300 MHz, DMSO d^{6.} 300°K): δ(ppm) 3.02 (2H, m, CH₂-CH₂-indole); 3.08 (2H, m, CH₂-CH₂-indole); 3.60 (2H, m, CH₂ β Trp); 5.52 (1H, m, CH α Trp); 5.73 (2H, m, CH₂-naphtyle); 6.23 (1H, d, Jₒ= 7 Hz, H₂ naphtyle); 6.77 (2H, m, H₅ indole and H₅ Trp); 6.98 (4H, m, H₆ Trp, H₄ and H₆ indole); 7.07 (1H, s, H₂ indole); 7.11 (1H, d, J= 2 Hz, H₂ Trp); 7.16 (1H, d, Jₒ= 8 Hz, H₄ Trp); 7.28 (3H, m, H₇ indole, H₇ Trp and H₃ naphtyle); 7.52 (3H, m, H_{4.} H₆ and H₇ naphtyle); 7.84 (2H, m, H₅ and H₈ naphtyle); 8.39 (2H, m, H₅ and H₆ *o*-pyrazine); 8.66 (1H, d, J= 2 Hz, H₃ *o*-pyrazine); 9.22 (1H, d, J= 8 Hz, NH amide); 10.76 (1H, s, NH indole); 10.78 (1H, s, NH indole Trp).
¹³C NMR (75 MHz, DMSO d^{6.} 300°K): δ(ppm) 22.1 (CH₂-CH₂-indole); 25.0 (CH₂-CH₂-indole); 27.6 (CH₂ β Trp); 44.4 (CH₂-naphtyle); 45.0 (CH α Trp); 109.4 (C₃ Trp); 111.3 (C₇ indole and C₇ Trp); 112.4 (C₃ indole); 117.8 (C₄ indole); 118.0 (C₅ indole); 118.2 (C₄ and C₅ Trp); 120.9 (C₆ indole and C₆ Trp); 121.2 (C₂ naphtyle); 122.7 (C₂ indole and C₈ naphtyle); 124.8 (C₂ Trp and C₃ naphtyle); 126.1 (C₆ naphtyle); 126.3 (C₇ naphtyle); 126.6 (C₉ Trp); 127.0 (Cg indole); 127.3 (C₄ naphtyle); 128.3 (C₅ naphtyle); 129.1 (C₉ naphtyle); 130.1 (C₁ naphtyle); 132.7 (C₁₀ naphtyle); 136.0 (C₈ indole and C₈ Trp); 143.3 (C_{2.} C_{3.} C₅ and C₆ *o*-pyrazine); 155.0 (Cquaternary triazole); 155.6 (Cquaternary triazole); 162.4 (CO amide).

### Compound 33:

1H NMR (300 MHz, DMSO d^{6.} 300°K): δ(ppm) 1.37 (3H, m, H₃ and H₅ piperidine); 1.54 (1H, m, H₅ piperidine); 2.17 (1H, m, H₄ piperidine); 2.74 (2H, m, H₂ and H₆ piperidine); 3.09 (2H, m, H₂ and H₆ piperidine); 3.34 (2H, m, CH₂ β Trp); 4.02 (2H, s, CH₂-phenyle); 5.10 (3H, m, CH α Trp and CH₂-*m*,*p*-dichlorophenyle); 6.50 (1H, dd, Jₒ= 8 Hz and Jₘ= 2 Hz, H₆ *m*,*p-*dichlorophenyle); 6.83 (1H, d, J= 2 Hz, H₂ Trp); 6.87 (1H, t, Jₒ= 7 Hz, H₅ Trp); 7.03 (4H, m, H₆ Trp, H₂ and H₆ phenyle, H₅ *m*,*p*-dichlorophenyle); 7.15 (3H, m, H_{3.} H₄ and H₅ phenyle); 7.27 (3H, m, H₄ and H₇ Trp, H₂ *m*,*p*-dichlorophenyle); 8.19 (1H, m, NH piperidine TFA salt); 8.50 (1H, m, NH piperidine TFA salt); 8.67 (1H, d, j= 8 Hz, Nh amide); 10.77 (1H, d, J= 2 Hz, NH indole Trp).
¹³C NMR (75 MHz, DMSO d^{6.} 300°K): δ(ppm) 24.3 (C₃ piperidine); 25.0 (C₅ piperidine); 28.7 (CH₂ β Trp); 30.0 (CH₂-phenyle); 42.1 (C₂ and C₆ piperidine); 44.2 (CH α Trp); 44.6 (CH₂-*m*,*p-*dichlorophenyle); 109.7 (C₃ Trp); 111.3 (C₇ Trp); 117.9 (C₄ Trp); 118.2 (C₅ Trp); 120.8 (C₆ Trp); 124.6 (C₂ Trp); 125.8 (C₆ *m*,*p*-dichlorophenyle); 126.4 (C₄ phenyle); 127.0 (C₉ Trp); 127.8 (C₂ *m*,*p*-dichlorophenyle); 128.2 (C₂ and C₆ phenyle); 128.3 (C₃ and C₅ phenyle); 129.9 (C₄ *m*,*p*-dichlorophenyle); 130.2 (C₅ *m*,*p*-dichlorophenyle); 131.1 (C₃ *m*,*p*-dichlorophenyle); 135.5 (C₁ phenyle); 135.9 (C₈ Trp); 136.4 (C₁ *m*,*p*-dichlorophenyle); 153.4 (Cquaternary triazole); 155.4 (Cquaternary triazole); 172.5 (CO amide).

### Compound 34:

1H NMR (300 MHz, DMSO d^{6.} 300°K): δ(ppm) 3.36 (2H, m, CH₂ β Trp); 3.68 (2H, m, CH₂-*o-*pyridyle); 4.05 (2H, s, CH₂-phenyl); 5.11 (3H, m, CH α Trp and CH₂-*m*,*p*-dichlorophenyle); 6.35 (1H, dd, Jₒ= 8 Hz and Jₘ= 2 Hz, H₆ *m*,*p*-dichlorophenyle); 6.84 (2H, m, H₂ and H₅ Trp); 7.02 (4H, m, H₆ Trp, H₂ and H₆ phenyle, H₅ *m*,*p*-dichlorophenyle); 7.12-7.19 (5H, m, H₃. H₄ and H₅ phenyle, H₄ Trp and H₂ *m*,*p*-dichlorophenyle); 7.31 (2H, m, H₃ *o*-pyridyle and H₇ Trp); 7.61 (1H, t, Jₒ= 7 Hz, H₅ *o*-pyridyle); 8.06 (1H, t, Jₒ= 8 Hz, H₄ *o*-pyridyle); 8.63 (1H, d, J_{αβ}= 5 Hz, H₆ *o*-pyridyle); 9.15 (1H, d, J= 8 Hz, NH amide); 10.78 (1h, s, NH indole Trp).
¹³C NMR (75 MHz, DMSO d^{6.} 300°K): δ(ppm) 28.9 (CH₂ β Trp); 29.9 (CH₂-phenyle); 41.1 (CH₂-*o*-pyridyle); 44.7 (CH₂-*m*,*p*-dichorophenyle); 44.8 (CH α Trp); 109.3 (C₃ Trp); 111.3 (C₇ Trp); 117.7 (C₄ Trp); 118.2 (C₅ Trp); 120.8 (C₆ Trp); 123.6 (C₂ Trp); 125.7 (C₆ *m*,*p-*dichlorophenyle); 125.8 (C₄ phenyle); 126.5 (C₃ and C₅ *o*-pyridyle); 126.8 (C₉ Trp); 127.8 (C₂ *m*,*p*-dichlorophenyle); 128.3 (C₂ and C₆ phenyle); 128.4 (C₃ and C₅ phenyle); 130.0 (C₄ *m*,*p-*dichlorophenyle); 130.2 (C₅ *m*,*p*-dichlorophenyle); 131.1 (C₃ *m*,*p*-dichlorophenyle); 135.4 (C₁ phenyle); 135.9 (C₈ Trp); 136.1 (C₁ *m*,*p*-dichlorophenyle); 141.7 (C₄ and C₆ *o*-pyridyle); 152.5 (C₂ *o*-pyridyle); 153.4 (quaternary triazole); 155.2 (Cquaternary triazole); 167.2 (CO amide).

### Compound 35:

1H NMR (300 MHz, DMSO d^{6.} 300°K): δ(ppm) 1.44 (1H, m, H₃ proline); 1.56 (1H, m, H₄ proline); 1.74 (1H, m, H₄ proline); 2.11 (1H, m, H₃ proline); 3.08 (2H, m, H₅ proline); 3.31 (2H, m, CH₂ β Trp); 3.99 (1H, m, CH α proline); 4.09 (2H, m, CH₂-phenyle); 5.07 (1H, m, CH α Trp); 5.15 (2H, m, CH₂-*m*,*p*-dichlorophenyle); 6.43 (1H, dd, Jₒ= 8 Hz and Jₘ= 2 Hz, H₆ *m*,*p-*dichlorophenyle); 6.83 (1H, t, Jₒ= 8 Hz, H₅ Trp); 6.92 (1H, d, J= 2 Hz, H₂ Trp); 7.02 (1H, t, Jₒ= 8 Hz, H₆ Trp); 7.08-7.19 (7H, m, H₂ and H₅ *m,p*-dichlorophenyle, CHaromatics phenyle); 7.24 (1H, d, Jₒ= 8 Hz, H₄ Trp); 7.29 (1H, d, Jₒ= 8 Hz, H₇ Trp); 8.40 (1H, m, NH proline TFA salt); 9.11 (1H, m, NH proline TFA salt); 9.28 (1H, d, J= 8 Hz, NH amide); 10.81 (1H, d, J= 2 Hz, NH indole Trp).
¹³C NMR (75 MHz, DMSO d^{6.} 300°K): δ(ppm) 23.0 (C₄ proline); 29.0 (CH₂ β Trp); 29.3 (C₃ proline); 29.9 (CH₂-phenyle); 44.6 (CH₂-*m*,*p*-dichlorophenyle); 44.7 (CH α Trp); 45.5 (C₅ proline); 58.8 (CH α proline); 109.1 (C₃ Trp); 111.3 (C7 Trp); 117.7 (C₄ Trp); 118.2 (C₅ Trp); 120.9 (C₆ Trp); 124.2 (C₂ Trp); 125.8 (C₆ *m*,*p*-dichlorophenyle); 126.5 (C₄ phenyle); 126.7 (C₉ Trp); 128.0 (C₂ *m*,*p*-dichlorophenyle); 128.3 (C₂ and C₆ phenyle); 128.4 (C₃ and C₅ phenyle); 130.2 (C₄ *m*,*p*-dichlorophenyle); 130.5 (C₅ *m*,*p*-dichlorophenyle); 131.3 (C₃ *m*,*p-*dichlorophenyle); 125.7 (C₁ phenyle); 136.0 (C₈ Trp); 136.3 (C₁ *m*,*p*-dichlorophenyle); 153.5 (Cquaternary triazole); 154.7 (Cquaternary triazole); 167.8 (CO amide).

### Compound 36:

1H NMR (300 MHz, DMSO d^{6.} 300°K): δ(ppm) 3.56 (2H, m, CH₂ β Trp); 4.15 (2H, s, CH₂-phenyle); 5.22 (1H, d, J= 18 Hz, CH₂-*m*,*p*-dichlorophenyle); 5.36 (1H, d, J= 18 Hz, CH₂-*m*,*p-*dichlorophenyle); 5.47 (1H, m, CH α Trp); 6.54 (1H, dd, Jₒ= 8 Hz and Jₘ= 2 Hz, H₆ *m*,*p-*dichlorophenyle); 6.78 (1H, d, J= 2 Hz, H₂ Trp); 6.89 (1H, t, Jₒ= 7 Hz, H₅ Trp); 7.03 (1H, t, Jₒ= 8 Hz, H₆ Trp); 7.05 (1H, s, H₂ *m*,*p*-dichorophenyle); 7.08-7.19 (6H, m, CHaromatics phenyle); 7.29 (1H, d, Jₒ= 8 Hz, H₄ Trp); 7.37 (1H, d, Jₒ= 8 Hz, H₇ Trp); 7.73 (1H, m, NH amide); 7.80-7.87 (2H, m, H₄ and H₅ *o*-pyridyle); 8.50 (1H, d, J_{αβ}= 5 Hz, H₆ *o*-pyridyle); 9.12 (1H, d, Jₒ= 8 Hz, H₃ *o*-pyridyle); 10.78 (1H, s, NH indole Trp).
¹³C NMR (75 MHz, DMSO d^{6.} 300°K): δ(ppm) 28.3 (CH₂ β Trp); 30.1 (CH₂-phenyle); 45.4 (CH α Trp); 45.8 (CH₂-*m*,*p*-dichlorophenyle); 109.7 (C₃ Trp); 111.8 (C₇ Trp); 118.4 (C₄ Trp); 118.7 (C₅ Trp); 121.3 (C₆ Trp); 122.1 (C₃ *o*-pyridyle); 124.4 (C₂ Trp); 126.0 (C₆ *m*,*p-*dichlorophenyle); 127.0 (C₄ phenyle); 127.2 (C₅ *o*-pyridyle); 127.4 (C₉ Trp); 128.0 (C₂ *m*,*p-*dichlorophenyle); 128.8 (C₂ and C₆ phenyle); 129.0 (C₃ and C₅ phenyle); 130.2 (C₄ *m*,*p-*dichlorophenyle); 130.4 (C₅ *m*,*p*-dichlorophenyle); 131.4 (C₃ *m*,*p*-dichlorophenyle); 135.0 (C₁ phenyle); 135.8 (C₈ Trp); 136.4 (C₁ *m*,*p*-dichlorophenyle); 137.9 (C₄ *o*-pyridyle); 148.5 (C₆ *o-*pyridyle); 148.9 (C₂ *o*-pyridyle); 154.6 (Cquaternary triazole); 155.8 (Cquaternary triazole); 163.8 (CO amide).

### Compound 37:

1H NMR (300 MHz, DMSO d^{6.} 300°K): δ(ppm) 1.07 (1H, m, H₅ tetrahydropyrane); 1.25 (3H, m, H₃ and H₅ tetrahydropyrane); 2.07 (1H, m, H₄ tetrahydropyrane); 3.13 (2H, m, H₂ and H₆ tetrahydropyrane); 3.35 (2H, m, CH₂ β Trp); 3.69 (2H, m, H₂ and H₆ tetrahydropyrane); 4.07 (2H, s, CH₂-phenyle); 5.19 (3H, m, CH α Trp and CH₂-*m*,*p*-dichlorophenyle); 6.56 (1H, d, Jₒ= 8 Hz, H₆ *m*,*p*-dichlorophenyle); 6.86 (1H, s, H₂ Trp); 6.88 (1H, t, Jₒ= 7 Hz, H₅ Trp); 7.03 (4H, m, H₆ Trp, H₂ and H₆ phenyle, H₅ *m*,*p*-dichlorophenyle); 7.16 (4H, m, H_{3.} H₄ and H₅ phenyle, H₂ *m*,*p*-dichlorophenyle); 7.28 (2H, m, H₄ and H₇ Trp); 8.49 (1h, d, J= 8 Hz, NH amide); 10.77 (1H, s, NH indole Trp).
¹³C NMR (75 MHz, DMSO d^{6.} 300°K): δ(ppm) 28.6 (C₃ and C₅ tetrahydropyrane); 29.2 (CH₂ β Trp); 30.3 (CH₂ phenyle); 40.8 (C₄ tetrahydropyrane); 44.7 (CH α Trp); 45.4 (CH₂ *m*,*p-*dichlorophenyle); 66.5 and 66.7 (C₂ and C₆ tetrahydropyrane); 109.9 (C₃ Trp); 111.7 (C₇ Trp); 118.3 (C₄ Trp); 118.6 (C₅ Trp); 121.2 (C₆ Trp); 124.9 (C₂ Trp); 126.4 (C₆ *m*,*p*-dichlorophenyle); 127.1 (C₄ phenyle); 127.4 (C₉ Trp); 128.3 (C₂ *m*,*p*-dichlorophenyle); 128.7 (C₂ and C₆ phenyle); 128.9 (C₃ and C₅ phenyle); 130.5 (C₄ *m*,*p*-dichlorophenyle); 130.7 (C₅ *m*,*p*-dichlorophenyle); 131.6 (C₃ *m*,*p*-dichlorophenyle); 135.4 (C₁ phenyle); 136.4 (C₈ Trp); 136.5 (C₁ *m*,*p-*dichlorophenyle); 154.2 (Cquaternary triazole); 156.1 (Cquaternary triazole); 174.0 (CO amide).

### Compound 38:

1H NMR (300 MHz, DMSO d^{6.} 300°K): δ(ppm) 3.58 (2h, d, J= 7 Hz, CH₂ β Trp); 4.13 (2H, s, CH₂-phenyle); 5.19 (1H, d, J= 18 Hz, CH₂-*m*,*p*-dichlorophenyle); 5.34 (1H, d, J= 18 Hz, CH₂-*m*,*p*-dichlorophenyle), 5.48 (1H, m, CH α Trp); 6.55 (1H, d, Jₒ= 8 Hz, H₆ *m*,*p*-dichlorophenyle); 6.71 (1H, s, H₂ Trp); 6.88 (1H, t, Jₒ= 8 Hz, H₅ Trp); 7.02 (1H, t, Jₒ= 8 Hz, H₆ Trp); 7.03 (1H, d, Jₒ= 8 Hz, H₅ *m*,*p*-dichlorophenyle); 7.08-7.20 (6H, m, CHaromatics phenyle and H₂ *m*,*p-*dichlorophenyle); 7.28 (1H, d, Jₒ= 8 Hz, H₇ Trp); 7.40 (1H, d, Jₒ= 8 Hz, H₄ Trp); 8.56 (1H, s large, H₆ *o*-pyrazine); 8.77 (1H, d, J= 2 Hz, H₅ *o*-pyrazine); 8.82 (1H, s, H₃ *o*-pyrazine); 9.25 (1H, d, J= 8 Hz, NH amide); 10.75 (1H, s, NH indole Trp).

### Compound 39:

1H NMR (300 MHz, DMSO d^{6.} 300°K): δ(ppm) 1.01-1.26 (3H, m, H₃ and H₅ piperidine); 2.84 (1H, m, H₅ piperidine); 1.97 (3H, m, H₄ piperidine, CH₂-CH₂-CH₂-indole); 2.65 (6H, m, H₂ and H₆ piperidine, CH₂-CH₂-CH₂-indole); 2.96 (2H, m, H₂ and H₆ piperidine); 3.35 (2H, m, CH₂ β Trp); 5.15 (1H, m, CH α Trp); 5.56 (1H, d, J= 18 Hz, CH₂-naphtyle); 5.68 (1H, d, 18 Hz, CH₂-naphtyle); 6.26 (1H, d, Jₒ=7 Hz, H₇ naphtyle); 6.71 (1H, t, Jₒ=7 Hz, H₅ Trp); 6.88 (1H, t, Jₒ= 8 Hz, H₆ Trp); 6.95-7.05 (4H, m, H₂ Trp, H_{2.} H₅ and H₆ indole); 7.11 (1H, d, Jₒ= 8 Hz, H₄ Trp); 7.23-7.36 (4H, m, H₇ Trp, H₃ naphtyle, H₄ and H₇ indole); 7.60 (2H, m, H₆ and H₇ naphtyle); 7.83 (1H, d, Jₒ= 8 Hz, H₄ naphtyle); 7.88 (1H, m, H₅ naphtyle); 7.97 (1H, m, H₈ naphtyle); 8.20 (1H, s large, NH piperidine TFA salt); 8.40 (1H, s large, NH piperidine TFA salt); 8.58 (1H, d, J= 8 Hz, NH amide); 10.70 (1H, s, NH indole); 10.76 (1H, s, NH indole Trp).
¹³C NMR (75 MHz, DMSO d^{6.} 300°K): δ(ppm) 23.6 (CH₂-CH₂-CH₂-indole); 24.0 (C₃ and C₅ piperidine); 24.9 (CH₂-CH₂-CH₂-indole); 27.1 (CH₂-CH₂-CH₂-indole); 28.6 (CH₂ β Trp); 37.9 (C₄ piperidine); 41.9 and 42.1 (C₂ and C₆ piperidine); 43.6 (CH₂-naphtyle); 44.2 (CH α Trp); 109.8 (C₃ Trp); 111.2 (C₇ indole and C₇ Trp); 113.5 (C₃ indole); 117.9 (C₄ Trp); 118.0 (C₄ inddole); 118.1 (C₅ Trp and C₅ indole); 120.8 (C₆ indole and C₆ Trp); 121.4 (C₂ naphtyle); 122.2 (C₂ indole); 122.6 (C₈ naphtyle); 123.4 (C₂ Trp); 125.3 (C₃ naphtyle); 126.2 (C₆ naphtyle); 126.5 (C₇ naphtyle); 127.0 (C₉ indole and C₉ Trp); 127.6 (C₄ naphtyle); 128.5 (C₅ naphtyle); 129.4 (C₉ naphtyle); 131.3 (C₁ naphtyle); 133.0 (C₁₀ anphtyle); 135.9 (C₈ Trp); 136.2 (C₈ indole); 155.0 (Cquaternary triazole); 155.3 (Cquaternary triazole); 172.4 (CO amide).

### Compound 40:

1H NMR (300 MHz, DMSO d^{6.} 300°K): δ(ppm) 1.95 (2H, m, CH₂-CH₂-CH₂-indole); 2.67 (4H, m, CH₂-CH₂-CH₂-indole); 3.38 (3H, m, CH₂ β Trp and CH₂ *o*-pyridyle); 3.59 (1H, d, J= 15 Hz, CH₂-*o*-pyridyle); 5.10 (1H, m, CH α Trp); 5.58 (1H, d, J= 18 Hz, CH₂-naphtyle); 5.74 (1H, d, J= 18 Hz, CH₂-naphtyle); 6.24 (1H, d, Jₒ= 7 Hz, H₂ naphtyle); 6.64 (1H, t, Jₒ= 8 Hz, H₅ Trp); 6.88 (1H, t, Jₒ= 7 Hz, H₆ Trp); 6.97 (3H, m, H₂ Trp, H₂ and H₅ indole); 7.06 (2H, m, H₄ Trp, H₆ indole); 7.18 (1H, t, Jₒ= 8 Hz, H₅ *o*-pyridyle); 7.32 (4H, m, H₇ Trp, H₃ naphtyle, H₄ and H₇ indole); 7.50 (1H, t, Jₒ= 8 Hz, H₃ *o*-pyridyle); 7.58 (2H, m, H₆ and H₇ naphtyle); 7.80-7.97 (4H, m, H₄ *o*-pyridyle, H_{4.} H₅ and H₈ naphtyle); 8.55 (1H, d, J_{αβ}= 5 Hz, H₆ *o*-pyridyle); 9.08 (1h, d, J= 8 Hz, NH amide); 10.69 (1H, s, NH indole); 10.79 (1H, s, NH indole Trp). ¹³C NMR (75 MHz, DMSO d^{6.} 300°K): δ(ppm) 23.5 (CH₂-CH₂-CH₂-indole); 24.0 (CH₂-CH₂-CH₂-indole); 27.0 (CH₂-CH₂-CH₂-indole); 28.8 (CH₂ β Trp); 41.4 (CH₂-*o*-pyridyle); 43.8 (CH₂-naphtyle); 44.8 (CH α Trp); 109.4 (C₃ Trp); 111.2 (C₇ indole and C₇ Trp); 113.5 (C₃ indole); 117.7 (C₄ Trp); 118.0 (C₄ indole and C₅ Trp); 118.2 (C₅ indole); 120.8 (C₆ indole and C₆ Trp); 121.4 (C₃ *o*-pyridyle); 122.2 (C₂ naphtyle); 122.5 (C₈ naphtyle); 123.3 (C₂ Trp); 125.3 (C₅ *o-*pyridyle); 125.4 (C₃ naphtyle); 126.2 (C₆ naphtyle); 126.5 (C₇ naphtyle); 126.8 (C₉ indole); 126.9 (C₉ Trp); 127.8 (C₄ naphtyle); 128.6 (C₅ naphtyle); 129.4 (C₉ naphtyle); 1309 (C₁ naphtyle); 133.0 (C₁₀ naphtyle); 135.9 (C₈ Trp); 136.2 (C₈ indole); 140.8 (C₄ and C₆ *o*-pyridyle); 152.8 (C₂ *o*-pyridyle); 155.1 (Cquaternary triazole); 155.3 (Cquaternary triazole); 167.4 (CO amide).

### Compound 43:

1H NMR (300 MHz, DMSO d^{6.} 300°K): δ(ppm) 1.82 (4H, m, H₃ and H₅ piperidine, CH₂-CH₂-CH₂-indole); 2.36 (2H, m, H₃ and H₅ piperidine); 2.63 (4H, m, CH₂-CH₂-CH₂-indole); 3.09 (4H, m, H₂ and H₆ piperidine); 3.39 (2H, d, J= 7 Hz, CH₂ β Trp); 4.99 (2H, s, CH₂-phenyle); 5.24 (1H, m, CH α Trp); 6.76 (2H, d, Jₒ= 7 Hz, H₂ and H₆ phenyle); 6.87 (1H, t, Jₒ= 7 Hz, H₅ Trp); 6.95 (1H, t, Jₒ= 7 Hz, H₆ Trp); 7.02 (4H, m, H₂ Trp, H_{2.} H₅ and H₆ indole); 7.16-7.25 (4H, m, H₄ Trp, H₃, H₄ and H₅ phenyle); 7.33 (2H, m, H₄ indole and H₇ Trp); 7.42 (1H, d, Jₒ= 8 Hz, H₇ indole); 8.60 (1H, s large, NH piperidine TFA salt); 9.00 (1H, s large, 1H piperidine TFA salt); 9.04 (1H, d, J= 8 Hz, NH amide); 10.74 (1H, s, NH indole); 10.84 (1H, s, NH indole Trp). ¹³C NMR (75 MHz, DMSO d^{6.} 300°K): δ(ppm) 23.8 (C₃ and C₅ piperidine); 24.1 (CH₂-CH₂-CH₂-indole); 27.2 (CH₂-CH₂-CH₂-indole); 27.9 (CH₂-CH₂-CH₂-indole); 29.0 (CH₂ β Trp); 40.3 (C₂ and C₆ piperidine); 45.6 (CH₂-phenyle); 45.9 (CH α Trp); 55.5 (C₄ piperidine); 109.0 (C₃ Trp); 111.3 (C₇ Trp); 111.4 (C₇ indole); 113.5 (C₃ indole); 117.8 (C₄ indole); 118.1 (C₄ Trp and C₅ indole); 118.4 (C₅ Trp); 120.8 (C₆ indole); 121.0 (C₆ Trp); 122.3 (C₂ indole); 123.5 (C₂ Trp); 126.0 (C₂ and C₆ phenyle); 126.8 (C₉ Trp); 127.0 (C₉ indole); 127.7 (C₄ phenyle); 128.7 (C₃ and C₅ phenyle); 135.2 (C₁ phenyle); 136.0 (C₈ indole); 136.3 (C₈ Trp); 154.1 (Cquaternary triazole); 154.7 (Cquaternary triazole); 168.4 (CO amide).

### Compound 44:

1H NMR (300 MHz, DMSO d^{6.} 300°K): δ(ppm) 1.37 (1H, m, H₃ proline); 1.52 (1H, m, H₄ proline); 1.72 (1H, m, H₄ proline); 1.96 (2H, m, CH₂-CH₂-CH₂-indole); 2.10 (1H, m, H₃ proline); 2.71 (4H, m, CH₂-CH₂-CH₂-indole); 3.05 (2H, m, H₅ proline); 3.34 (2H, m, CH₂ β Trp); 4.00 (1H, m, CH α proline); 4.98 (1H, m, CH α Trp); 5.65 (1H, d, J= 18 Hz, CH₂-naphtyle); 5.78 (1H, d, J= 18 Hz, CH₂-naphtyle); 6.30 (1H, d, Jₒ= 7 Hz, H₂ naphtyle); 6.53 (1H, t, Jₒ= 7 Hz, H₅ Trp); 6.73 (1H, d, Jₒ= 8 Hz, H₄ Trp); 691 (3H, m, H₆ Trp, H₅ and H₆ indole); 6.99 (2H, s, H₂ Trp and H₂ indole); 7.04 (1H, m, H₄ indole); 7.24-7.36 (3H, m, H₇ Trp, H₇ indole, H₃ naphtyle); 7.62 (2H, In, H₆ and H₇ naphtyle); 7.87 (1H, d, Jₒ= 8 Hz, H₄ naphtyle); 7.92 (1H, m, H₅ naphtyle); 8.03 (1H, m, H₈ naphtyle); 8.33 (1H, m, NH proline TFA salt); 9.20 (1H, m, NH proline TFA salt); 9.32 (1H, d, J= 8 Hz, NH amide); 10.70 (1H, s, NH indole); 10.80 (1H, s, NH indole Trp).
¹³C NMR (75 MHz, DMSO d^{6.} 300°K): δ(ppm) 23.0 (C₄ proline); 23.7 (CH₂-CH₂-CH₂-indole); 24.1 (CH₂-CH₂-CH₂-indole); 27.3 (CH₂-CH₂-CH₂-indole); 29.3 (CH₂ β Trp and C₃ proline); 43.4 (CH₂-naphtyle); 44.7 (CH α Trp); 45.4 (C₅ proline); 58.8 (CH α proline); 109.2 (C₃ Trp); 111.2 (C₇ indole and C₇ Trp); 113.6 (C₃ indole); 117.6 (C₄ Trp); 118.0 (C₄ indole and C₅ Trp); 118.2 (C₅ indole); 120.7 (C₆ indole and C₆ Trp); 121.4 (C₂ naphtyle and C₇ indole); 122.5 (C₈ naphtyle and C₂ Trp); 125.4 (C₃ naphtyle); 126.3 (C₆ naphtyle); 126.6 (C₉ indole); 126.9 (C₉ Trp); 127.9 (C₇ naphtyle); 128.7 (C₄ and C₅ naphtyle); 129.4 (C₉ naphtyle); 131.4 (C₁ naphtyle); 133.1 (C₁₀ naphtyle); 135.9 (C₈ Trp); 136.2 (C₈ indole); 154.8 (Cquaternary triazole); 154.9 (quaternary triazole); 167.7 (CO amide).

### Compound 45:

1H NMR (300 MHz, DMSO d^{6.} 300°K): δ(ppm) 2.00 (2H, t, J= 7 Hz, CH₂-CH₂-CH₂-indole); 2.68 (2H, t, J= 7 Hz, CH₂-CH₂-CH₂-indole); 2.79 (2H, t, 7 Hz, CH₂-CH₂-CH₂-indole); 3.57 (2H, m, CH₂ β Trp); 5.47 (1H, m, CH α Trp); 5.77 (1H, d, J= 18 Hz, CH₂-naphtyle); 5.86 (1H, d, J= 18 Hz, CH₂-naphtyle); 6.33 (1H, d, Jₒ= 7 Hz, H₂ naphtyle); 6.73 (1H, t, Jₒ= 8 Hz, H₅ Trp); 6.89 (1H, t, Jₒ= 8 Hz, H₆ Trp); 6.98 (1H, t, Jₒ= 8 Hz, H₅ indole); 7.01-7.11 (4H, m, H₂ Trp, H₂. H₄ and H₆ indole); 7.29 (2H, m, H₄ Trp and H₇ indole); 7.37 (1H, d, Jₒ= 8 Hz, H₇ Trp); 7.48 (1H, m, H₃ naphtyle); 7.59 (2H, m, H₆ and H₇ naphtyle); 7.86 (2H, m, H₄ naphtyle and H₄ *o*-pyridyle); 8.00 (3H, m, H₅ and H₈ naphtyle, H₅ *o*-pyridyle); 8.39 (1H, d, J_{αβ}= 5 Hz, H₆ *o*-pyridyle); 9.17 (1H, d, Jₒ= 8 Hz, H₃ *o*-pyridyle); 10.72 (1H, s, NH indole); 10.80 (1h, d, J= 2 Hz, NH indole Trp). ¹³C NMR (75 MHz, DMSO d^{6.} 300°K): δ(ppm) 23.3 (CH₂-CH₂-CH₂-mdole); 23.9 (CH₂-CH₂-CH₂-indole); 26.6 (CH₂-CH₂-CH₂-indole); 27.9 (CH₂ β Trp); 44.7 (CH₂-naphtyle); 45.0 (CH α Trp); 109.1 (C₃ Trp); 111.3 (C₇ indole and C₇ Trp); 113.2 (C₃ indole); 117.9 (C₄ Trp); 118.1 (C₄ indole and C₅ Trp); 118.2 (C₅ indole); 121.4 (C₆ indole and C₆ Trp); 121.6 (C₂ naphtyle); 122.3 (C₂ indole and C₃ *o*-pyridyle); 122.7 (C₈ naphtyle and C₂ Trp); 125.3 (C₅ *o*-pyridyle); 126.1 (C₇ naphtyle); 126.4 (C₃ naphtyle); 126.9 (C₉ indole and C₉ Trp); 127.7 (C₅ naphtyle); 128.4 (C₄ naphtyle); 129.2 (C₉ naphtyle); 129.8 (C₁ naphtyle); 132.9 (C₁₀ naphtyle); 136.1 (C₈ Trp); 136.2 (C₈ indole); 137.3 (C₄ *o*-pyridyle); 148.4 (C₂ and C₆ *o*-pyridyle); 155.4 (Cquaternary triazole); 155.8 (Cquaternary triazole); 163.5 (CO amide).

### Compound 46:

1H NMR (300 MHz, DMSO d^{6.} 300°K): δ(ppm),75 (1H, m, H₅ tetrahydropyrane); 1.07 (3H, m, H₃ and H₅ tetrahydropyrane); 1.83 (1H, m, H₄ tetrahydropyrane); 1.99 (2H, t, J= 7 Hz, CH₂-CH₂-CH₂-indole); 2.70 (4H, m, CH₂CH₂-CH₂-indole); 3.01 (2H, m, H₂ and H₆ tetrahydropyrane); 3.37 (2H, m, CH₂ β Trp); 3.58 (2H, m, H₂ and H₆ tetrahydropyrane); 5.20 (1H, m, CH α Trp); 5.68 (1H, d, J= 18 Hz, CH₂-naphtyle); 5.80 (1H, d, J= 18 Hz, CH₂-naphtyle); 6.35 (1H, d, Jₒ= 7 Hz, H₂ naphtyle); 6.73 (1H, t, Jₒ= 8 Hz, H₅ Trp); 6.89 (1H, t, Jₒ= 8 Hz, H₆ Trp); 6.98-7.07 (4H, m, H₂ Trp, H₂. H₅ and H₆ indole); 7.12 (1H, d, Jₒ= 8 Hz, H₄ Trp); 7.29 (3H, m, H₃ naphtyle, H₄ and H₇ indole); 7.35 (1H, d, Jₒ= 8 Hz, H₇ Trp); 7.61 (2H, m, H₆ and H₇ naphtyle); 7.85 (2H, m, H₄ and H₅ naphtyle); 797 (1H, m, H₈ naphtyle); 8.42 (1H, d, J= 8 Hz, Nh amide); 10.71 (1H, s, NH indole); 10.78 (1H, d, J= 2 Hz, NH indole Trp).
¹³C NMR (75 MHz, DMSO d^{6.} 300°K): δ(ppm) 23.4 (CH₂-CH₂-CH₂-indole); 23.9 (CH₂-CH₂-CH₂-indole); 26.7 (CH₂-CH₂-CH₂-indole); 27.8 (CH₂ β Trp); 28.6 (C₃ and C₅ tetrahydropyrane); 40.1 (C₄ tetrahydropyrane); 44.3 (CH α Trp); 44.3 (CH₂-naphtyle); 66.0 and 66.1 (C₂ and C₆ tetrahydropyrane); 109.3 (C₃ Trp); 111.3 (C₇ indole and C₇ Trp); 113.3 (C₃ indole); 117.8 (C₄ Trp); 118.0 (C₄ indole and C₅ Trp); 118.1 (C₅indole); 120.8 (C₆ indole and C₆ Trp); 121.4 (C₂ naphtyle); 122.2 (C₂ indole); 122.6 (C Trp and C₈ naphtyle); 125.2 (C₃ naphtyle); 126.3 (C₆ naphtyle); 126.6 (C₇ naphtyle); 126.9 (C₉ indole and C₉ Tr); 127.8 (C₄ naphtyle); 128.5 (C₅ naphtyle); 129.4 (C₉ naphtyle); 130.4 (C₁ naphtyle); 133.1 (C₁₀ naphtyle); 135.9 (C₈ Trp); 136.2 (C₈ indole); 155.5 (Cquaternary triazole); 155.7 (Cquaternary triazole); 173.5 (CO amide).

### Compound 47:

1H NMR (300 MHz, DMSO d^{6.} 300°K): δ(ppm) 1.84 (2H, m, H₃ and H₅ piperidine); 2.39 (2H, m, H₃ and H₅ piperidine); 4.45 (6H, m, CH₂CH₂-indole, H₂ and H₆ piperidine); 3.13 (2H, m, H₂ and H₆ piperidine); 3.40 (2H, d, J= 7 Hz, CH₂ β Trp); 3.69 (3H, s, OCH₃); 4.96 (2H, s, CH₂-*p-*methoxyphenyle); 5.27 (1H, m, CH α Trp); 6.72 (4H, s, CHaromatics *p*-mthoxypheyle); 7.05 (4H, m, H₂. H₅ and H₆ indole, H₂ Trp); 7.24 (1H, d, Jₒ= 8 Hz, H₄ Trp); 7.34 (3H, m, H₄ and H₇ indole, H₇ Trp); 8.62 (1H, s large, NH piperidine TFA salt); 9.04 (2H, m, NH piperidine TFA salt); 10.79 (1H, s, NH indole); 10.85 (1H, s, NH indole Trp).
¹³C NMR (75 MHz, DMSO d^{6.} 300°K): δ(ppm) 22.3 (C₃ and C₅ piperidine); 25.4 (CH₂-CH₂-indole); 27.9 (CH₂-CH₂-indole); 29.1 (CH₂ β Trp); 40.1 (C₂ and C₆ piperidine); 45.0 (CH₂-*p-*methoxyphenyle); 45.9 (CH α Trp); 55.5 (OCH₃); 55.5 (C₄ piperidine); 109.2 (C₃ Trp); 111.3 (C₇ indole and C₇ Trp); 113.0 (C₃ indole); 114.0 (C₃ and C₅ *p*-methoxyphenyle); 117.9 (C₄ Trp); 118.0 (C₅ Trp); 118.2 (C₄ indole); 118.4 (C₅ indole); 120.9 (C₆ indole); 121.0 (C₆ Trp); 126.7 (C₉ indole); 126.8 (C₉ Trp); 127.2 (C₁ *p*-methoxyphenyle); 127.3 (C₂ and C₆ *p-*methoxyphenyle); 136.0 (C₈ Trp); 136.1 (C₈ indole); 154.0 (Cquaternary triazole); 154.5 (Cquaternary triazole); 158.7 (C₄ *p*-methoxyphenyle); 168.4 (CO amide).

### Compound 48:

1H NMR (300 MHz, DMSO d^{6.} 300°K): δ(ppm) 1.77 (2H, m, H₃ and H₅ piperidine); 2.33 (2H, m, H₃ and H₅ piperidine); 2.86-3.02 (6H, m, CH₂-CH₂-indole, H₂ and H₆ piperidine); 3.10 (2H, m, H₂ and H₆ piperidine); 3.36 (2H, d, J= 7 Hz, CH₂ β Trp); 5.07 (1H, m, CH α Trp); 5.57 (2H, m, CH₂-naphtyle); 6.25 (1H, d, Jₒ= 7 Hz, H₇ naphtyle); 6.56 (1H, t, Jₒ= 8 Hz, H₅ Trp); 6.77 (1H, t, Jₒ= 8 Hz, H₆ Trp); 6.83 (1H, d, Jₒ= 8 Hz, H₄ Trp); 6.99 (3H, m, H₂ Trp, H₅ and H₆ indole); 7.07 (1H, d, J= 2 Hz, H₂ indole); 7.11 (1H, d, Jₒ= 8 Hz, H₄ indole); 7.19 (1H, t, Jₒ= 8 Hz, H₃ naphtyle); 7.29 (2H, m, H₇ indole and H₇ Trp); 7.61 (2H, m, H₆ and H₇ naphtyle); 7.84 (2H, m, H₄ and H₅ naphtyle); 8.00 (1H, d, Jₒ= 8 Hz, H₈ naphtyle); 8.70 (1h, s large, NH piperidine TFA salt); 9.06 (2H, m, NH amide and NH piperidine TFA salt); 10.75 (1H, s, NH indole); 10.82 (1H, d, J= 2 Hz, NH indole Tip).
¹³C NMR (75 MHz, DMSO d^{6.} 300°K): δ(ppm) 22.6 (C₃ and C₅ piperidine); 25.3 (CH₂-CH₂-indole); 27.9 (CH2-CH2-indole); 29.2 (CH₂ β Trp); 40.1 (C₂ and C₆ piperidine); 45.9 (CH₂-naphtyle); 45.9 (CH α Trp); 55.5 (C₄ piperidine); 109.2 (C₃ Trp); 111.3 (C₇ Trp); 111.4 (C₇ indole); 112.9 (C₃ indole); 117.5 (C₄ indole); 117.8 (C₄ Trp); 118.1 (C₅ indole); 118.2 (C₅ Trp); 120.8 (C₆ indole and C₆ Trp); 121.5 (C₂ Trp and C₂ naplatyle); 122.4 (C₂ indole and C₈ naphtyle); 125.3 (C₃ naphtyle); 126.3 (C₆ naphtyle); 126.6 (C₇ naphtyle, C₉ indole and C₉ Trp); 127.9 (C₄ naphtyle); 128.6 (C₅ naphtyle); 129.4 (C₉ naphtyle); 131.2 (C₁ naphtyle); 133.1 (C₁₀ naphtyle); 136.0 (C₈ Trp); 136.1 (C₈ indole); 154.5 (Cquatemarytriazole); 154.8 (Cquaternary triazole); 168.5 (CO amide).

### Compound 49:

1H NMR (300 MHz, DMSO d^{6.} 300°K): δ(ppm) 1.76 (2H, m, H₃ and H₅ piperidine); 2.31 (2H, m, H₃ and H₅ piperidine); 2.86 (4H, m, CH₂-CH₂-phenyle, H₂ and H₆ piperidine); 3.11 (4H, m, H₂ and H₆ piperidine, CH₂-CH₂-phenyle); 3.35 (2H, d, J= 7 Hz, CH₂ β Trp); 5.06 (1H, m, CH α Trp); 5.60 (2H, m, CH₂-naphtyle); 6.20 (1H, d, Jₒ= 7 Hz, H₂ naphtyle); 6.55 (1H, t, Jₒ= 7 Hz, H₅ Trp); 6.80 (1H, d, Jₒ= 8 Hz, H₄ Trp); 6.96 (1H, t, Jₒ= 8 Hz, H₆ Trp); 7.05-7.09 (4H, m, H₂ Trp, H₃. H₄ and H₅ phenyle); 7.14-7.21 (2H, m, H₂ and H₆ phenyle); 7.30 (1H, d, Jₒ= 8 Hz, H₇ Trp); 7.61 (2H, m, H₆ and H₇ naphtyle); 7.84 (1H, d, Jₒ= 8 Hz, H₄ naphtyle); 8.00 (2H, m, H₅ and H₈ naphtyle); 8.57 (1H, s large, NH piperidine TFA salt); 9.04 (2H, m, NH amide and NH piperidine TFA salt); 10.82 (1H, d, J= 2 Hz, NH indole Trp).
¹³C NMR (75 MHz, DMSO d^{6.} 300°K): δ(ppm) 25.6 (C₃ and C₅ piperidine); 27.9 (CH₂-CH₂-phenyle); 29.2 (CH₂ β Trp); 32.2 (CH₂-CH₂-phenyle); 40.1 (C₂ and C₆ piperidine); 43.2 (CH₂-naphtyle); 45.8 (CH α Trp); 55.4 (C₄ piperidine); 109.2 (C₃ Trp); 111.4 (C₇ Trp); 118.2 (C₄ Trp); 120.8 (C₅ Trp), 121.5 (C₆ Trp); 122.5 (C₂ Trp); 124.3 (C₂ and C₈ naphtyle); 125.4 (C₃ naphtyle); 126.0 (C₄ phenyle); 126.3 (C₆ naphtyle); 126.6 (C₉ Trp); 127.9 (C₄ and C₇ naphtyle); 128.2 (C_{2.} C_{3.} C₅ and C₆ phenyle); 128.6 (C₅ naphtyle); 129.5 (C₉ naphtyle); 131.2 (C₁ naphtyle); 133.1 (C₁₀ naphtyle); 136.0 (C₈ Trp); 140.4 (C₁ phenyle); 154.3 (Cquaternary triazole); 154.6 (Cquaternary triazole); 168.5 (CO amide).

### Compound 50:

1H NMR (300 MHz, DMSO d^{6.} 300°K): δ(ppm) 2.89 (4H, m, CH₂-CH₂-phenyle); 3.34-3.64 (4H, m, CH₂-*o*-pyridyle and CH₂ β Trp); 5.12 (1H, m, CH α Trp); 5.5 (1H, d, J= 18 Hz, CH₂-naphtyle); 5.72 (1H, d, J= 18 Hz, CH₂-naphtyle); 6.16 (1H, d, Jₒ= 7 Hz, H₂ naphtyle); 6.65 (1H, t, Jₒ= 8 Hz, H₅ Trp); 697 (2H, m, H₄ phenyle and H₆ Trp); 6.98-7.08 (3H, m, H₃ naphtyle, H₂ and H₄ Trp); 7.14-7.19 (4H, m, H_{2.} H_{3.} H₅ and H₆ phenyle); 7.29 (1H, d, Jₒ= 8 Hz, H₇ Trp); 7.56-7.59 (4H, m, H₃ and H₅ *o*-pyridyle, H₆ and H₇ naphtyle); 7.79-7.95 (4H, m, H₄ *o*-pyridyle, H₄. H₅ and H₈ naphtyle); 8.57 (1H, d, J_{αβ}= 5 Hz, H₆ *o*-pyridyle); 9.10 (1H, d, J= 8 Hz, NH amide); 10.79 (1H, s, NH indole Trp).
¹³C NMR (75 MHz, DMSO d^{6.} 300°K): δ(ppm) 26.2 (CH₂-CH₂-phenyle); 29.3 (CH₂ β Trp); 32.5 (CH₂-CH₂-phenyle); 41.7 (CH₂-*o*-pyridyle); 44.0 (CH₂-naphtyle); 45.2 (CH α Trp); 109.9 (C₃ Trp); 111.7 (C₇ Trp); 118.2 (C₄ Trp); 118.6 (C₅ Trp); 121.2 (C₆ Trp); 121.8 (C₂ naphtyle); 123.0 (C₈ naphtyle); 123.8 (C₂ Tip); 125.7 (C₃ and C₅ *o*-pyridyle); 126.0 (C₃ naphtyle); 126.5 (C₆ naphtyle); 126.7 (C₄ phenyle); 127.1 (C₇ naphtyle); 128.2 (C₉ Trp); 128.7 (C₄ naphtyle); 129.0 (C_{2.} C_{3.} C₅ and C₆ phenyle); 129.8 (C₅ naphtyle); 131.4 (C₉ Trp); 131.4 (C₉ naphtyle); 133.4 (C₁ naphtyle); 136.4 (C₁₀ naphtyle and C₈ Trp); 140.7 (C₁ phenyle); 141.4 (C₄ and C₆ *o-*pyridyle); 153.0 (C₂ *o*-pyridyle); 154.8 (Cquaternary triazole); 155.7 (Cquaternary triazole); 167.7 (CO amide).

### Compound 51:

1H NMR (300 MHz, DMSO d^{6.} 300°K): δ(ppm) 2.03 (2H, m, CH₂-CH₂-CH₂-indole); 2.65-2.78 (4H, m, CH₂-CH₂-CH₂-indole); 3.59 (2H, m, CH₂ β Trp); 5.50 (1H, m, CH α Trp); 5.75 (2H, m, CH₂-naphtyle); 6.26 (1H, d, Jₒ= 7 Hz, H₂ naphtyle); 6.75 (1H, t, Jₒ= 8 Hz, H₅ Trp); 6.89 (1H, t, Jₒ= 8 Hz, H₆ Trp); 6.98-7.05 (3H, m, H₂. H₅ and H₆ indole); 7.11 (1H, s, H₂ Trp); 7.22-7.31 (3H, m, H₄ Trp, H₄ and H₇ indole); 7.39 (1H, d, Jₒ= 8 Hz, H₇ Trp); 7.49-7.60 (3H, m, H₄ H₆ and H₇ naphtyle); 7.83 (2H, m, H₅ and H₈ naphtyle); 8.40 (2H, m, H₅ and H₆ *o*-pyridyle); 8.66 (1H, d, J= 2 Hz, H₃ *o*-pyridyle); 9.20 (1h, d, J= 8 Hz, NH amide); 10.70 (1H, s, NH indole); 10.76 (1H, d, J= 2 Hz, NH indole Trp).
¹³C NMR (75 MHz, DMSO d^{6.} 300°K): δ(ppm) 22.2 (CH₂-CH₂-CH₂-indole); 23.8 (CH₂-CH₂-CH₂-indole); 27.3 (CH₂-CH₂-CH₂-indole); 28.1 (CH₂ β Trp); 44.9 (CH₂-naphtyle); 45.5 (CH α Trp); 109.9 (C₃ Trp); 111.7 (C₇ indole and C₇ Trp); 113.8 (C₃ indole); 118.4 (C₄ Trp); 118.5 (C₄ indole and C₅ Trp); 118.6 (C₅ indole); 121.3 (C₆ Trp); 121.7 (C₆ indole); 122.7 (C₂ naphtyle); 123.2 (C₈ naphtyle); 124.4 (C₂ Trp); 124.7 (C₂ indole); 125.3 (C₃ naphtyle); 126.5 (C₆ naphtyle); 126.8 (C₇ naphtyle); 127.4 (C₉ indole); 127.8 (C₄ and C₅ naphtyle); 128.8 (C₉ Trp); 129.6 (C₉ naphtyle); 130.7 (C₁ naphtyle); 133.2 (C₁₀ naphtyle); 136.4 (C₈ Trp); 136.7 (C₈ indole); 143.7 (C_{2.} C_{3.} C₅ and C₆ *o*-pyridyle); 155.4 (Cquaternary triazole); 156.2 (Cquaternary triazole); 162.8 (CO amide).

### Compound 52:

1H NMR (300 MHz, DMSO d^{6.} 300°K): δ(ppm) 2.87-2.98 (4H, m, CH₂)-CH₂-indole); 3.39 (2H, m, CH₂ β Trp); 3.69 (2H, m, CH₂-*o*-pyridyle); 5.17 (3H, m, CH₂-*m,p*-dichlorophenyle and CH α Trp); 6.49 (1H, dd, Jₒ= 8 Hz and Jₘ= 2 Hz, H₆ *m,p*-dichlorophenyle); 6.90 (2H, m, H₅ indole and H₅ Trp); 7.02-7.11 (5H, m, H₂ and H₆ indole, H₂ and H₆ Trp, H₂ *m*,*p*-dichlorophenyle); 7.17 (1H, m, H₄ Trp and H₃ *o*-pyridyle); 7.22-7.33 (4H, m, H₄ and H₇ indole, H₇ Trp, H₅ *m*,*p-*dichlorophenyle); 7.59 (1H, t, Jₒ= 8 Hz, H₅ *o*-pyridyle); 8.05 (1H, td, Jₒ= 8 Hz and Jₘ= 2 Hz, H₄ *o*-pyridyle); 8.63 (1H, d, J_{αβ}= 5 Hz, H₆ *o*-pyridyle); 9.18 (1H, d, J= 8 Hz, NH amide) 10.79 (2H, m, NH indole and NH indole Trp).
¹³C NMR (75 MHz, DMSO d^{6.} 300°K): δ(ppm) 22.2 (CH₂-CH₂-indole); 25.2 (CH₂-CH₂-indole); 28.8 (CH₂ β Trp); 41.3 (CH₂-*o*-pyridyle); 44.6 (CH₂-*m*,*p*-dichlorophenyle); 44.8 (CH α Trp); 109.2 (C₃ Trp); 111.4 (C₇ indole and C₇ Trp); 112.6 (C₃ indole); 117.7 (C₄ Trp); 117.9 (C₅ indole); 118.2 (C₅ Trp); 118.3 (C₄ indole); 120.9 (C₆ indole and C₆ Trp); 123.6 (C₂ indole and C₂ Trp); 125.7 (C₃ and C₅ *o*-pyridyle); 126.6 (C₉ indole); 126.8 (C₉ Trp); 128.0 (C₂ *m*,*p-*dichlorophenyle); 128.3 (C₄ *m*,*p*-dichlorophenyle); 130.3 (C₄ *m*,*p*-dichlorophenyle); 130.6 (C₅ *m*,*p*-dichlorophenyle); 131.4 (C₃ *m*,*p*-dichlorophenyle); 135.9 (C₈ Trp); 136.1 (C₁ *m*,*p-*dichlorophenyle); 136.3 (C₈ indole); 167.5 (CO amide).

### Compound 53:

1H NMR (300 MHz, DMSO d⁶ 300°K): δ(ppm) 1.45 (1H, m, H₃ proline); 1.56 (1H, m, H₄ proline); 1.76 (1H, m, H₄ proline); 2.10 (1H, m, H₃ proline); 2.93-3.05 (4H, m, CH2-CH₂-indole); 3.10 (2H, m, H₅ proline); 3.35 (2H, d, J= 8 Hz, CH₂ β Trp); 4.01 (1H, m, CH α proline); 5.15 (3H, m, CH₂-*m*,*p*-dichlorophenyle and CH α Trp); 6.54 (1H, dd, Jₒ= 8 Hz and Jₘ= 2 Hz, H₆ *m*,*p*-dichlorophenyle); 6.87 (1H, t, Jₒ= 8 Hz, H₅ indole); 6.93 (1H, t, Jₒ= 8 Hz, H₅ Trp); 7.03-7.11 (4H, m, H₂ and H₆ Trp, H₂ *m*,*p*-dichlorophenyle and H₆ indole); 7.14 (1H, d, Jₒ= 8 Hz, H₄ Trp); 7.25 (1H, d, J= 2 Hz, H₂ indole); 7.29-7.36 (4H, m, H₄ and H₇ indole, H₇ Trp, H₅ *m,p-*dichlorophenyle); 8.43 (1H, m, NH proline TFA salt); 9.25 (1H, m, NH proline TFA salt); 9.31 (1H, d, J= 8 Hz, NH amide); 10.81 (1H, s, NH indole Trp); 10.84 (1H, d, J= 2 Hz, NH indole). ¹³C NMR (75 MHz, DMSO d^{6.} 300°K): δ(ppm) 22.4 (CH₂-CH₂-indole); 23.1 (C₄ proline); 25.3 (CH₂-CH₂-indole); 28.9 (CH₂ β Trp); 29.4 (C₃ proline); 44.4 (CH₂-*m*,*p*-dichlorophenyle); 44.7 (CH α Trp); 45.5 (C₅ proline); 58.8 (CH α proline); 109.1 (C₃ Trp); 111.4 (C₇ indole and C₇ Trp); 112.8 (C₃ indole); 117.7 (C₄ Trp); 117.9 (C₅ indole); 118.2 (C₅ Trp and C₄ indole); 120.9 (C₆ indole and C₆ Trp); 122.6 (C₂ indole and C₂ Trp); 125.7 (C₂ *m*,*p*-dichlorophenyle); 126.7 (C₉ indole and C₉ Trp); 128.1 (C₆ *m*,*p*-dichlorophenyle); 130.4 (C₄ *m*,*p*-dichlorophenyle); 130.9 (C₅ *m*,*p*-dichlorophenyle); 131.5 (C₃ *m*,*p*-dichlorophenyle); 136.0 (C₈ Trp); 136.1 (C₈ indole); 136.6 (C₁ *m*,*p*-dichlorophenyle); 154.3 (Cquaternary triazole); 154.6 (Cquaternary triazole); 167.9 (CO amide).

### Compound 54:

1H NMR (300 MHz, DMSO d^{6.} 300°K): δ(ppm) 1.31 (3H, m, H₃ and H₅ piperidine); 1.54 (1H, m, H₃ piperidine); 2.20 (1H, m, H₄ piperidine); 2.71 (2H, m, H₂ and H₆ piperidine); 2.82 (4H, m, CH₂-CH₂-phenyle); 2.98 (1H, d, J= 13 Hz, H₂ piperidine); 3.13 (1H, d, J= 13 Hz, H₆ piperidine); 3.34 (1H, dd, J= 13 Hz and 9 Hz, CH₂ β naphtylalanine); 3.46 (1H, dd, J= 13 Hz and 6 Hz, CH₂ β naphtylalanine); 3.67 (1H, s, OCH₃); 5.07 (2H, s, CH₂-*p*-methoxyphenyle); 5.35 (1H, m, CH α naphtylalanine); 6.69 (2H, d Jₒ= 9 Hz, H₃ and H₅ *p*-methoxyphenyle); 6.75 (2H, d, Jₒ= 9 Hz, H₂ and H₆ *p*-methoxyphenyle); 7.11 (1H, d, Jₒ= 7 Hz, H₃ naphtyle); 7.12-7.26 (4H, m, H₆ and H₇ naphtyle, H₂ and H₆ phenyle); 7.34 (1H, d, Jₒ= 8 Hz, H₄ naphtyle); 7.47 (2H, m, H₃ and H₅ phenyle); 7.64 (1H, s, H₁ naphtyle); 7.77 (2H, d, Jₒ= 8 Hz, H₅ and H₈ naphtyle); 7.86 (1H, m, H₄ phenyle); 8.13 (1H, m, NH piperidine TFA salt); 8.50 (1H, m, NH piperidine TFA salt); 8.76 (1H, d, J= 8 Hz, NH amide).
¹³C NMR (75 MHz, DMSO d^{6.} 300°K): δ(ppm) 24.2 (C₃ piperidine); 25.1 (C₅ piperidine); 26.1 (CH₂-CH₂-phenyle); 32.2 (CH₂-CH₂-phenyle); 38.1 (C₄ piperidine); 42.1 (C₂ and C₆ piperidine, CH₂ β naphtylalanine); 44.7 (CH α naphtylalanine); 44.9 (CH₂-*p*-methoxyphenyle); 55.0 (OCH₃); 113.9 (C₃ and C₅ *p*-methoxyphenyle); 125.4 (C₆ naphtyle); 125.9 (C₇ naphtyle); 126.0 (C₁ naphtyle); 127.3 (C₄ phenyle, C₂ and C₆ *p*-methoxyphenyle); 127.4 (C₁ *p*-methoxyphenyle); 127.5 (C₄ naphtyle); 127.8 (C₅ and C₈ naphtyle); 127.9 (C₃ and C₅ phenyle); 128.2 (C₂ and C₆ phenyle); 131.7 (C₁₀ naphtyle); 132.8 (C₉ naphtyle); 135.3 (C₂ naphtyle); 140.4 (C₁ phenyle); 154.1 (Cquaterniare triazole); 154.5 (Cquaternary triazole); 158.6 (C₄ *p*-methoxyphenyle); 172.4 (CO amide).

**Table 1** depicts further 1,2,4-triazole derivatives as well as their ESI mass spectrometry data.

**Table 1: 1,2,4-Triazole Derivatives with Structure, Name and MS data**

| **No.** | **Structure** | **Chemical name** | **ESI-MS found (M+H)⁺** |
|---|---|---|---|
| **1** | 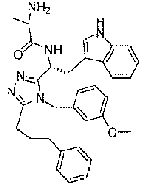 | N-((*R*)-1-(4-(3-methoxybenzyl)-5-(3-phenylpropyl)-4*H*-1,2,4-triazol-3-yl)-2-(1*H-*indol-3-yl)ethyl)-2-amino-2-methylpropanamide | 551.2 |
| **2** | 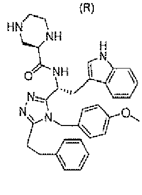 | (2*R*)-N-((*R*)-1-(4-(4-methoxybenzyl)-5-phenethyl-4*H*-1,2,4-triazol-3-yl)-2-(1*H-*indol-3-yl)ethyl)piperazine-2-carboxamide | 564.3 |
| **3** | 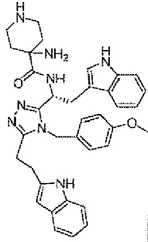 | N-((*R*)-1-(5-(2-(1*H*-indol-2-yl)ethyl)-4-(4-methoxybenzyl)-4H-1,2,4-triazol-3-yl)-2-(1*H*-indol-3-yl)ethyl)-4-aminopiperidine-4-carboxamide | 617.5 |
| **4** | 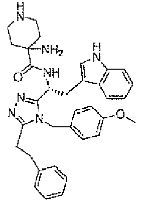 | N-((*R*)-1-(4-(4-methoxybenzyl)-5-phenethyl-4*H*-1,2,4-triazol-3-yl)-2-(1*H-*indol-3-yl)ethyl)-4-aminopiperidine-4-carboxamide | 578.6 |
| **5** | 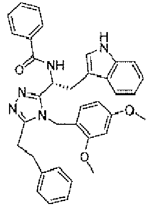 | N-((*R*)-1-(4-(2,4-dimethoxybenzyl)-5-phenethyl-4*H*-1,2,4-triazol-3-yl)-2-(1*H-*indol-3-yl)ethyl)benzamide | 586.5 |
| **6** | 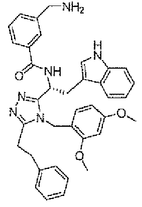 | N-((*R*)-1-(4-(2,4-dimethoxybenzyl)-5-phenethyl-4*H*-1,2,4-triazol-3-yl)-2-(1*H-*indol-3-yl)ethyl)-3-(aminomethyl)benzamide | 615.4 |
| **7** | 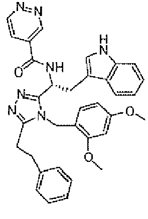 | N-((*R*)-1-(4-(2,4-dimethoxybenzyl)-5-phenethyl-4*H*-1,2,4-triazol-3-yl)-2-(1*H-*indol-3-yl)ethyl)pyridazine-4-carboxamide | 588.3 |
| **8** | 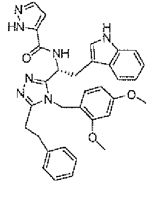 | N-((*R*)-1-(4-(2,4-dimethoxybenzyl)-5-phenethyl-4*H*-1,2,4-triazol-3-yl)-2-(1*H-*indol-3-yl)ethyl)-1*H*-pyrazole-5-carboxamide | 576.3 |
| **10** | 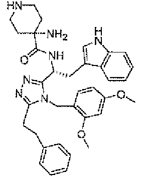 | N-((*R*)-1-(4-(2,4-dimethoxybenzyl)-5-phenethyl-4*H*-1,2,4-triazol-3-yl)-2-(1*H-*indol-3-yl)ethyl)-4-aminopiperidine-4-carboxamide | 608.3 |
| **11** | 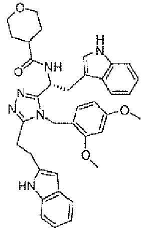 | N-((*R*)-1-(5-(2-(1*H*-indol-2-yl)ethyl)-4-(2,4-dimethoxybenzyl)-4*H*-1,2,4-triazol-3-yl)-2-(1*H*-indol-3-yl)ethyl)-tetrahydro-2*H*-pyran-4-carboxamide | 633.3 |
| **12** | 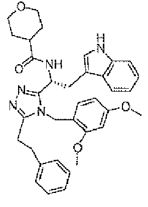 | N-((*R*)-1-(4-(2,4-dimethoxybenzyl)-5-phenethyl-4*H*-1,2,4-triazol-3-yl)-2-(1*H-*indol-3-yl)ethyl)-tetrahydro-2H-pyran-4-carboxamide | 594.2 |
| **13** | 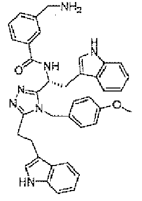 | N-((*R*)-1-(5-(2-(1*H*-indol-3-yl)ethyl)-4-(4-methoxybenzyl)-4H-1,2,4-triazol-3-yl)-2-(1*H*-indol-3-yl)ethyl)-3-(aminomethyl)benzamide | 624.3 |
| **14** | 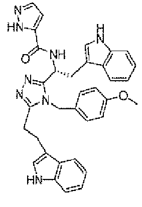 | N-((*R*)-1-(5-(2-(1*H*-indol-3-yl)ethyl)-4-(4-methoxybenzyl)-4*H*-1,2,4-triazol-3-yl)-2-(1*H*-indol-3-yl)ethyl)-1*H*-pyrazole-5-carboxamide | 585.1 |
| **15** | 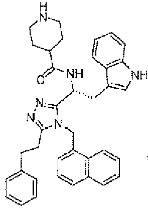 | N-((*R*)-2-(1*H*-indol-3-yl)-1-(4-((naphthalen-1-yl)methyl)-5-phenethyl-4*H*-1,2,4-triazol-3-yl)ethyl)piperidine-4-carboxamide | 583.2 |
| **16** | 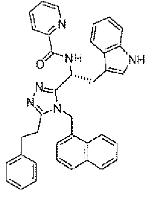 | N-((*R*)-2-(1*H*-indol-3-yl)-1-(4-((naphthalen-1-yl)methyl)-5-phenethyl-4*H*-1,2,4-triazol-3-yl)ethyl)picolinamide | 577.0 |
| **17** | 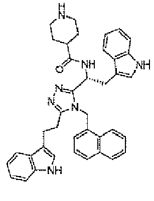 | N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)piperidine-4-carboxamide | 622.0 |
| **18** | 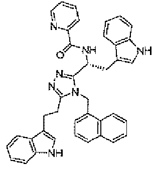 | N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)picolinamide | 616.0 |
| **19** | 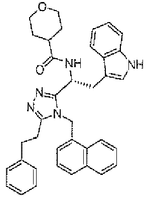 | N-((*R*)-2-(1*H*-indol-3-yl)-1-(4-((naphthalen-1-yl)methyl)-5-phenethyl-4*H*-1,2,4-triazol-3-yl)ethyl)-tetrahydro-2*H*-pyran-4-carboxamide | 584.1 |
| **20** | 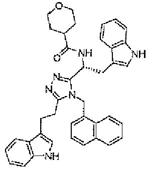 | N-((*R*)-1-(5-(2-(*H*-indol-3-yl)ethyl)-4-((naphthalen-1-yl)methyl)-4*H*-1,2,4-triazol- 3-yl)-2-(1*H-*indol-3-yl)ethyl)-tetrahydro-2*H-*pyran-4-carboxamide | 623.1 |
| **21** | 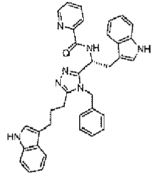 | N-((R)-1-(5-(3-(1*H*-indol-3-yl)propyl)-4-benzyl-4*H*-1,2,4-triazol-3-yl)-2-(1*H*-indol-3-yl)ethyl)picolinamide | 580.1 |
| **22** | 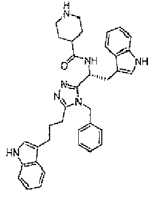 | N-((*R*)-1-(5-(3-(1*H*-indol-3-yl)propyl)-4-benzyl-4*H*-1,2,4-triazol-3-yl)-2-(1*H*-indol-3-yl)ethyl)piperidine-4-carboxamide | 586.1 |
| **23** | 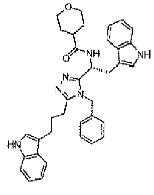 | N-((*R*)-1-(5-(3-(1*H*-indol-3-yl)propyl)-4-benzyl-4*H*-1,2,4-triazol-3-yl)-2-(1*H*-indol-3-yl)ethyl)-tetrahydro-2*H*-pyran-4-carboxamide | 587.0 |
| **29** | 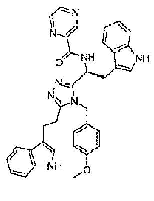 | N-((*S*)-1-(5-(2-(1*H*-indol-3-yl)ethyl)-4-(4-methoxybenzyl)-4*H*-1,2,4-triazol-3-yl)-2-(1*H*-indol-3-yl)ethyl)pyrazine-2-carboxamide | 597.2 |
| **30** | 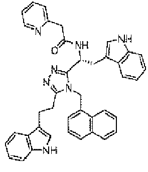 | N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)-2-(pyridin-2-yl)acetamide | 630.0 |
| **31** | 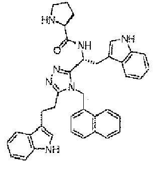 | (2*S*)-N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)pyrrolidine-2-carboxamide | 608.1 |
| **32** | 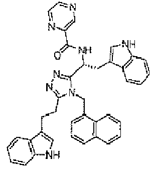 | N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)pyrazine-2-carboxamide | 617.1 |
| **33** | 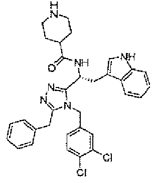 | N-((*R*)-1-(4-(3,4-dichlorobenzyl)-5-benzyl-4*H*-1,2,4-triazol-3-yl)-2-(1*H*-indol-3-yl)ethyl)piperidine-4-carboxamide | 587.0 |
| **34** | 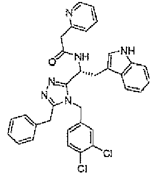 | N-((*R*)-1-(4-(3,4-dichlorobenzyl)-5-benzyl-4*H*-1,2,4-triazol-3-yl)-2-(1*H*-indol-3-yl)ethyl)-2-(pyridin-2-yl)acetamide | 595.0 |
| **35** | 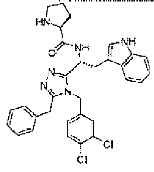 | (25)-N-((*R*)-1-(4-(3,4-dichlorobenzyl)-5-benzyl-4*H*-1,2,4-triazol-3-yl)-2-(1*H*-indol-3-yl)ethyl)pyrrolidine-2-carboxarmide | 573.0 |
| **36** | 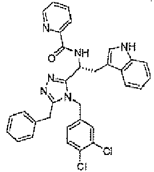 | N-((*R*)-1-(4-(3,4-dichlorobenzyl)-5-benzyl-4*H*-1,2,4-triazol-3-yl)-2-(1*H*-indol-3-yl)ethyl)picolinamide | 581.0 |
| **37** | 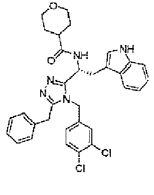 | N-((*R*)-1-(4-(3,4-dichlorobenzyl)-5-benzyl-4*H*-1,2,4-triazol-3-yl)-2-(1*H*-indol-3-yl)ethyl)-tetrahydro-2*H*-pyran-4-carboxamide | 588.2 |
| **38** | 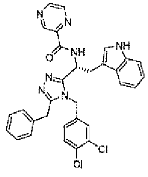 | N-((*R*)-1-(4-(3,4-dichlorobenzyl)-5-benzyl-4*H*-1,2,4-triazol-3-yl)-2-(1*H*-indol-3-yl)ethyl)pyrazine-2-carboxamide | 581.8 |
| **39** | 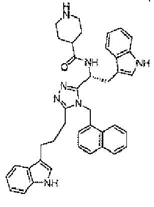 | N-((*R*)-1-(5-(3-(1*H*-indol-3-yl)propyl)-4-((naphthalen-1-yl)methyl)-4H-1,2,4-triazol-3-yl)-2-(1*H*-indol-3-yl)ethyl)piperidine-4-carboxamide | 636.0 |
| **40** | 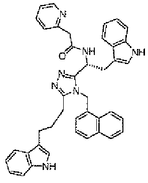 | N-((*R*)-1-(5-(3-(1*H*-indol-3-yl)propyl)-4-((naphthalen-1-yl)methyl)-4*H*-1,2,4-triazol-3-yl)-2-(1*H*-indol-3-yl)ethyl)-2-(pyridin-2-yl)acetamide | 644.0 |
| **41** | 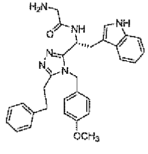 | N-((*R*)-1-(4-(4-methoxybenzyl)-5-phenethyl-4*H*-1,2,4-triazol-3-yl)-2-(1*H-*indol-3-yl)ethyl)-2-aminoacetamide | 509.2 |
| **42** | 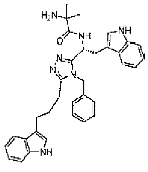 | N-((*R*)-1-(5-(3-(1*H*-indol-3-yl)propyl)-4-benzyl-4*H*-1,2,4-triazol-3-yl)-2-(1*H*-indol-3-yl)ethyl)-2-amino-2-methylpropanamide | 560.3 |
| **43** | 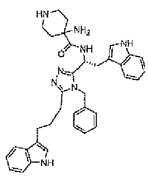 | N-((*R*)-1-(5-(3-(1*H*-indol-3-yl)propyl)-4-benzyl-4*H*-1,2,4-triazol-3-yl)-2-(1*H*-indol-3-yl)ethyl)-4-aminopiperidine-4-carboxamide | 601.2 |
| **44** | 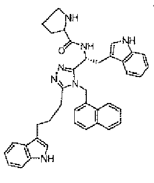 | N-((*R*)-1-(5-(3-(1*H*-indol-3-yl)propyl)-4-((naphthalen-1-yl)methyl)-4*H*-1,2,4-triazol-3-yl)-2-(1*H*-indol-3-yl)ethyl)pyrrolidine-2-carboxamide | 622.1 |
| **45** | 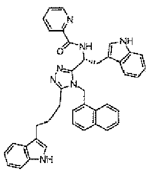 | N-((*R*)-1-(5-(3-(1*H*-indol-3-yl)propyl)-4-((naphthalen-1-yl)methyl)-4*H*-1,2,4-thazol-3-yl)-2-(1*H*-indol-3-yl)ethyl)picolinamide | 630.1 |
| **46** | 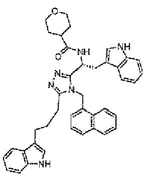 | N-((*R*)-1-(5-(3-(1*H*-indol-3-yl)propyl)-4-((naphthalen-1-yl)methyl)-4*H*-1,2,4-triazol-3-yl)-2-(1*H*-indol-3-yl)ethyl)-tetrahydro-2H-pyran-4-carboxamide | 637.1 |
| **47** | 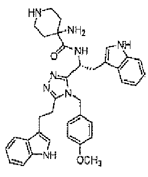 | N-((*R*)-1-(5-(2-(1*H*-indol-3-yl)ethyl)-4-(4-methoxybenzyl)-4*H*-1,2,4-triazol-3-yl)-2-(1*H*-indol-3-yl)ethyl)-4-aminopiperidine-4-carboxamide | 617.2 |
| **48** | 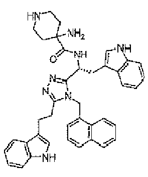 | N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)-4- aminopiperidine-4-carboxamide | 637.3 |
| **49** | 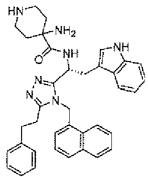 | N-((*R*)-2-(1*H*-indol-3-yl)-1-(4-((naphthalen-1-yl)methyl)-5-phenethyl-4*H*-1,2,4-triazol-3-yl)ethyl)-4-aminopiperidine-4-carboxamide | 598.3 |
| **50** | 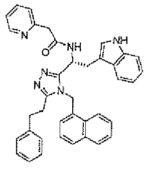 | N-((*R*)-2-(1*H*-indol-3-yl)-1-(4-((naphthalen-1-yl)methyl)-5-phenethyl-4*H*-1,2,4-triazol-3-yl)ethyl)-2-(pyridin-2-yl)acetamide | 591.0 |
| **51** | 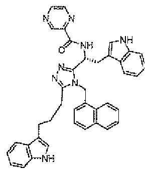 | N-((*R*)-1-(5-(3-(1*H*-indol-3-yl)propyl)-4-((naphthalen-1-yl)methyl)-4*H*-1,2,4-triazol-3-yl)-2-(1*H*-indol-3-yl)ethyl)pyrazine-2-carboxamide | 631.0 |
| **52** | 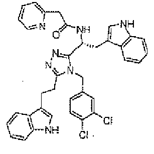 | N-((*R*)-1-(5-(2-(1*H*-indol-3-yl)ethyl)-4-(3,4-dichlorobenzyl)-4H-1,2,4-triazol-3-yl)-2-(1*H*-indol-3-yl)ethyl)-2-(pyridin-2-yl)acetamide | 648.1 |
| **53** | 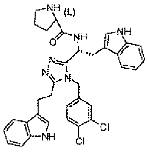 | N-((*R*)-1-(5-(2-(1H-indol-3-yl)ethyl)-4-(3,4-dichlorobenzyl)-4*H*-1,2,4-triazol-3-yl)-2-(1*H*-indol-3-yl)ethyl)pyrrolidine-2-carboxamide | 626.2 |
| **54** | 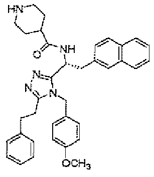 | N-((*R*)-1-(4-(4-methoxybenzyl)-5-phenethyl-4*H*-1,2,4-triazol-3-yl)-2-(naphthalen-2-yl)ethyl)piperidine-4-carboxamide | 574.1 |
| **55** | 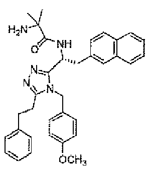 | N-((*R*)-1-(4-(4-methoxybenzyl)-5-phenethyl-4*H*-1,2,4-triazol-3-yl)-2-(naphthalen-2-yl)ethyl)-2-amino-2-methylpropanamide | 548.2 |
| **56** | 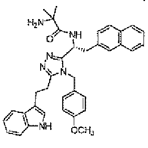 | N-((*R*)-1-(5-(2-(1*H*-indol-3-yl)ethyl)-4-(4-methoxybenzyl)-4*H*-1,2,4-triazol-3-yl)-2-(naphthalen-2-yl)ethyl)-2-amino-2-methylpropanamide | 587.1 |
| **57** | 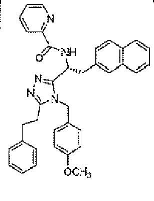 | N-((*R*)-1-(4-(4-methoxybenzyl)-5-phenethyl-4*H*-1,2,4-triazol-3-yl)-2-(naphthaien-2-yl)ethyl)picolinamide | 568.1 |
| **58** | 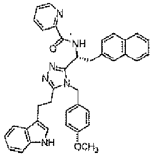 | N-((*R*)-1-(5-(2-(1*H*-indol-3-yl)ethyl)-4-(4-methoxybenzyl)-4*H*-1,2,4-triazol-3-yl)-2-(naphthalen-2-yl)ethyl)picolinamide | 607.1 |
| **59** | 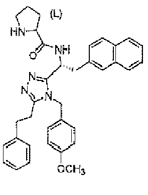 | N-((*R*)-1-(4-(4-methoxybenzyl)-5-phenethyl-4*H*-1,2,4-triazol-3-yl)-2-(naphthalen-2-yl)ethyl)pyrrolidine-2-carboxamide | 560.3 |
| **60** | 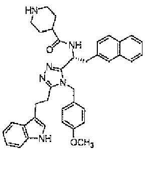 | N-((*R*)-1-(5-(2-(1*H*-indol-3-yl)ethyl)-4-(4-methoxybenzyl)-4*H*-1,2,4-triazol-3-yl)-2-(naphthalen-2-yl)ethyl)piperidine-4-carboxamide | 613.4 |
| **61** | 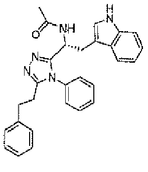 | N-((*R*)-2-(1*H*-indol-3-yl)-1-(5-phenethyl-4-phenyl-4*H*-1,2,4-triazol-3-yl)ethyl)acetamide | 450.0 |
| **62** | 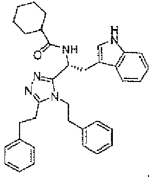 | N-((*R*)-2-(1*H*-indol-3-yl)-1-(4,5-diphenethyl-4*H*-1,2,4-triazol-3-yl)ethyl)cyclohexanecarboxamide | 546.2 |
| **63** | 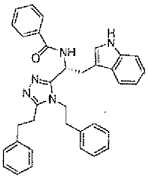 | N-((*R*)-2-(1*H*-indol-3-yl)-1-(4,5-diphenethyl-4*H*-1,2,4-triazol-3-yl)ethyl)benzamide | 540.1 |
| **64** | 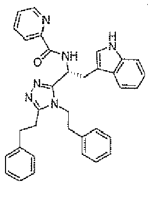 | N-((*R*)-2-(1*H*-indol-3-yl)-1-(4,5-diphenethyl-4*H*-1,2,4-triazol-3-yl)ethyl)picolinamide | 541.1 |
| **65** | 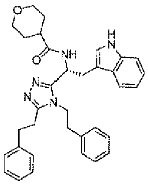 | N-((*R*)-2-(1*H*-indol-3-yl)-1-(4,5-diphenethyl-4*H*-1,2,4-triazol-3-yl)ethyl)-tetrahydro-2*H*-pyran-4-carboxamide | 548.3 |
| **66** | 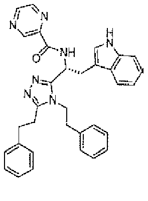 | N-((*R*)-2-(1*H*-indol-3-yl)-1-(4,5-diphenethyl-4*H*-1,2,4-triazol-3-yl)ethyl)pyrazine-2-carboxamide | 542.3 |
| **67** | 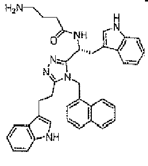 | N-((*R*)-1-(5-(2-(1*H*-indol-3-yl)ethyl)-4-((naphthalen-1-yl)methyl)-4*H*-1,2,4-triazol-3-yl)-2-(1*H*-indol-3-yl)ethyl)-4-aminobutanamide | 596.1 |
| **68** | 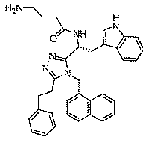 | N-((*R*)-2-(1*H*-indol-3-yl)-1-(4-((naphthalen-1-yl)methyl)-5-phenethyl-4*H*-1,2,4-triazol-3-yl)ethyl)-4-aminobutanamide | 557.2 |
| **69** | 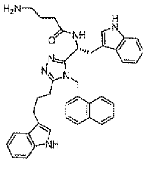 | N-((*R*)-1-(5-(3-(1*H*-indol-3-yl)propyl)-4-((naphthalen-1-yl)methyl)-4*H*-1,2,4-triazol-3-yl)-2-(1*H*-indol-3-yl)ethyl)-4-aminobutanamide | 610.4 |
| **70** | 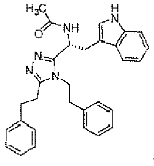 | N-((*R*)-2-(1*H*-indol-3-yl)-1-(4,5-diphenethyl-4*H*-1,2,4-triazol-3-yl)ethyl)acetamide | 478.2 |
| **71** | 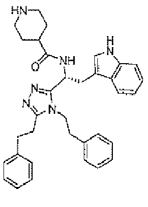 | N-((*R*)-2-(1*H*-indol-3-yl)-1-(4,5-diphenethyl-4*H*-1,2,4-triazol-3-yl)ethyl)piperidine-4-carboxamide | 547.2 |
| **72** | 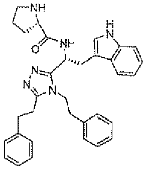 | (2*S*)-N-((*R*)-2-(1*H*-indol-3-yl)-1-(4,5- diphenethyl-4*H*-1,2,4-triazol-3-yl)ethyl)pyrrolidine-2-carboxamide | 533.2 |
| **73** | 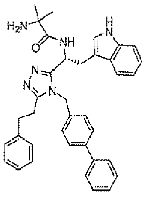 | 2-amino-N-((*R*)-1-(4-(4-phenylbenzyl)-5-phenethyl-4*H*-1,2,4-triazol-3-yl)-2-(1*H-*indol-3-yl)ethyl)-2-methylpropanamide | 583.2 |
| **74** | 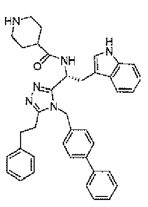 | N-((*R*)-1-(4-(4-phenylbenzyl)-5-phenethyl-4*H*-1,2,4-triazol-3-yl)-2-(1*H*-indol-3-yl)ethyl)piperidine-4-carboxamide | 609.0 |
| **75** | 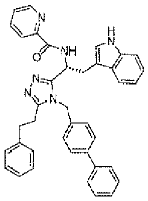 | N-((*R*)-1-(4-(4-phenylbenzyl)-5-phenethyl-4*H*-1,2,4-triazol-3-yl)-2-(1*H*-indol-3-yl)ethyl)picolinamide | 603.0 |
| **76** | 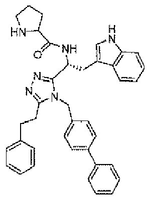 | N-((*R*)-1-(4-(4-phenylbenzyl)-5-phenethyl-4*H*-1,2,4-triazol-3-yl)-2-(1*H*-indol-3-yl)ethyl)pyrrolidine-2-carboxamide | 595.2 |
| **77** | 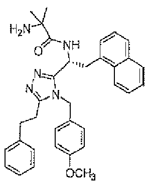 | N-((*R*)-1-(4-(4-methoxybenzyl)-5-phenethyl-4*H*-1,2,4,-triazol-3-yl)-2-(naphthalen-1-yl)ethyl)-2-amino-2-methylpropanamide | 547.9 |
| **78** | 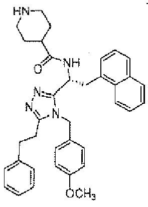 | N-((*R*)-1-(4-(4-methoxybenzyl)-5-phenethyl-4*H*-1,2,4-triazol-3-yl)-2-(naphthalen-1-yl)ethyl)piperidine-4- carboxamide | 573.9 |
| **19** | 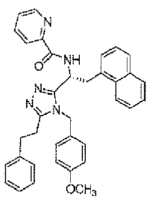 | N-((*R*)-1-(4-(4-methoxybenzyl)-5-phenethyl-4*H*-1,2,4-triazol-3-yl)-2-(naphthalen-1-yl)ethyl)picolinamide | 567.9 |
| **80** | 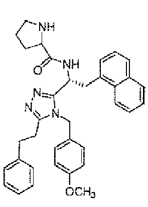 | N-((*R*)-1-(4-(4-methoxybenzyl)-5-phenethyl-4*H*-1,2,4-triazol-3-yl)-2-(naphthalen-1-yl)ethyl)pyrrolidine-2-carboxamide | 560.2 |
| **81** | 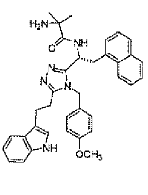 | N-((*R*)-1-(5-(2-(1*H*-indol-3-yl)ethyl)-4-(4-methoxybenzyl)-4*H*-1,2,4-triazol-3-yl)-2-(naphthalen-1-yl)ethyl)-2-amino-2-methylpropanamide | 587.0 |
| **82** | 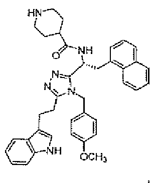 | N-((*R*)-1-(5-(2-(1*H*-indol-3-yl)ethyl)-4-(4-methoxybenzyl)-4*H*-1,2,4-triazol-3-yl)-2-(naphthalen-1-yl)ethyl)piperidine-4-carboxamide | 613.3 |
| **83** | 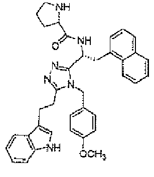 | N-((*R*)-1-(5-(2-(1*H*-indol-3-yl)ethyl)-4-(4-methoxybenzyl)-4*H*-1,2,4-triazol-3-yl)-2-(naphthalen-1-yl)ethyl)pyrrolidine-2-carboxamide | 599.3 |
| **84** | 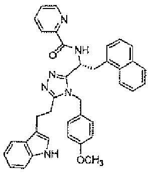 | N-((*R*)-1-(5-(2-(1*H*-indol-3-yl)ethyl)-4-(4-methoxybenzyl)-4*H*-1,2,4-triazol-3-yl)-2-(naphthalen-1-yl)ethyl)picolinamide | 607.3 |
| **85** | 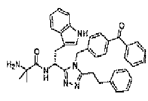 | 2-amino-N-((*R*)-1-(4-(4-benzoylbenzyl)-5-phenethyl-4*H*-1,2,4-triazol-3-yl)-2-(1*H-*indol-3-yl)ethyl)-2-methylpropanamide | 611.3 |
| **86** | 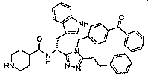 | N-((*R*)-1-(4-(4-benzoylbenzy)I-5-phenethyl-4*H*-1,2,4-triazol-3-yl)-2-(1*H*- indol-3-yl)ethyl)piperidine-4-carboxamide | 637.4 |
| **87** | 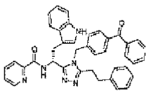 | N-((*R*)-1-(4-(4-benzoylbenzy)I-5-phenethyl-4*H*-1,2,4-triazol-3-yl)-2-(1*H-*indol-3-yl)ethyl)picolinamide | 631.3 |
| **88** | 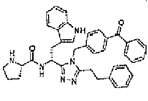 | (2*S*)-N-((*R*)-1-(4-(4-benzoylbenzy)I-5-phenethyl-4*H*-1,2,4-triazol-3-yl)-2-(1*H-*indol-3-yl)ethyl)pyrrolidine-2-carboxamide | 623.3 |

### II) GHS-R 1a Receptor-ligand binding assay (membrane preparations from transfected LLC PK-1 cells)

The GHS-R 1 a receptor binding/affinity studies were performed according to Guerlavais et al. (J. Med. Chem. 2003, 46: 1191-1203).

Isolated plasma membranes from LLC PK-1 cells, a renal epithelial cell line originally derived from porcine kidneys (ECACC No. 86121112) (10 µg of protein), that were transiently transfected with human GHS-R 1 a cDNA (Guerlavais et al., J. Med. Chem. 2003, 46: 1191-1203), were incubated in homogenization buffer HB [50 mM Tris (pH 7,3), 5 mM MgCl₂, 2,5 mM EDTA, and 30 µg/mL bacitracin (Sigma)] for 60 min at 25° C (steady-state conditions) with 60 pM ¹²⁵I-His⁹-ghrelin (Amersham) in the presence or absence of competing compounds (compounds of the invention).

The binding affinity for each compound to be tested for the human GHS-R 1a was measured by displacement of the radiolabelled ghrelin with increasing concentrations of the test compound (10⁻¹¹M to 10⁻²M) (each experiment being performed in triplicates).

Nonspecific binding was defined using an excess (10⁻⁶ M) of ghrelin. The binding reaction was stopped by addition of 4 mL of ice-cold HB followed by rapid filtration over Whatman GP/C filters presoaked with 0.5% polyethyleneimine to prevent excessive binding of radioligand to the filters. Filters were rinsed three times with 3 mL of ice-cold wash buffer [50 mM Tris (pH 7.3), 10 mM MgCl₂, 2.5 mM EDTA, and 0.015% (w/v) X-100 Triton], and the radioactivity bound to membranes was measured in a gamma-counter (Kontron Analytical Gamma Matic, Automatic gamma counting system).

The concentration of test compounds required to inhibit radiolabelled ghrelin binding by 50% (IC₅₀) was determined by fitting competitive binding curves using non-linear regression (PRISM 3.0, Graph Pad San Diego, USA).

In the following **table 2** results obtained for selected compounds of the invention are presented in comparison to an example of the prior art. IC₆₀ values given are the mean of at least two independent experiments performed in triplicates.

**Table 2: GHS-R 1a Receptor-ligand binding assay test results (IC₅₀ values for a number of selected exemplary compounds)**

| **No.** | **GHS-R 1a IC₅₀ [nM]** |
|---|---|
| **1** | 380 |
| **2** | 143 |
| **3** | 16 |
| **4** | 6,6 |
| **5** | 11,8 |
| **6** | 204 |
| **8** | 350 |
| **10** | 1,3 |
| **11** | 12 |
| **12** | 15,8 |
| **13** | 11,1 |
| **14** | 89,8 |
| **15** | 17 |
| **16** | 125 |
| **17** | 4 |
| **18** | 5,3 |
| **19** | 390 |
| **20** | 180 |
| **21** | 80 |
| **22** | 40 |
| **23** | 370 |
| **30** | 125 |
| **31** | 505 |
| **32** | 122 |
| **33** | 5 |
| **34** | 110 |
| **36** | 70 |
| **37** | 200 |
| **38** | 430 |
| **39** | 21,1 |
| **40** | 20,9 |
| **42** | 500 |
| **43** | 35 |
| **44** | 600 |
| **45** | 29 |
| **46** | 143 |
| **47** | 150 |
| **48** | 12 |
| **49** | 44,5 |
| **50** | 107 |
| **51** | 107 |
| **52** | 48 |
| **53** | 158 |
| **54** | 0,3 |
| **55** | 13,7 |
| **57** | 0,7 |
| **58** | 1,0 |
| **59** | 27,8 |
| **62** | 707 |
| **63** | 535 |
| **64** | 198 |
| **66** | 825 |
| **67** | 0,27 |
| **68** | 182 |
| **69** | 126 |
| **71** | 92 |
| **73** | 150 |
| **74** | 95 |
| **77** | 12,3 |
| **78** | 2,2 |
| **79** | 1,45 |
| **80** | 18,8 |

### III) In vitro intracellular Calcium release assay using human GHS-R 1a transfected CHO cells

The potential of the compounds of the invention to modulate GHS receptor activity was assessed by an *in vitro* intracellular Calcium release assay employing CHO cells that were transfected with human GHS-R 1a.

Release of intracellular calcium or inhibition thereof was measured using the fluorescent calcium indicator assay (FLIPR) and Fluo-4 AM.

CHO cells (CHO-KI Chinese Hamster Ovary cell line, ATCC No. CCL-61) were transiently transfected with human GHS-R 1 a cDNA by electroporation and plated into 96-well black bottom plates (Corning 3603) (80,000 cells/well). Transient transfections were performed using the Easyject Optima Electroporator (Equibio), according to the manufacturer's instructions.

Transfected cells were grown in Dulbecco's modified Eagle's medium without phenol red, supplemented with 10% (v/v) non-essential amino acids, 2 nM glutamine and streptomycin-penicillin (250 µg/ml-250 u/ml) (all purchased from Cambrex) at 37°C, 5% CO₂ in a humidified atmosphere for 24 hours.

After incubation, transfected cells were washed with 150µl Buffer A [Hanks' balanced salt solution (Sigma H-6648), 0.5% (v/v) BSA (Sigma A-7906), 20mM CaCl₂, 2.5mM probenecid (pH 7.4, dissolved in 1M NaOH) (Sigma P-8761)] and were then loaded with fluorescent calcium indicator Fluo-4 AM (10⁻⁶ M) (Interchim UP72972) prepared in Buffer A, additionally containing 0.06% pluronic acid (Molecular probes P-6867) (a mild-ionic detergent which facilitates Fluo-4AM ester loading).(Loading: 100µl per well of Buffer A containing 120µl/ml Pluronic Acid and 1 µM Fluo-4AM was added to the cells).

After loading with Fluo-4 AM, transfected cells were incubated for 1 hour in the dark at 37° C.

Compounds to be tested were dissolved in Buffer A in triplicates at a concentration of 10⁻⁶ M and distributed into another 96-well plate (Fisher Labosi A1210500).

Following incubation, excess Fluo-4AM was removed, 100µl of Buffer A was added to each well at room temperature and immediately removed by aspiration. This was then repeated, before adding 50µl Buffer A to each well.

Transfected cells were further incubated room temperature for 30 min to allow complete de-esterification of intracellular Fluo-4AM esters.

Subsequently, both plates, the black-bottom plate containing transfected cells and the microtiter plate containing the compounds to be tested, were then placed into a temperature-regulated (25°C) FlexStation machine (benchtop scanning fluorometer Flex Station II, Molecular Devices, Sunnyvale, California, USA) for fluorescence output measurements.

Since Fluo-4AM exhibits a large fluorescence intensity increase upon binding of calcium, fluorescence output can be used directly as a proportional measure of intracellular calcium release.

Basal fluorescence output from the transfected cells was measured for 15 sec and then 50µl of the compounds to be tested were automatically distributed into the wells containing the transfected cells. The fluorescence output was then recorded for a further 45 sec.

Excitation and emission wavelengths were 485 nm and 525 nm, respectively. Basal fluorescence intensity of Fluo-4AM-loaded transfected cells without compounds to be tested varied between 800-1200 arbitrary units, whereas maximal fluorescence output of dye-loaded transfected cells upon incubation with the compounds to be tested varied between 5000-7000 arbitrary units and was equivalent to that achieved by stimulation of dye-loaded transfected cells with 10⁻⁶ M ghrelin.

For each compound to be tested change in fluorescence output upon addition of the respective compound was compared with the basal fluorescence output measured with a negative control, i.e. addition 50 µl of buffer A to transfected cells only.

The ability and extent to which each compound to be tested caused calcium release was determined relative to the basal level (0%) and the maximum level (100%) achieved with 1 µM ghrelin.

For the compounds to be tested that were identified as GHS receptor agonists, EC₅₀ and KI values were determined using a dose-response curve.

As for the compounds to be tested that were identified as GHS receptor antagonists, IC₅₀ and Kb (antagonist dissociation constant) values were determined using antagonist inhibition curves in the presence of 10⁻⁷ M ghrelin (submaximal concentration). IC₅₀ values were calculated as the molar concentration of GHS receptor antagonist that reduced the maximal response of ghrelin by 50%. Kb values were estimated using the Cheng-Prusoff Equation (Lazareno S and Birdsall NJ, Trends Pharmacol Sci. 1993, 14(6):237-239).

**Table 3** shows individual Kb values for antagonistic compounds and %activation and EC₅₀ values for agonistic compounds.

**Table 3: Kb values, %activation and EC₅₀ values for a number of selected exemplary compounds**

| **No.** | **Kb value [nM]** | **%activation / EC₅₀ values [nM]** |
|---|---|---|
| **1** | | 47% |
| **2** | | 40% / |
| | | 17 |
| **3** | | 106% / |
| | | 4,4 |
| **4** | | 90% / |
| | | 4,1 |
| **5** | | 31% / |
| | | 16 |
| **6** | | 4% |
| **7** | 57 | 3% |
| **8** | 64 | 0% |
| **10** | | 100% / |
| | | 2,0 |
| **11** | 40 | 0% |
| **12** | 68 | 3% |
| **13** | | 70% / |
| | | 2,5 |
| **14** | 12,8 | 15% |
| **15** | | 120% / |
| | | 2,0 |
| **16** | 79 | |
| **17** | | 94% / |
| | | 2,4 |
| **18** | | 1% |
| **19** | 275 | 0% |
| **20** | | 80% / |
| | | 0,6 |
| **21** | 300 | 0% |
| **22** | | 150% / |
| | | 1,6 |
| **23** | 50 | 15% |
| **29** | 215 | 0% |
| **30** | | 0% |
| **31** | | 100% / |
| | | 2,7 |
| **32** | | 0% |
| **33** | | 120% / |
| | | 1,5 |
| **34** | 340 | 0% |
| **35** | | 60% / |
| | | 17 |
| **36** | 215 | 20% |
| **37** | 215 | 0% |
| **39** | | / |
| | | 1,0 |
| **42** | | 100% / |
| | | 34 |
| **43** | J MV 3496 | / |
| | | 6,4 |
| **44** | JMV 3511 | 1 00% / |
| | | 12 |
| **47** | | 100%/ |
| | | 38 |
| **48** | | 100%/ |
| | | 3,9 |
| **49** | | 100% / |
| | | 10,1 |

## Claims

**1.** Process of manufacturing 1,2,4-triazole derivatives comprising the following steps:
(f) reacting a compound of formula (I) with a compound of formula (II) in a solvent in the presence of a coupling reagent and a base at a temperature Tₐ to yield a compound of formula (III) ("peptide coupling or acylation") 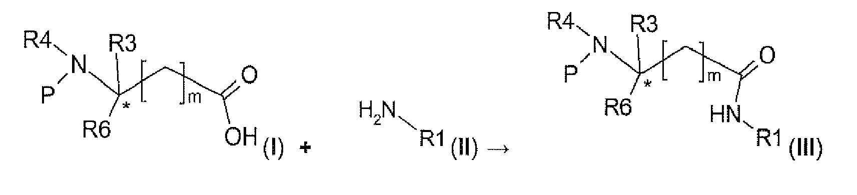
(g) reacting a compound of formula (III) in a solvent with a thionating reagent at a temperature T_{b} to yield a compound of formula (IV) ("thionation") 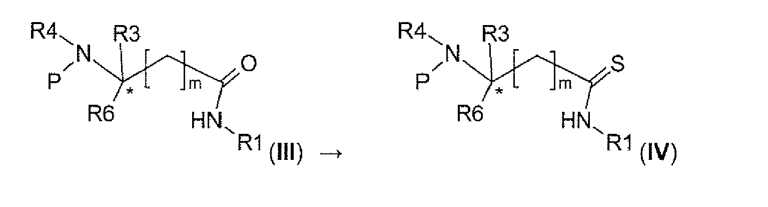
(h) optionally, reacting a compound of formula (V) with hydrazine in a solvent at a temperature T_{c} to yield a compound of formula (VI) ("hydrazinolysis") 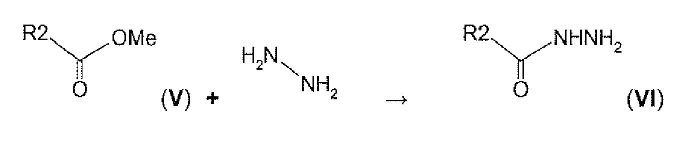
(i) reacting a compound of formula (IV) with a compound of formula (VI) in a solvent in the presence of a silver-compound and an acid at a temperature T_{d} to yield a compound of formula (VII) ("cyclization") 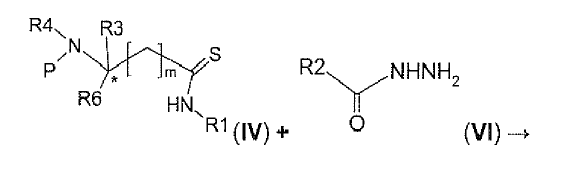 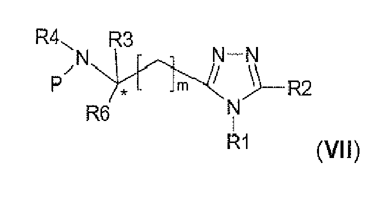
(j) reacting a compound of formula (VII) in a solvent in the presence of an acid at a temperature Tₑ to yield a compound of formula (VIII) ("deprotection") 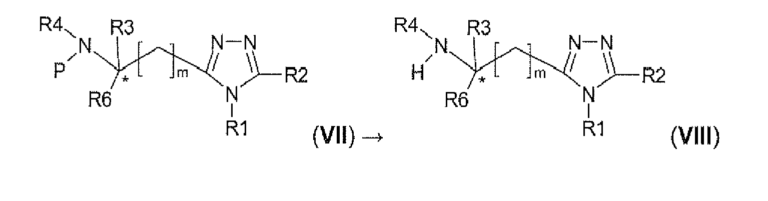
wherein:
R1, R2 are independently of one another selected from the group consisting of "hydrogen atom, alkyl, alkenyl, alkynyl, cycloalkyl, cycloalkylalkyl, aryl, heteroaryl, arylalkyl, heteroarylalkyl, heterocyclyl, heterocyclylalkyl, alkylsulfonyl, arylsulfonyl, arylalkylsulfonyl" which are optionally substituted in the alkyl, cycloalkyl, cycloalkylalkyl, aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, - O-alkyl, -O-aryl, -O-arylalkyl"; and preferably are selected from the group consisting of "alkyl, aryl, heteroaryl, arylalkyl, heteroarylalkyl" optionally being substituted by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
one of radicals R3, R4 is a hydrogen atom, whereas the other radical is selected from the group consisting of "hydrogen atom, alkyl, aryl, heteroaryl, arylalkyl, heteroarylalkyl, heterocyclyl, heterocyclylalkyl, -alkyl-O-aryl, -alkyl-O-arylalkyl, - alkyl-O-heteroaryl, -alkyl-O-heteroarylalkyl, -alkyl-O-heterocyclyl, alkyl-O-heterocyclylalkyl, -alkyl-CO-aryl, -alkyl-CO-arylalkyl, -alkyl-CO-heteroaryl, - alkyl-CO-heteroarylalkyl, -alkyl-CO-heterocyclyl, -alkyl-CO-heterocyclylalkyl, - alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, -alkyl-NH₂, -alkyl-NH-C(NH)-NH₂, alkylsulfonyl, arylsulfonyl, arylalkylsulfonyl, alkyl-S-alkyl, alkyl-S-H" which are optionally substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, - NO₂, alkyl, aryl, arylalkyl, -O-alkyl, -O-aryl, -O-arylalkyl"; and preferably are selected from the group consisting of "arylalkyl, heteroarylalkyl, heterocyclylalkyl, - alkyl-O-aryl, -alkyl-O-arylalkyl, -alkyl-O-heteroaryl, -alkyl-O-heteroarylalkyl, - alkyl-O-heterocyclyl, alkyl-O-heterocyclylalkyl, -alkyl-CO-aryl, -alkyl-CO-arylalkyl, -alkyl-CO-heteroaryl, -alkyl-CO-heteroarylalkyl, -alkyl-CO-heterocyclyl, alkyl-CO-heterocyclylalkyl, -alkyl-C(O)O-aryl, -alkyl-C(O)O-arylalkyl, -alkyl-C(O)O-heteroaryl, -alkyl-C(O)O-heteroarylalkyl, -alkyl-C(O)O-heterocyclyl, -alkyl-C(O)O-heterocyclylalkyl, -alkyl-CO-NH₂, -alkyl-CO-OH, - alkyl-NH₂, -alkyl-NH-C(NH)-NH₂," optionally being substituted in the aryl, heteroaryl, arylalkyl, heteroarylalkyl, heterocyclyl and/or heterocyclylalkyl group by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, -O-arylalkyl";
R6 is selected from the group consisting of "hydrogen atom, alkyl, cycloalkyl, cycloalkylalkyl" and preferably is a hydrogen atom;
R7, R8 are independently of one another selected from the group consisting of "hydrogen atom, alkyl, cycloalkyl, cycloalkylalkyl" and preferably are a hydrogen atom;
P is a protection group and preferably is selected from the group consisting of:
"Boc, Fmoc, Z, CBZ, Aloc, trityl, acetyl, benzyl"
m is 0, 1 or 2 and preferably is 0; and
* means a carbon atom of R or S configuration when chiral;

**2.** Process of manufacturing 1,2,4-triazole derivatives comprising the following step:
(d) reacting a compound of formula (IV) with a compound of formula (VI) in a solvent in the presence of a silver-compound and an acid at a temperature T_{d} to yield a compound of formula (VII) ("cyclization") 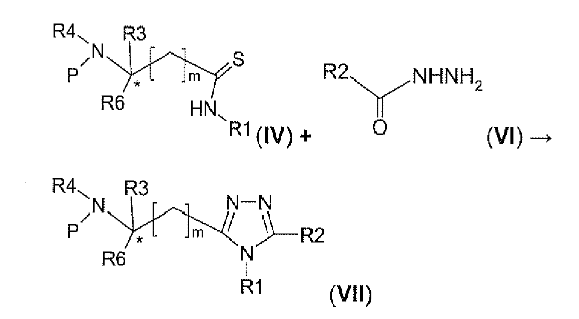 wherein:
R1, R2, R3, R4, R6, R7, R8, P, m have the meanings according to claim 1.

**3.** The process as claimed in claim 1, wherein following step (e)
(h) a compound of formula (VIII) is reacted in a solvent in the presence of a coupling reagent and a base or a reducing reagent or no reagent with a compound of formula (IX) at a temperature T_{f} to yield a compound of formula (X) ("R5 introduction") 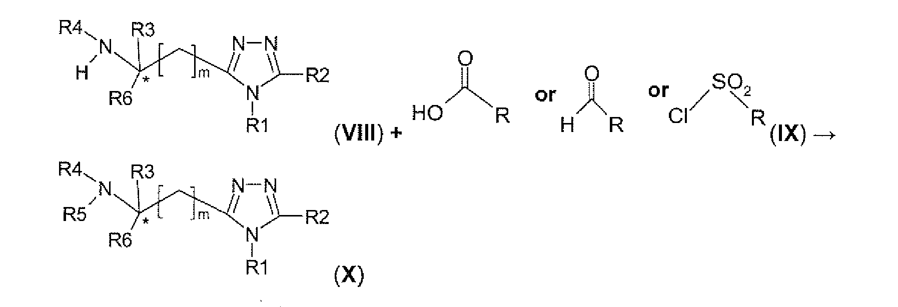
(i) optionally, reacting a compound of formula (X) in a solvent in the presence of an acid at a temperature T_{g} to yield a deprotected compound of formula (X) ("deprotection of R5")
wherein:
R5 is selected from the group consisting of "hydrogen atom, alkyl, cycloalkyl, cycloalkylalkyl, aryl, heteroaryl, arylalkyl, heteroarylalkyl, heterocyclyl, heterocyclylalkyl, -CO-alkyl, -CO-cycloalkyl, -CO-cycloalkylalkyl, -CO-aryl, -CO-arylalkyl, - CO-heteroaryl, -CO-heteroarylalkyl, -CO-heterocyclyl, -CO-heterocyclylalkyl, - CO-C*(R9R10)-NH₂, -CO-CH₂-C*(R9R10)-NH2, -CO-C*(R9R10)-CH₂-NH₂, alkylsulfonyl, arylsulfonyl, arylalkylsulfonyl" which are optionally substituted by up to 3 substituents independently selected from the group consisting of "halogen, -F, -Cl, -Br, -I, -N₃, -CN, -NR7R8, -OH, -NO₂, alkyl, aryl, arylalkyl, -O-alkyl, -O-aryl, - O-arylalkyl"; and preferably is selected from the group consisting of "hydrogen atom, -CO-alkyl, -CO-cycloalkyl, -CO-aryl, -CO-heteroaryl, -CO-arylalkyl, - CO-heteroarylalkyl, -CO-heterocyclyl, -CO-C*(R9R10)-NH_{2,} -CO-CH₂-C*(R9R10)-NH₂, -CO-C*(R9R10)-CH₂-NH₂, optionally being substituted by up to 3 substituents independently selected from the group consisting of "halogen, -F, - Cl, -Br, -I, -N₃, -CN, -NR7R8, -OH, -NO₂, alkyl, aryl, arylalkyl, -O-alkyl, -O-aryl, -O-arylalkyl";
R9, R10 are independently of one another selected from the group consisting of "hydrogen atom, alkyl, natural alpha-amino acid side chain, unnatural alpha-amino acid side chain" and preferably are selected from the group consisting of "hydrogen atom, alkyl";
m is 0, 1 or 2 and preferably is 0; and
* means a carbon atom of R or S configuration when chiral.

**4.** The process as claimed in any of claims 1 to 3, wherein the silver-compound in step (d) is selected from the group consisting of: "silver salts, silver acetate, silver benzoate, silver oxide" and preferably is silver benzoate.

**5.** The process as claimed in any of claims 1 to 4, wherein
the coupling reagent in step (a) and step (f) is independently from each other selected from the group consisting of: "benzotriazol-1-yl-oxy-tris-(dimethylamino)-phosphonium hexafluorphosphate (BOP), N-ethyl-N'-(3-dimethylaminopropyl) carbodiimide hydrochloride (EDC), 2-(1H-benzotriazol-1-yl)-1,1,3,3,-tetramethyluronoium hexafluorphosphate (HBTU)";
the base in step (a) and step (f) is an organic base and is independently from each other selected from the group consisting of: "N-methyl-morpholine, diisopropylethylamine";
the thionating reagent in step (b) is selected from the group consisting of: "Lawesson's reagent";
the acid in step (d), step (e) and step (g) is independently from each other an organic acid and is preferably selected from the group consisting of: "carboxylic acid, trifluoroacetic acid (TFA), TFA in the presence of anisole and thioanisole, hydrochloric acid, acetic acid";
the reducing reagent in step (f) is selected from the group consisting of: "NaBH₃CN, NaBH₄";
the solvent in steps (a) to (g) is an organic solvent and is preferably and independently from each other selected from the group consisting of: "dichloromethane (DCM), acetonitrile (ACN), ethanol, tetrahydrofuran (THF), dimethylether (DME), dimethylformamide (DMF)".

**6.** The process as claimed in any of claims 1 to 5, wherein
temperature Tₐ independently is room temperature (22° ± 4° C),
temperature T_{b} independently is between 75° C to 90° C, preferably 80° C or 85° C,
temperature T_{c} independently is between 75° C to 90° C, preferably 80° C or 85° C,
temperature T_{d} independently is room temperature (22° ± 4° C),
temperature Tₑ independently is room temperature (22° ± 4° C),
temperature T_{f} independently is room temperature (22° ± 4° C),
temperature T_{g} independently is room temperature (22° ± 4° C).

**7.** A triazole compound selected from the group consisting of:
| **Compound No.** | **Structure** |
|---|---|
| **1** | 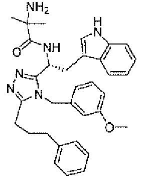 |
| **2** | 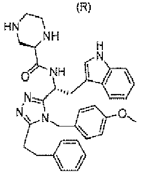 |
| **3** | 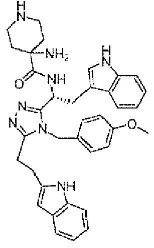 |
| **4** | 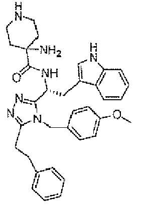 |
| **5** | 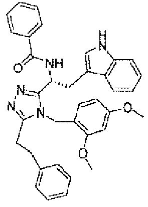 |
| **6** | 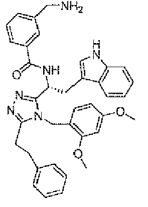 |
| **7** | 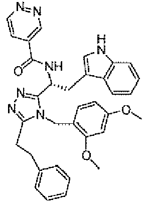 |
| **8** | 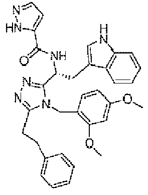 |
| **10** | 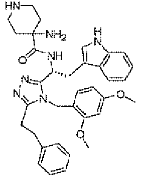 |
| **11** | 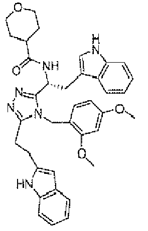 |
| **12** | 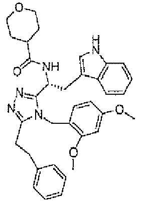 |
| **13** | 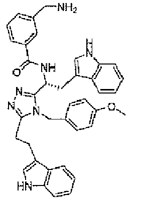 |
| **14** | 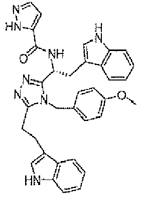 |
| **15** | 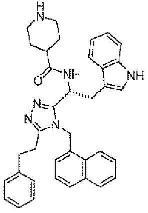 |
| **16** | 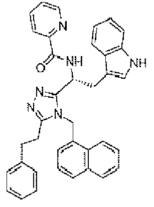 |
| **17** | 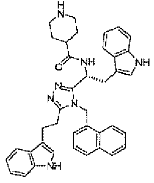 |
| **18** | 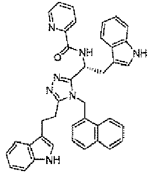 |
| **19** | 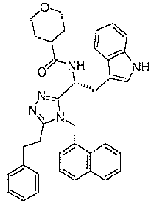 |
| **20** | 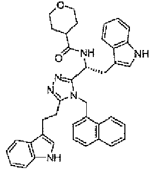 |
| **21** | 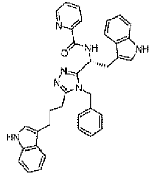 |
| **22** | 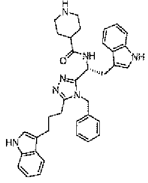 |
| **23** | 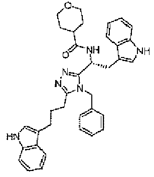 |
| **29** | 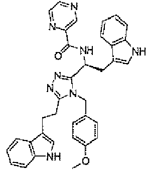 |
| **30** | 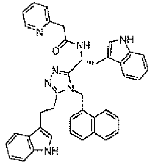 |
| **31** | 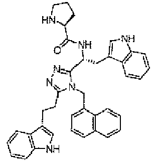 |
| **32** | 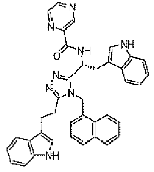 |
| **33** | 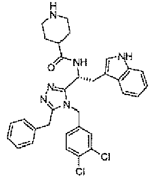 |
| **34** | 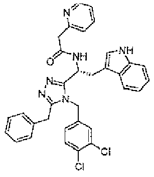 |
| **35** | 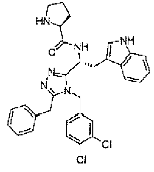 |
| **36** | 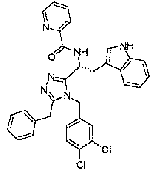 |
| **37** | 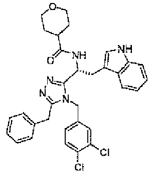 |
| **38** | 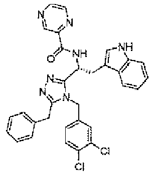 |
| **39** | 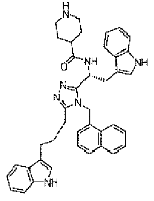 |
| **40** | 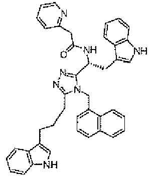 |
| **41** | 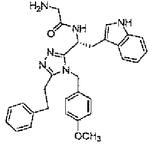 |
| **42** | 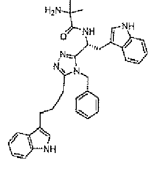 |
| **43** | 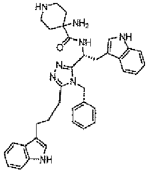 |
| **44** | 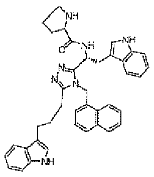 |
| **45** | 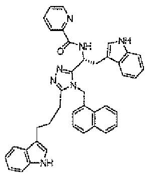 |
| **46** | 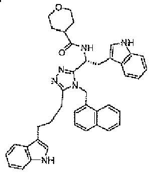 |
| **47** | 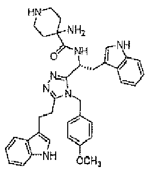 |
| **48** | 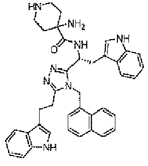 |
| **49** | 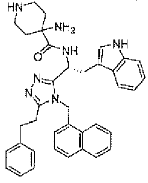 |
| **50** | 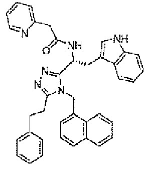 |
| **51** | 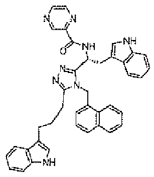 |
| **52** | 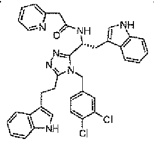 |
| **53** | 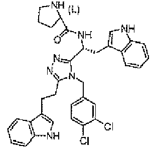 |
| **54** | 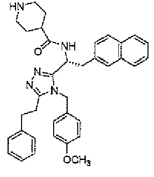 |
| **55** | 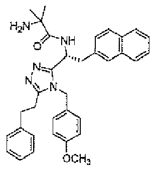 |
| **56** | 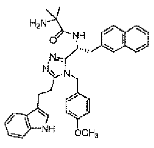 |
| **57** | 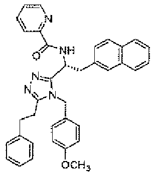 |
| **58** | 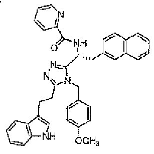 |
| **59** | 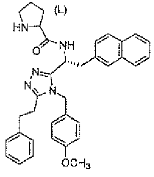 |
| **60** | 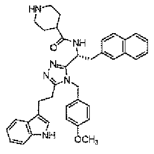 |
| **61** | 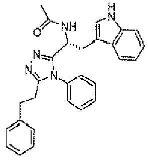 |
| **62** | 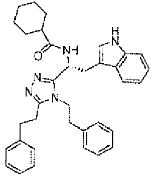 |
| **63** | 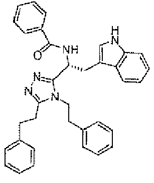 |
| **64** | 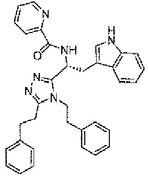 |
| **65** | 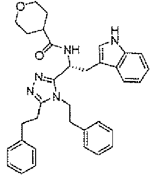 |
| **66** | 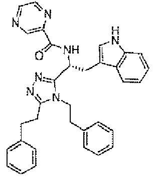 |
| **67** | 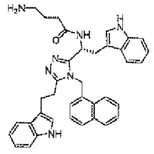 |
| **68** | 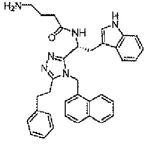 |
| **69** | 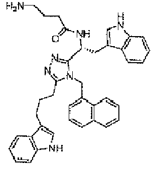 |
| **70** | 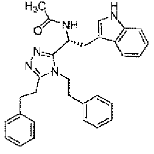 |
| **71** | 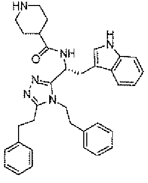 |
| **72** | 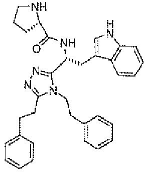 |
| **73** | 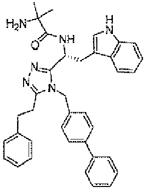 |
| **74** | 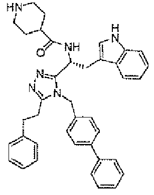 |
| **75** | 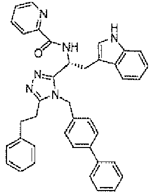 |
| **76** | 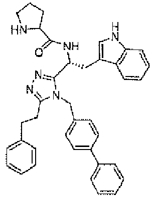 |
| **77** | 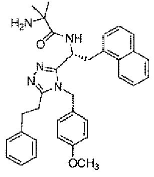 |
| **78** | 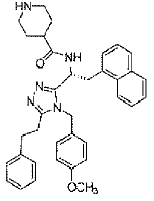 |
| **79** | 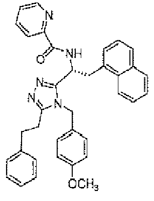 |
| **80** | 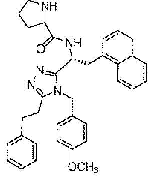 |
| **81** | 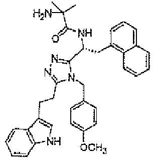 |
| **82** | 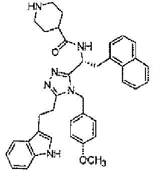 |
| **83** | 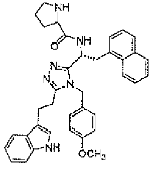 |
| **84** | 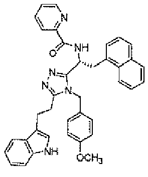 |
| **85** | 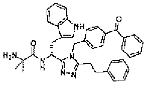 |
| **86** | 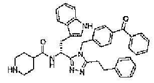 |
| **87** | 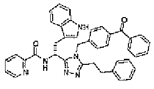 |
| **88** | 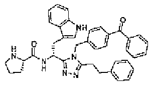 |

**14.** Medicament comprising at least one compound as claimed in claim 7 or at least one pharmaceutical composition as claimed in any of claims 8 to 12 for use in the prophylaxis or treatment of physiological and/or pathophysiological conditions selected from the group consisting of "acute fatigue syndrome and muscle loss following election surgery, adipogenesis, adiposity, age-related decline of thymic function, age-related functional decline ("ARFD") in the elderly, aging disorder in companion animals, Alzheimer's disease, anorexia (e.g.associated with cachexia or aging); anxiety, blood pressure (lowering), body weight gain/reduction, bone fracture repair (acceleration), bone remodeling stimulation, cachexia and protein loss reduction due to chronic illness such as cancer or AIDS, cardiac dysfunctions (e.g. associated with valvular disease, myocarial infarction, cardiac hypertrophy or congestive heart failure), cardiomyopathy, cartilage growth stimulation, catabolic disorders in connection with pulmonary dysfunction and ventilator dependency, catabolic side effects of glucocorticoids, catabolic state of aging, central nervous system disorders (in combination with antidepressants), chronic dialysis, chronic fatigue syndrome (CFS), cognitive function improvement (e.g. in dementia, Alzheimer's disease), complicated fractures (e.g. disctraction osteogenesis), complications associated with transplantation, congestive heart failure (alone/in combination with corticotropin releasing factor antagonists), Crohn's disease and ulcerative colits, Cushing's syndrome, dementia, depressions, short-, medium- and/or long-term regulation of energy balance, short-, medium- and/or long-term regulation of food intake (stimulation and/or inhibition), fraility (e.g. in elderly humans), gastrectomy (ghrelin replacement therapy), gastric postoperative ileus, glycemic control improvement, growth hormone release stimulation in the elderly, growth hormone replacement in stressed patients, growth promotion in livestock, growth retardation associated with the Prader-Willi syndrome and Turner's syndrome, growth retardation in connection with Crohn's disease, growth retardation, hair/nail growth maintenance, hip fractures, hunger, hypercortisolism, hyperinsulinemia including nesidioblastosis, hypothermia, immune deficiency in individuals with a depressed T4/T8 cell ratio, immune response improvement to vaccination, immune system stimulation in companion animals, immune system stimulation, immunosuppression in immunosuppressed patients, inflammation or inflammatory effects, inflammatory bowel disease, insulin resistance in the heart, insulin resistance in type 2 diabetic patients, insulin resistance including NIDDM, diabetes, diabetes type I, diabetes type II, intrauterine growth retardation, irritable bowel syndrome, lipodystrophy (e.g. HIV-induced), metabolic homeostasis maintenance, milk production increase in livestock, muscle mass/strength increase, muscle mobility improvement, muscle strength improvement, muscle strength/function maintenance in elderly humans, muscular atrophy, musculoskeletal impairment (e.g. in elderly), Noonan's syndrome, obesity and growth retardation associated with obesity, osteoblast stimulation, osteochondrodysplasias, osteoporosis, ovulation induction (adjuvant treatment), physiological short stature including growth hormone deficient children, postoperative ileus, protein catabolic response attenuation after major surgery/trauma, protein kinase B activity enhancement, psychosocial deprivation, pulmonary dysfunction and ventilator dependency, pulmonary function improvement, pulsatile growth hormone release induction, recovery of burn patients and reducing hospitalization of burn patients (acceleration), renal failure or insufficiency resulting from growth retardation, renal homeostasis maintenance in the frail elderly, sarcopenia, schizophrenia, sensory function maintenance (e.g. hearing, sight, olefaction and taste), short bowel syndrome, short stature associated with chronic illness, skeletal dysplasia, skin thickness maintenance, sleep disorders, sleep quality improvement, thrombocytopenia, thymic development stimulation, tooth repair or growth, tumor cell proliferation, ventricular dysfunction or reperfusion events, wasting in connection with AIDS, wasting in connection with chronic liver disease, wasting in connection with chronic obstructive pulmonary disease (COPD), wasting in connection with multiple sclerosis or other neurodegenerative disorders, wasting secondary to fractures, wool growth stimulation in sheep, wound healing (acceleration) and/or wound healing delay".

**15.** The medicament as claimed in claim 15, where the physiological and/or pathophysiological conditions are selected from the group consisting of "growth retardation, cachexia, short-, medium- and/or long term regulation of energy balance; short-, medium- and/or long term regulation (stimulation and/or inhibition) of food intake; adipogenesis, adiposity and/or obesity; body weight gain and/or reduction; diabetes, diabetes type I, diabetes type II, tumor cell proliferation; inflammation, inflammatory effects, gastric postoperative ileus, postoperative ileus and/or gastrectomy (ghrelin replacement therapy)"
